# EUROPEAN PATENT APPLICATION

(11) **EP 2 412 711 A1**
(43) Date of publication of application: **01.02.2012**
(21) Application number: 10756210.0
(22) Date of filing: 26.03.2010
(51) Int. Cl.: C07D 231/56, A61K 31/416, A61K 31/427, A61K 31/437, A61K 31/444, A61K 31/4545, A61K 31/4709, A61K 31/5377, A61K 31/538, A61P 9/06, C07D 417/12, C07D 471/04

(54) **INDAZOLEPROPIONIC ACID AMIDE COMPOUND**

(30) Priority: 27.03.2009 JP 2009079420
(71) Applicant: Mitsubishi Tanabe Pharma Corporation, Osaka-shi Osaka 541-8505 (JP)
(72) Inventor: YAMAGUCHI, Tetsuo, Osaka-shi Osaka 541-8505 (JP); USHIROGOCHI, Hideki, Osaka-shi Osaka 541-8505 (JP); HIRAI, Miki, Osaka-shi Osaka 541-8505 (JP); IMANISHI, Yasuhiro, Osaka-shi Osaka 541-8505 (JP)
(74) Representative: HOFFMANN EITLE
(86) International application number: PCT/JP2010/055338
(87) International publication number: WO 2010/110414

(57) **Abstract**

Disclosed is a compound which is useful in preventing and treating cardiac arrhythmia such as atrial fibrillation. A compound represented by formula (1) or a pharmaceutically acceptable salt of the same. 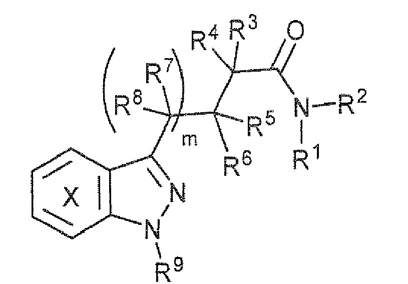 In formula (1), ring X represents benzene or pyridine; R¹ represents an optionally substituted alkyl group; R² represents an optionally substituted aryl group, an optionally substituted heterocyclic group, an optionally substituted arylalkyl group or an optionally substituted heterocyclic group-substituted alkyl group; R³, R⁴, R⁵, R⁶, R⁷, R⁸ and R⁹ represent each hydrogen or an alkyl group, provided that R³ and R⁵ may be bonded to each other to form, together with the carbon atom adjacent thereto, a cycloalkyl group; and m represents 0 or 1.

## Description

### TECHNICAL FIELD

The present invention relates to compounds with I_{Kur} blocking activity useful for preventing or treating diseases such as atrial fibrillation.

### BACKGROUND ART

Atrial fibrillation is one of the most general arrhythmia in clinical stages, in which irregular and frequent excitations of atrium stop a series of contraction and expansion as an auxiliary pump of atrium, and particularly, incidences thereof increase with advancing age. Atrial fibrillation is not a life-threatening arrhythmia, but worsens heart functions and is known to cause a complication such as congestive heart failure, thromboembolism, ventricular fibrillation, etc.

Antiarrhythmic agents launched until now have been developed as therapeutic agents for ventricular anhythmia and atrial or supraventricular arrhythmia. Malignant ventricular arrhythmia immediately threatens life and requires to be treated urgently, and class Ia (e.g., procainamide, quinidine), class Ic (e.g., flecainide, propafenone) or class III (e.g., dofetilide, amiodarone) agents have been used in drug treatment of ventricular arrhythmia. It has been reported that these class I and class III agents prevent a recurrence of atrial fibrillation (Nonpatent Document 1). However, they have potentials to increase mortalities due to their potentially lethal ventricular arrhythmogenic activities (Nonpatent Documents 2 to 4).

Since atrial fibrillation shortens cardiac action potential duration (APD), APD-prolonging agents may be theoretically a therapeutic agent for atrial fibrillation. A prolongation of cardiac APD is caused by increasing inward currents (i.e., Na⁺ or Ca²⁺ currents which are referred to as I_{Na} or I_{Ca}, respectively, hereinafter) or decreasing outward currents of repolarized potassium K⁺. Delayed rectifier (I_{K}) K⁺ currents are main outward currents involved in a repolarization process of action potential, and transient outward currents (Iₜₒ) and inward rectifier (I_{KI}) K⁺ currents are associated with an initial phase and a terminal phase of the repolarization, respectively. In cellular electrophysiological study, I_{K} comprises two subtypes of pharmacologically and kinetically different K⁺ currents, i.e., I_{Kr} (rapid activation) and I_{Ks} (slow activation) (Nonpatent Document 5).

Dofetilide, class III antiarrhythmic agent, shows an antiarrhythmic activity by blocking I_{Kr} which is an I_{K} rapid activating ingredient and exists in human atrium and ventricle (Nonpatent Document 1). Since an I_{Kr} blocker prolongs APD and refractory periods both in atrium and ventricle without affecting conduction itself, it has potentials to be theoretically a useful agent in the treatment of arrhythmia such as atrial fibrillation (Nonpatent Document 4). However, it has been reported that said agent has an arrhythmogenic activity and develops polymorphic torsades de pointes (Nonpatent Document 6).
On the other hand, it has been reported that amiodarone has class III property (Nonpatent Documents 7 and 8). However, since it has activities on multiple ion channels and is not a selective class III agent, a usage thereof has been strictly limited in terms of adverse effects thereof (Nonpatent Documents 9 to 11). Therefore, currently available agents such as amiodarone and dofetilide have serious adverse effects such as potentially lethal ventricular arrhythmogenic activities, and hence, high safe agents with beneficial efficacy have been desired.

Recently, ultra-rapid activated delayed rectifier K⁺ currents (I_{Kur}) which are prolonged outward currents have been identified in human atrial myocytes. I_{Kur} specifically exists in atrium, not in human ventricle. A molecular correlation of I_{Kur} in human atrium is potassium channel referred to as Kv 1.5, and Kv 1.5 mRNA (Nonpatent Document 12) and protein (Nonpatent Document 13) have been detected in human atrial tissues. It has been believed that I_{Kur} widely contributes to a repolarization in human atrium due to a rapid activation and a delayed inactivation thereof. Therefore, it would appear that since a compound having an I_{Kur} blocking activity prolongs a refractory in atrium without delaying a ventricular depolarization and prolonging a refractory period in ventricle, it may resolve adverse effect problems such as an arrhythmia-induced QT extension syndrome after a depolarization found in the current class III agents (Nonpatent Documents 14 and 15).

In contrast, it has been shown that a reentry (reciprocation) is a remarkable mechanism which causes a supraventricular arrhythmia in human (Nonpatent Document 16). Specifically, reciprocations occur at random in different locations in atrium, and atrial fibrillation is caused by several times of repetitions of electrical excitations by a single stimulation. Accordingly, an increase of myocardial refractory by a prolongation of cardiac APD prevents and/or stops reentry arrhythmia. Additionally, since cardiac APD depends on contributions of potassium currents I_{Kr}, I_{Ks}, I_{Kur} which relate to a repolarization phase and transient outward currents Iₜₒ, it is desired that a blocker which acts on any one of these currents prolongs action potential durations and produces an antiarrhythmic effect.

Patent Document 1 discloses useful indazole derivatives as a SGK-1 inhibitor, but does not disclose any I_{Kur} blocking activities.

[Patent Document 1] WO2005/011681

[Nonpatent Document 1] Circulation, 102:2665-2670
[Nonpatent Document 2] Am. J. Cardiol., 65:20B-29B, 1990
[Nonpatent Document 3] Lancet, 348:7-12, 1996
[Nonpatent Document 4] Expert Opin. Invest. Drugs, 9:2695-2704, 2000
[Nonpatent Document 5] J. Gen. Physiol. 1990, 96:195-215
[Nonpatent Document 6] Am. J. Cardiol., 72:44B-49B, 1993
[Nonpatent Document 7] Br. J. Phannacol., 39:675-689, 1970
[Nonpatent Document 8] Br. J. Pharmacol., 39:657-667, 1974
[Nonpatent Document 9] J. Am. Coll. Cardiol., 20:1063-1065, 1992
[Nonpatent Document 10] Circulation, 104:2118-2150, 2001
[Nonpatent Document 11] A. Curr. Opin. Pharmacol. 2:154-159, 2002
[Nonpatent Document 12] Basic Res. Cardio, 97:424-433,2002
[Nonpatent Document 13] J. Clin. Invest., 96:282-292, 1995
[Nonpatent Document 14] J. Med. Chem., 46:486-498,2003
[Nonpatent Document 15] Naunyn-Schmedieberg's Arch. Pharmacol., 366:482-287, 2002
[Nonpatent Document 16] Nature, 415:219-226, 2002

### DISCLOSURE OF INVENTION

### PROBLEMS TO BE RESOLVED BY THE INVENTION

The present invention is directed to provide useful compounds for preventing or treating atrial fibrillation having less adverse effects and excellent I_{Kur} blocking activities.

### MEANS OF SOLVING THE PROBLEMS

According to extensive studies for solving the above problems, the present inventors have found that a compound of the following formula has an excellent I_{Kur} blocking activity, and the present invention has been achieved.

The present invention is as follows. 1. A compound of the general formula: 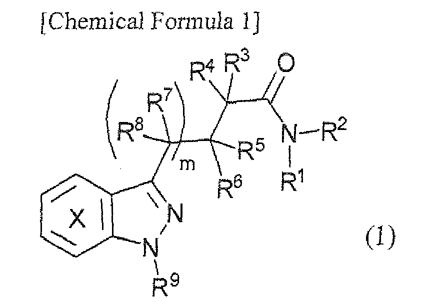 wherein Ring X is benzene or pyridine;
R¹ is optionally substituted alkyl;
R² is optionally substituted aryl, optionally substituted heterocyclic group, optionally substituted arylalkyl or optionally substituted heterocyclic-substituted alkyl;
provided that if Ring X is pyridine, R¹ and R² may combine each other together with the adjacent nitrogen atom to form a heterocyclic group of the following formula: 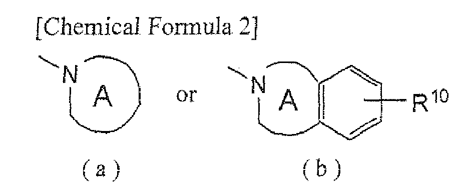 wherein Ring A is heterocyclic group, and R¹⁰ is hydrogen or alkyl;
R³, R⁴, R⁵, R⁶, R⁷, R⁸ and R⁹ are each hydrogen or alkyl; and R³ and R⁵ may combine each other together with the adjacent carbon atom to form cycloalkyl;
m is 0 or 1; or a pharmaceutically acceptable salt thereof.

2. The compound of the above 1, wherein R¹ is alkyl optionally substituted by 1 to 3 groups selected from (1) halogen, (2) hydroxyl, (3) optionally substituted amino, (4) alkylsulfonyl, (5) optionally substituted aminosulfonyl, (6) alkoxy, (7) cyano, (8) heterocyclic group, (9) optionally substituted carbamoyloxy, (10) optionally substituted carbamoyl, and (11) heterocyclic-substituted carbonyloxy, or a pharmaceutically acceptable salt thereof.

3. The compound of the above 1, wherein R¹ is alkyl optionally substituted by 1 to 3 groups selected from (1) hydroxyl, (2) amino optionally substituted by 1 or 2 groups selected from alkanoyl, alkoxycarbonyl, alkylsulfonyl, or aminosulfonyl optionally mono- or di-substituted by alkyl, and (3) carbamoyloxy optionally mono- or di-substituted by alkyl, or a pharmaceutically acceptable salt thereof.

4. The compound of any one of the above 1 to 3, wherein R² is aryl optionally substituted by 1 to 3 groups selected from (1) halogen, (2) alkyl, (3) alkoxy, (4) haloalkyl, (5) optionally substituted amino and (6) optionally substituted carbamoyl; arylalkyl optionally substituted by 1 to 3 groups selected from (1) halogen and (2) alkyl; or heterocyclic group, or a pharmaceutically acceptable salt thereof.

5. The compound of any one of the above 1 to 3, wherein R² is aryl optionally substituted by 1 to 3 groups selected from alkyl and alkoxy, or a pharmaceutically acceptable salt thereof.

6. The compound of any one of the above 1 to 5, wherein cycloalkyl which R³ and R⁵ combine each other together with the adjacent carbon atom to form is cyclopropyl, or a pharmaceutically acceptable salt thereof.

7. The compound of any one of the above 1 to 6, wherein m is 0, or a pharmaceutically acceptable salt thereof.

8. The compound of any one of the above 1 to 7, wherein R⁹ is hydrogen, or a pharmaceutically acceptable salt thereof.

9. A medicine comprising the compound of any one of the above 1 to 8 or a pharmaceutically acceptable salt thereof.
10. An I_{Kur} blocking agent comprising as the active ingredient the compound of any one of the above 1 to 8 or a pharmaceutically acceptable salt thereof.
11. A preventive or therapeutic agent for cardiac arrhythmia comprising as the active ingredient the compound of any one of the above 1 to 8 or a pharmaceutically acceptable salt thereof.
12. A preventive or therapeutic agent for atrial fibrillation comprising as the active ingredient the compound of any one of the above 1 to 8 or a pharmaceutically acceptable salt thereof.

Each group represented by each symbol in the present specification is illustrated below. Each abbreviation used in the present specification represents the following meaning.
THF: tetrahydrofuran
DMF: N,N-dimethylformamide
DMSO: dimethyl sulfoxide
DMA: dimethylacetamide
DME: 1,2-dimethoxyethane
LDA: lithium diisopropylamide
DBU: 1,8-diazabicyclo[5.4.0]-7-undecene
DBN: 1,5-diazabicyclo[4.3.0]non-5-ene
DCC: dicyclohexylcarbodiimide
WSC: 1-ethyl-3-(3'-dimethylaminopropyl)carbodiimide hydrochloride
HOBt: 1-hydroxybenzotriazole
TFA: trifluoroacetic acid
Ac: acetyl
Me: methyl
Et: ethyl
Pr: normal propyl
ⁱPr: isopropyl
Bu: normal butyl
ⁱBu: isobutyl
^{t}Bu: tertiary butyl
Boc: tertiary butoxycarbonyl
Cbz: carbobenzoxy
Bn: benzyl
Ph: phenyl
PMB; p-methoxybenzyl

"Alkyl" includes, for example, straight or branched chain C1 to C6 alkyl, specifically methyl, ethyl, propyl, isopropyl, butyl, isobutyl, tert-butyl, pentyl, hexyl, etc.

The aryl in "aryl'' and "arylalkyl" includes, for example, 3- to 15-membered monocyclic, bicyclic or tricyclic aromatic carbocyclic group, specifically phenyl, naphthyl, phenanthryl, anthryl, etc.

The heterocyclic group in "heterocyclic group" as well as "hetcrocyclic-substituted alkyl" and "heterocyclic-substituted carbonyloxy" includes, for example, 3- to 15-membered monocyclic or bicyclic unsaturated heterocyclic group and saturated or partially-saturated heterocyclic group which contain 1 to 4 heteroatoms selected from nitrogen atom, oxygen atom and sulfur atom.
The unsaturated heterocyclic group and the saturated or partially-saturated heterocyclic group include, for example, pyrrolyl, imidazolyl, triazolyl, tetrazolyl, pyrazolyl, pyridyl, pyrazinyl, pyrimidinyl, pyridazinyl, azepinyl, diazepinyl, furyl, pyranyl, oxepinyl, thienyl, thiapyranyl, thiepinyl, oxazolyl, isoxazolyl, thiazolyl, isothiazolyl, furazanyl, oxadiazolyl, oxadinyl, oxadiazinyl, oxazepinyl, oxadiazepinyl, thiadiazolyl, thiadinyl, thiadiazinyl, thiazepinyl, thiadiazepinyl, indolyl, isoindolyl, benzofuranyl, benzothiophenyl, indazolyl, quinolinyl, isoquinolinyl, quinoxalinyl, quinazolinyl, benzoxazolyl, benzothiazolyl, beiazoimidazolyl, pyrrolinyl, pyrrolidinyl, imidazolinyl, imidazolidinyl, pyrazolinyl, pyrazolidinyl, dihydropyridyl, tetrahydropyridyl, dihydropyrazinyl, tetrahydropyrazinyl, dihydropyrimidinyl, tetrahydropyrimidinyl, dihydroazepinyl, tetrahydroazepinyl, hexahydroazepinyl, dihydrodiazepinyl, tetrahydrodiazepinyl, dihydroxazepinyl, tetrahydroxazepinyl, hexahydroxazepinyl, dihydrofuryl, tetrahydrofuryl, dihydropyranyl, tetrahydropyranyl, dihydrothienyl, tetrahydrothienyl, dihydrothiapyranyl, tetrahydrothiapyranyl, piperidyl, piperazinyl, morpholinyl, thiamorpholinyl, homopiperidyl, etc.

"Alkoxycarbonyl" includes, for example, straight or branched chain C2 to C7 alkoxycarbonyl, specifically methoxycarbonyl, ethoxycarbonyl, propoxycarbonyl, isopropoxycarbonyl, butoxycarbonyl, isobutoxycarbonyl, tert-butoxycarbonyl, etc. A preferable one among them is C2 to C5 alkoxycarbonyl.

"Halogen" includes fluorine, chlorine, bromine, iodine. A preferable one among them is chlorine or fluorine.

"Alkoxy" includes, for example, straight or branched chain C1 to C6 alkoxy, specifically methoxy, ethoxy, propoxy, isopropoxy, butoxy, isobutoxy, tert-butoxy, pentyloxy, hexyloxy, etc. A preferable one among them is C1 to C4 alkoxy.

"Cycloalkyl which R³ and R⁵ combine each other together with the adjacent carbon atom to form" includes, for example, C3 to C8 cycloalkyl, specifically cyclopropyl, cyclobutyl, cyclopentyl, cyclohexyl, cycloheptyl, etc. A preferable one among them is cyclopropyl.

"Alkylsulfonyl" includes, for example, straight or branched chain C1 to C6 alkylsulfonyl, specifically methylsulfonyl, ethylsulfonyl, propylsulfonyl, isopropylsulfonyl, butylsulfonyl, etc. A preferable one among them is C 1 to C4 alkylsulfonyl.

"Alkanoyl" includes, for example, straight or branched chain C1 to C6 alkanoyl, specifically formyl, acetyl, propionyl, butyryl, pentanoyl, hexanoyl, etc. A preferable one among them is C1 to C4 alkanoyl.

"Haloalkyl" includes, for example, straight or branched chain C1 to C6 alkyl, preferably C1 to C4 alkyl, substituted with 1 to 6 halogens, specifically ftuoromethyl, chloromethyl, trifluoromethyl, 2,2,2-trifluoroethyl, etc.

The substituents of "substituted alkyl" in R¹ include, for example,
(1) hydroxyl,
(2) optionally substituted amino,
(3) alkylsulfonyl,
(4) arylsulfonyl,
(5) cyano,
(6) alkoxy,
(7) optionally substituted heterocyclic group,
(8) optionally substituted cycloalkyl,
(9) optionally substituted carbamoyl,
(10) optionally substituted carbamoyloxy,
(11) heterocyclic-substituted carbonyl,
(12) optionally substituted heterocyclic-substituted carbonyloxy,
(13) optionally substituted aminosulfonyl,
(14) alkoxycarbonyl,
(15) halogen, etc., and
the substituents may be the same or different 1 to 3 substituents among these substituents.
Particularly, (1) to (3), (5), (6), (7), (9), (10), (12), (13) and (15) are preferable among the above substituents of "substituted alkyl".

The substituents of "optionally substituted amino" in the substituents of the above "substituted alkyl" include, for example, 1 or 2 groups selected from the following (A) to (M).
(A) alkyl optionally substituted with alkoxy,
(B) alkanoyl optionally substituted with 1 to 3 groups selected from Group a,
(C) alkanoylamino,
(D) alkoxycarbonyl optionally substituted with 1 to 3 groups selected from Group a,
(E) alkylsulfonyl optionally substituted with 1 or 2 groups selected from Group b,
(F) heterocyclic-substituted sulfonyl optionally substituted with 1 or 2 groups selected from Group c,
(G) arylcarbonyl,
(H) aralkylcarbonyl,
(I) aminosulfonyl optionally mono- or di-substituted with alkyl,
(J) cycloalhylcarbonyl optionally substituted with alkyl or cyano,
(K) heterocyclic-substituted carbonyl,
(L) heterocyclic-substituted oxycarbonyl, or
(M) carbamoyl optionally mono- or di-substituted with alkyl, etc.
Particularly, (A), (B), (D) to (I) and (K) to (M) are preferable among the above substituents of "optionally substituted amino".

### [Group a]

(a) alkoxy,
(b) cyano,
(c) heterocyclic group,
(d) alkylsulfonyl, and
(e) halogen

### [Group b]

(a) alkoxy, and
(b) halogen

### [Group c]

(a) alkoxy,
(b) alkyl, and
(c) halogen
Preferable one includes alkoxy or halogen among Group a, and alkoxy among Group b.

The substituents of "optionally substituted heterocyclic group" in the above substituents of the "substituted alkyl" include, for example, (A) oxo, (B) alkoxycarbonyl, (C) alkanoyl, (D) alkyl, (E) alkylsulfonylamino, (F) alkylsulfonyl, (G) heterocyclic-substituted carbonyl, (H) aminosulfonyl optionally mono- or di-substituted with alkyl, (I) carbamoyl optionally mono- or di-substituted with alkyl or (J) halogen, and the substituents may be the same or different 1 to 3 groups.

The substituents of "optionally substituted cycloalkyl" in the above substituents of the "substituted alkyl" include, for example, (A) alkoxy, (B) hydroxyl or (C) alkyl, and the substituents may be the same or different 1 to 2 substituents.

The substituents of "optionally substituted carbamoyl" in the above substituents of the "substituted alkyl" include, for example, the same or different 1 to 2 alkyls, etc.

The substituents of "optionally substituted carbamoyloxy" in the above substituents of the "substituted alkyl" include, for example:
(A) heterocyclic group,
(B) alkyl optionally substituted with the same or different 1 or 2 groups selected from (a) alkoxy, (b) hydroxyl, (c) cyano, and (d) carbamoyl optionally substituted with the same or different 1 or 2 alkyls, etc.

The substituents of "optionally substituted heterocyclic-substituted carbonyloxy" in the above substituents of the "substituted alkyl" include, for example, (A) alkoxy, (B) alkyl, (C) alkanoyl, etc.

The substituents of "optionally substituted aminosulfonyl" in the above substituents of the "substituted alkyl" include, for example, alkyl, and the substituents may be the same or different 1 to 2 groups.

The substituents of "optionally substituted aryl", "optionally substituted heterocyclic group", "optionally substituted arylalkyl" and "optionally substituted heterocyclic-substituted alkyl" in R² include, for example:
(1) optionally substituted alkyl,
(2) optionally substituted alkoxy,
(3) halogen,
(4) heterocyclic group,
(5) amino optionally mono- or di-substituted with alkyl
(6) hydroxyl, or
(7) carbamoyl optionally mono- or di-substituted with alkyl, etc., and the substituents may be the same or different 1 to 3 substituents.

The substituents of "optionally substituted alkyl" in the above substituents of R² include, for example, (A) halogen, (B) alkoxycarbonyl, etc., and the substituents may be the same or different 1 to 3 groups.

The substituents of "optionally substituted alkoxy" in the above substituents of R² include, for example. 1 to 3 halogens, etc.

A preferable R² is optionally substituted aryl, particularly optionally substituted phenyl.

"Heterocyclic group" and the heterocyclic group in "heterocyclic-substituted carbonyl" and "heteracyclic-substituted carbonyloxy" of the substituent in R¹ preferably include 4- to 7-membered monocyclic heterocyclic group, specifically morpholinyl, thiomorpholinyl, pyrrolidinyl, piperazinyl, piperidyl, homopiperazinyl, hexahydroazepinyl, hexahydrooxazepinyl, azetidinyl, pyridyl, pyrimidyl, thiazolyl, pyrazolyl, tetrahydropyranyl, tetrahydrofuryl. Among them, morpholinyl, pyrrolidinyl, piperidyl, pyridyl, thiazolyl, or tetrahydrofuryl is preferable.

"heterocyclic group" of substituents in "optionally substituted amino" and "optionally substituted carbamoyloxy" and the heterocyclic group of "heterocyclic-substituted sulfonyl" and "heterocyclic-substituted carbonyl" of the substituents in R¹ preferably include the above heterocyclic group.

"Heterocyclic group" in R² preferably includes 4- to 7-membered monocyclic heterocyclic group or a condensed group of the monocyclic heterocyclic group with benzene ring, specifically pyridyl, pyrimidyl, indolyl, quinolyl, 2,3-dihydroindolyl, 1,2,3,4-tetrahydroquinolyl, etc.

"Heterocyclic group" of the substituent in R² preferably includes 4- to 7-membered monocyclic heterocyclic group, specifically pyrrolidinyl, piperidyl, piperazinyl, homopiperazinyl, morpholinyl, thiomorpholinyl, etc.

The heterocyclic group which R¹ and R² combine each other together with the adjacent nitrogen atom to form includes the following groups: 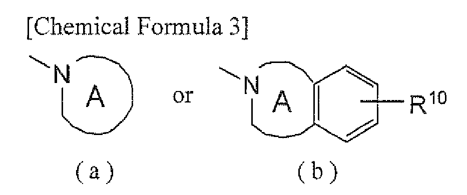 wherein Ring A is heterocyclic group, and R¹⁰ is hydrogen or alkyl.
The heterocyclic group represented by Ring A includes 4 to 7-membered monocyclic heterocyclic group, specifically pyrrolidinyl, piperidyl, piperazinyl, homopiperazinyl, morpholinyl, thiomorpholinyl, etc. A concrete example of formula (b) includes, for example, groups of the following formulae, and among them, (b3), (b5) and (b8) are preferable.

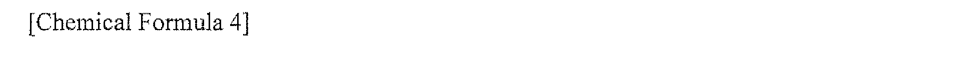

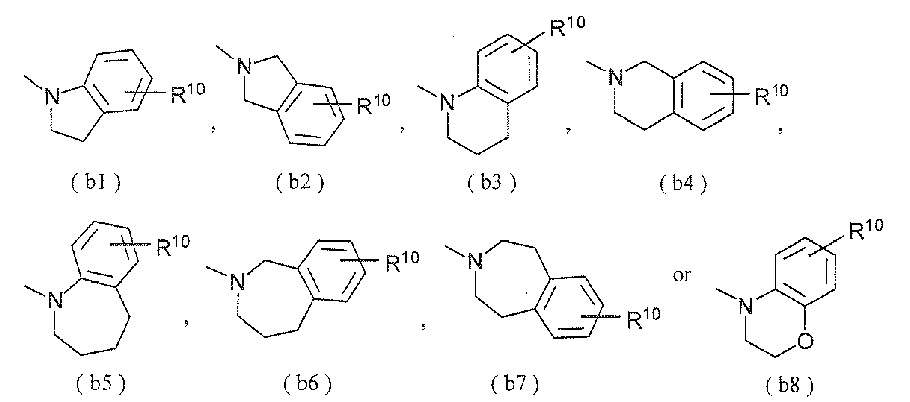

A pharmaceutically acceptable salt of the compound of the present invention includes, for example, an inorganic acid salt such as hydrochloride, sulfate, phosphate, hydrobromide, or an organic acid salt such as acetate, fumarate, oxalate, citrate, methanesulfonate, benzenesulfonate, tosylate, maleate, etc. The salt of the compound of the present invention having an acidic group such as carboxy also includes a salt with a base (e.g., an alkali metal salt such as sodium salt, potassium salt, an alkaline earth metal salt such as calcium salt, an organic base salt such as triethylamine salt, an amino acid salt such as lysine salt).
The compound of the present invention or a pharmaceutically acceptable salt thereof includes both an intramolecular salt thereof and a solvate thereof such as a hydrate thereof.
The compound (1) of the present invention may exist as optically active isomers based on its asymmetric carbons, diastereomers, and includes any isomers and a mixture thereof.

The compound (1) of the present invention may be prepared according to the following Methods.
Method 1: Compound (1-a) wherein m is 0 and R³ and R⁵ are hydrogen is prepared according to the following method. 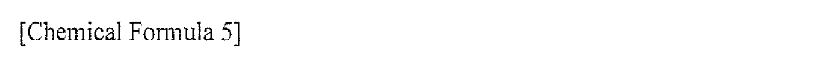 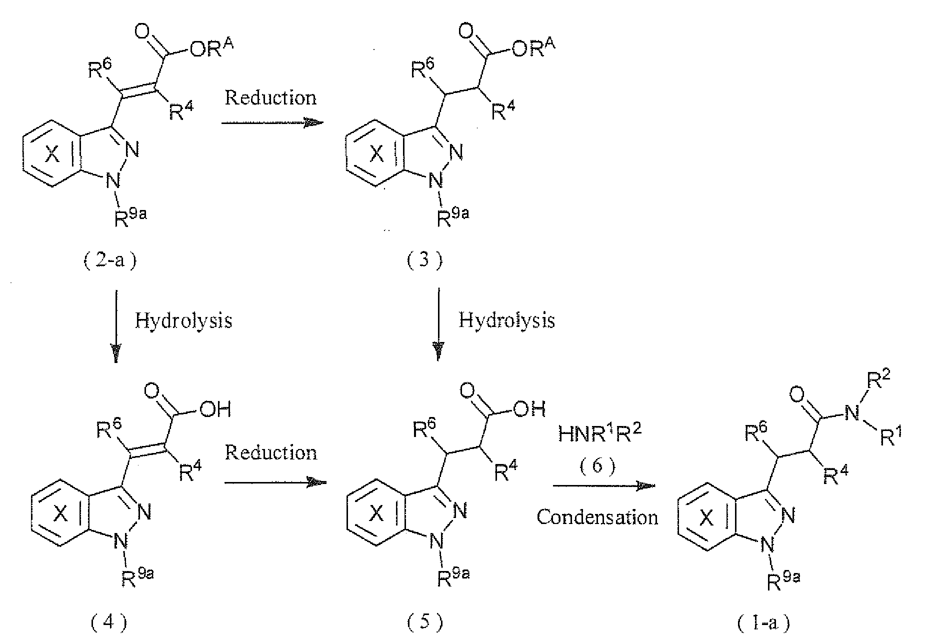 wherein R^{A} is alkyl, R^{9a} is hydrogen, alkyl or an amino protective group (including Boc, Cbz, PMB), and the other symbols have the same meanings as defined above.
The reduction of Compound (2-a) or (4) may be carried out by the treatment with a reducing agent (including sodium borohydride, lithium borohydride, lithium aluminum hydride) in a solvent (including methanol, ethanol, isopropanol, THF, diethyl ether), or by the catalytic reduction using a transition metal (including palladium-carbon, platinum oxide, Raney nickel, rhodium, ruthenium). A hydrogen source for the catalytic reduction may be formic acid, ammonium format, 1,4-cyclohexadiene, etc. The reaction proceeds usually at -20 to 150°C for 30 minutes to 48 hours.
The hydrolysis of Compound (2-a) or (3) may be carried out by the treatment with an aqueous solution of an acid (including hydrochloric acid, sulfuric acid) or a base (including sodium hydroxide, potassium hydroxide) in a solvent (including methanol, ethanol, isopropanol, dioxane, THF, diethyl ether) or without solvent. The reaction proceeds usually at -20 to 100°C for 30 minutes to 48 hours.
The condensation may be carried out by the following methods.
(1) Compound (5) is treated with a halogenating agent (including N-bromosuccinimide, N-chlorosuccinimide), triphenylphosphine and a base (including triethylamine, diisopropylethylamine, N,N-dimethylaniline) in a solvent (including dioxane, THF, methylene chloride), and then condensed with Compound (6) to give Compound (1-a). The reaction proceeds usually at 0°C to a reflux temperature of the solvent for 1 to 48 hours. The reaction may be optionally carried out under microwave irradiation, if needed.
(2) Compound (5) is condensed with Compound (6) in a solvent (including DMF, THF, dioxane), if needed, in the presence of a condensing agent (including DCC, WSC, carbonyldiimidazole, diethyl cyanophosphate) to give Compound (1-a). The reaction proceeds usually at 0°C to 100°C for 30 minutes to 24 hours. The reaction using a condensing agent may be also optionally carried out in the presence of HOBt, N-hydroxysuccinimide, etc., if needed.
(3) Compound (5) is converted into a mixed acid anhydride (including carbonate ester such as methyl chlorocarbonate, ethyl chlorocarbonate), and the mixed acid anhydride is condensed with Compound (6) in an appropriate solvent (including THF, toluene, nitrobenzene or a mixed solvent thereof) in the presence of a base (including triethylamine, pyridine) at -30°C to a reflux temperature of the solvent for 1 to 24 hours to give Compound (1-a).
Both Compound (3) and Compound (5) may be also prepared according to the method of Tetrahedron Lett., 41 (2000) 4363-4366.

Method 2: Compound (1-b) wherein m is 0, R³ is hydrogen and R⁵ is alkyl is prepared according to the following method. 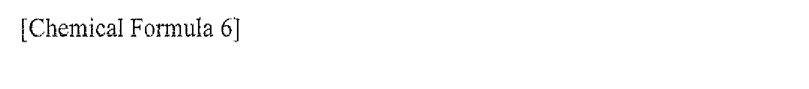 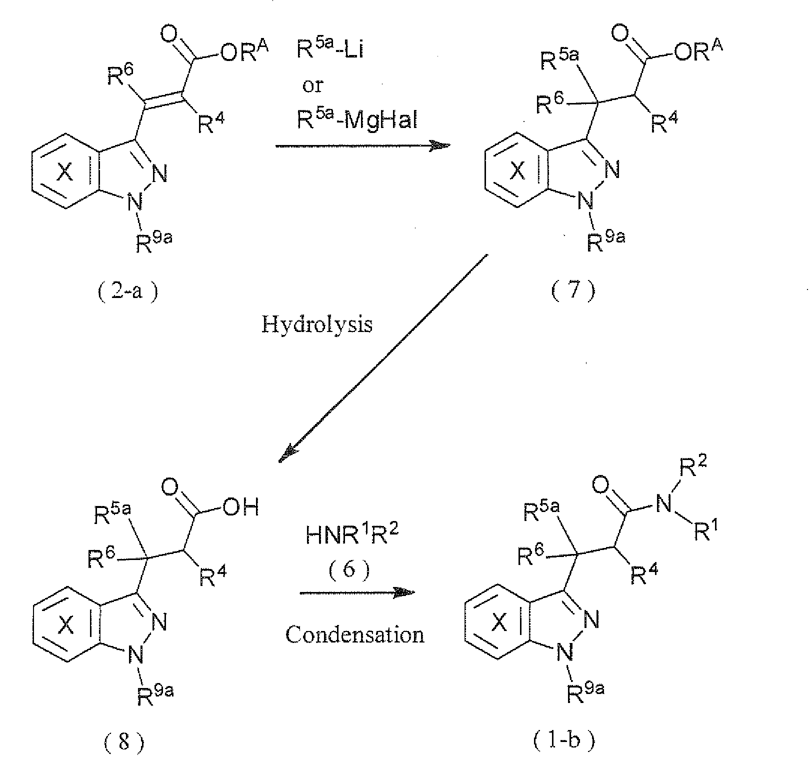 wherein Hal is halogen, R^{5a} is alkyl, and the other symbols have the same meanings as defined above.
Compound (2-a) is reacted with R^{5a}-Li or R^{5a}-MgHal in a solvent (including methylene chloride, THF, dioxane, diethyl ether, benzene, toluene, xylene, cyclohexane) in the presence of a monovalent copper salt (including copper (I) iodide, copper (I) bromide, copper (I) cyanide) at -78°C to room temperature for 1 to 24 hours to give Compound (7). The reaction may be also carried out in the presence of Lewis acid such as trimethylsilyl chloride, if needed.
The hydrolysis of Compound (7) and the condensation of Compound (8) may be carried out in the similar manner to Method 1.

Method 3: Compound (I-c) wherein in is 0 and R³ is alkcyl is prepared according to the following method. 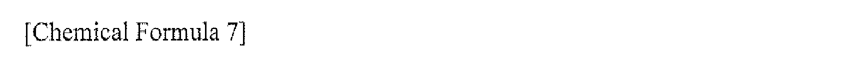 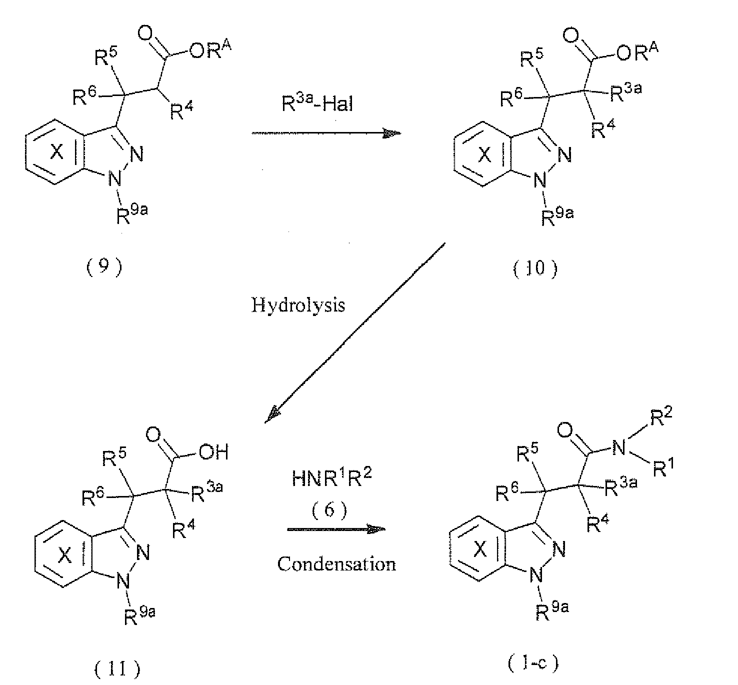 wherein R^{3a} is alkyl, and the other symbols have the same meanings as defined above.
Compound (9) is reacted with R^{3a}-Hal in a solvent (including THF, diethyl ether, DMF, DMSO, methanol, ethanol) in the presence of a base (including potassium t-butoxide, sodium methoxide, sodium ethoxide, sodium hydride, potassium hydride, LDA, butyl lithium) at -78°C to room temperature for 1 to 24 hours to give Compound (10).
The hydrolysis of Compound (10) and the condensation of Compound (11) may be carried out in the similar manner to Method 1.

Method 4: Compound (1-d) wherein m is 0 and R³ and R⁵ combine each other together with the adjacent carbon atom to form cyclopropyl is prepared according to the following method. 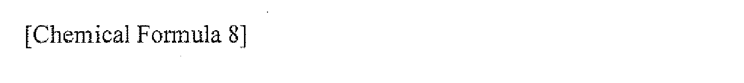 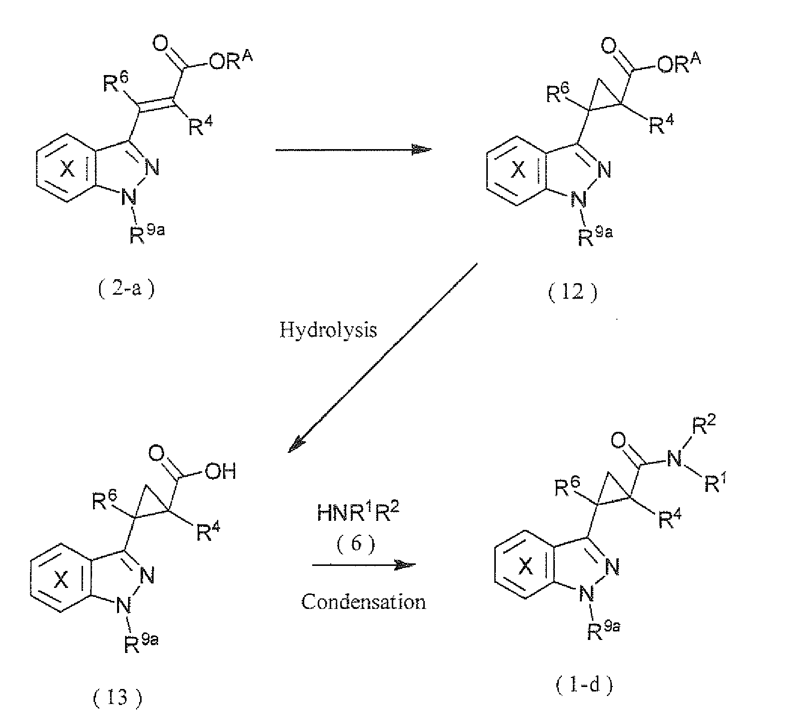 wherein each symbol has the same meaning as defined above.
Compound (12) is prepared according to the following method (1) or (2).
(1) Compound (2-a) is reacted with trimethylsulfoxonium halide in a solvent (including DMF, DMSO, DME) in the presence of a base (including sodium hydride, potassium hydride, potassium t-butoxide) at -20°C to room temperature for 1 to 24 hours to give Compound (12).
(2) Compound (2-a) is reacted with diazomethane in a solvent (including THF, diethyl ether, toluene, benzene, methanol, ethanol) at -20°C to room temperature for 1 to 12 hours to give Compound (12).
The hydrolysis of Compound (12) and the condensation of Compound (13) may be carried out in the similar manner to Method 1.

Method 5: Compound (1-e) wherein m is 1 is prepared according to the following method. 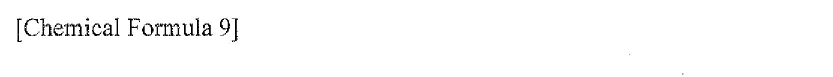 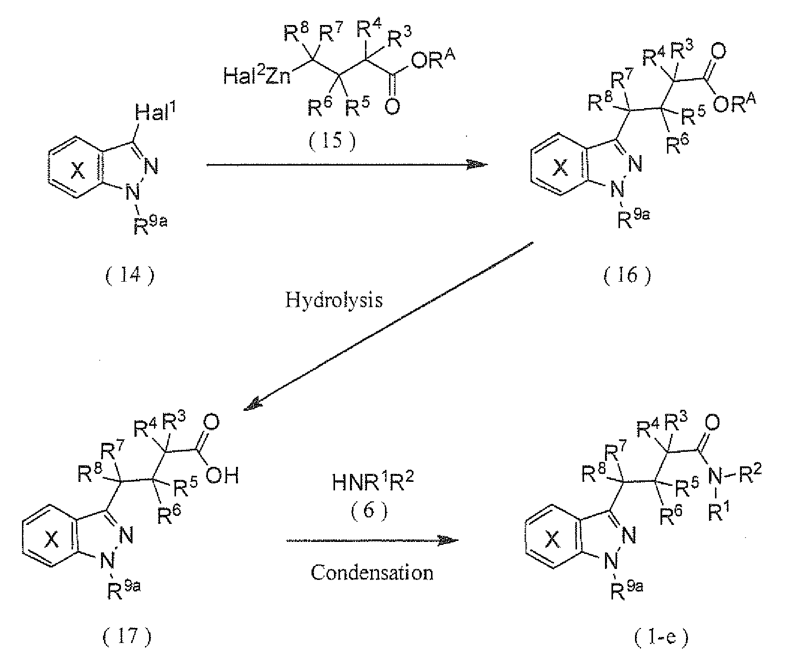 wherein Hal¹ and Hal² are each halogen, and the other symbols have the same meanings as defined above.
Compound (14) is reacted with Compound (15) in a solvent (including DME, THF, dioxane, DMF, DMA, toluene, benzene or a mixture thereof) in the presence of a catalyst [including a palladium catalyst such as tetrakis(triphenylphosphine)palladium (0), bis(triphenylphosphine)palladium (II) chloride, palladium (II) acetate, dichloro[1,1'-bis(diphenylphosphino)ferrocene]palladium (II)] at room temperature to a reflux temperature of the solvent for an hour to 3 days to give Compound (16).
The hydrolysis of Compound (16) and the condensation of Compound (17) may be carried out in the similar manner to Method 1.

Method 6: Compound (1-f) wherein m is 0 may be also prepared according to the following method. 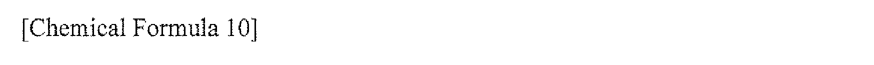 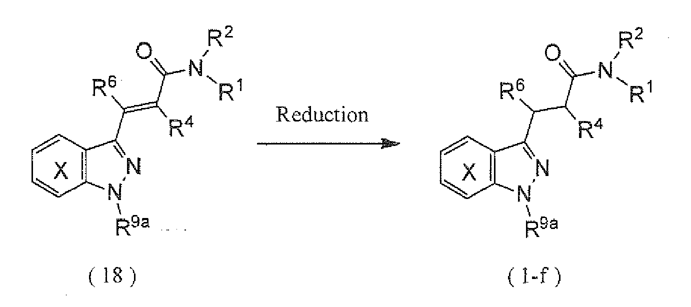 wherein each symbol has the same meaning as defined above.
The reduction may be carried out in the similar manner to Method 1.

Compound (2-a), Compound (14) and Compound (18) used in the above reactions may be prepared according to the method described in PCT/JP2008/067393. Compound (2-a), Compound (14) and Compound (18) are prepared according to the following method, for example.
Method 7: 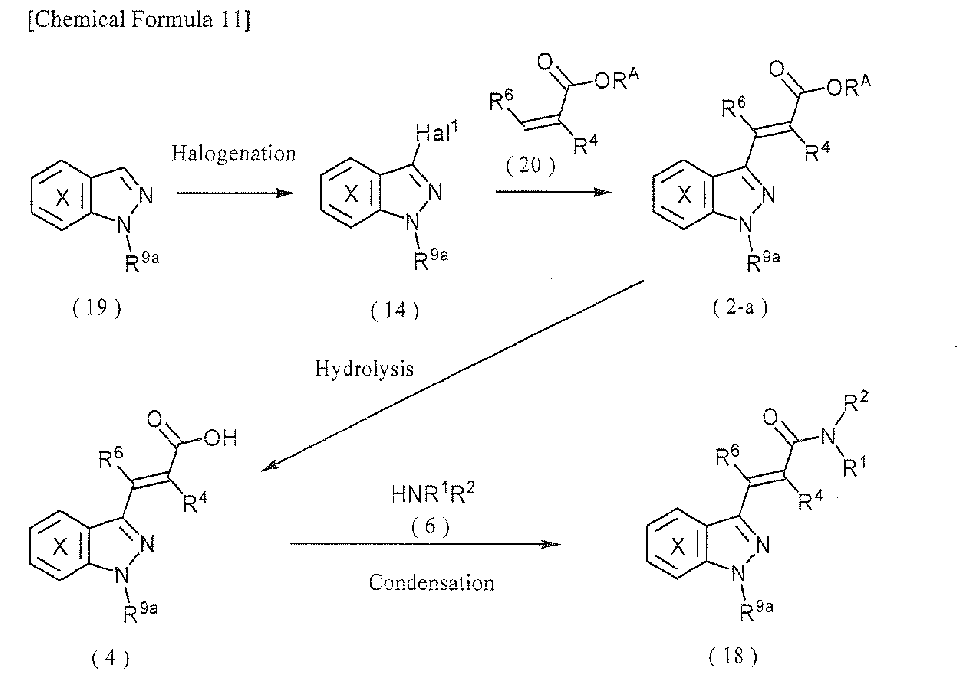 wherein each symbol has the same meaning as defined above.
Compound (14) is prepared according to the method described in Tetrahedron 55 (1999) 6917-6922, for example. Specifically, Compound (19) is reacted with halogen (including bromine, iodine) in a solvent (including DMF, DMSO) in the presence of an alkali (including potassium hydroxide, sodium hydroxide, potassium carbonate, sodium carbonate, potassium hydrogencarbonate, sodium hydrogencarbonate) or an organic base (including triethylamirle, diisopropylethylamine) under ice-cooling to room temperature for 30 minutes to 5 hours to give Compound (14).
Compound (2-a) is prepared according to the method described in Tetrahedron Lett. 41 (2000) 4363-4366 and Journal of the American Chemical Society, 1968, 90, 5518-5526, for example. Specifically, Compound (14) and Compound (20) are reacted in a solvent (including DMF, DMSO, dioxane, THF, diethyl ether, acetonitrile, methanol, ethanol, acetone, isopropanol) in the presence of a catalyst (including palladium acetate-triphenylphosphine, dichloro[1,1'-bis(diphenylphosphino)ferrocene]palladium (II)) and a base (including triethylamine, tetrabutylammonium iodide) to give Compound (2-a).
The hydrolysis of Compound (2-a) and the condensation of Compound (4) may be carried out in the similar manner to Method 1.

Method 8: Compound (2-a) is also prepared according to the following method. 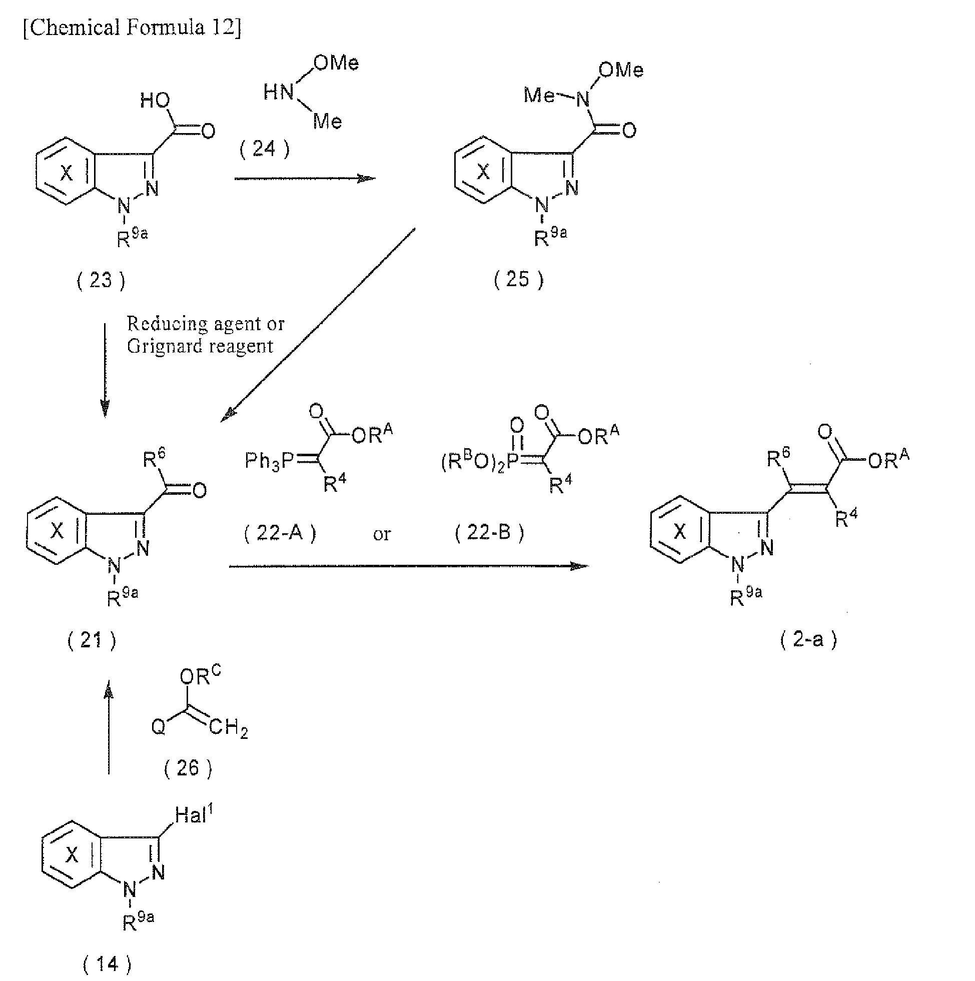 wherein R^{B} is alkyl or aryl, R^{A} and R^{C} are each alkyl, Q is hydrogen, -B(OH)₂, -B(OR^{D})(OR^{B}) or -Sn(R^{F})₃, R^{D} and R^{E} are each alkyl, or R^{D} and R^{E} combine each other to form straight or branched chain alkylene, R^{F} is alkyl, and the other symbols have the same meanings as defined above.
Compound (21) is prepared according to the following methods (1) to (3).
(1) Compound (14) is reacted with Compound (26) in a solvent (including DME, THF, 1,4-dioxane, DMF, DMA, toluene, benzene, water or a mixture thereof) in the presence of a palladium catalyst [i.e., a palladium catalyst including tetrakis(triphenylphosphine)palladium (0), bis(triphenylphosphine)palladium (II) chloride, palladium (II) acetate] at room temperature to a reflux temperature of the solvent for an hour to 3 days, and then treated with an acid (including hydrochloric acid, sulfuric acid) to give Compound (21) wherein R⁶ is methyl.
When Compound (26) wherein Q is -B(OH)₂ or -B(OR^{D})(OR^{E}) is used, a base is preferably added. For example, an inorganic base including alkali metal carbonate, alkali metal hydroxide, alkali metal phosphate, alkali metal fluoride, or an organic base including triethylamine may be used as the base.
When Compound (26) wherein Q is hydrogen is used, a ligand and a salt or a base are preferably added. For example, 1,3-bis(diphenylphosphino)propane, 1,1"-bis(diphenylphosphino)ferrocene, methyldiphenylphosphine as the ligand, and a metal salt including silver nitrate, thallium acetate or an organic base including triethylamine as the salt or the base may be used.
(2) Compound (23), or Compound (25), which is obtained by the condensation of Compound (23) and Compound (24) using a condensing agent (including DCC, WSC) according to the conventional method, is reduced by a reducing agent (including lithium aluminum hydride, sodium borohydride) in a solvent (including dioxane, THF, diethyl ether) at 0 to 100°C for 1 to 24 hours to give Compound (21) wherein R⁶ is hydrogen.
(3) Compound (25) is reacted with R⁶MgHal (wherein each symbol has the same meaning as defined above) in a solvent (including dioxane, THF, diethyl ether) under ice-cooling to room temperature for 1 to 12 hours to give Compound (21) wherein R⁶ is alkyl.
Compound (21) is reacted with Compound (22-A) in a solvent (including dioxane, THF, diethyl ether) at room temperature to a reflux temperature of the solvent for 1 to 12 hours to give Compound (2-a).
Compound (21) is also reacted with Compound (22-B) in a solvent (including dioxane, THF, diethyl ether) in the presence of a base (including sodium hydride, lithium diisopropylamide, n-butyllithium) at room temperature to a reflux temperature of the solvent for 1 to 12 hours to give Compound (2-a).

Method 9: Compound (2-b) wherein R⁴ is hydrogen and R⁶ is alkyl is prepared according to the following method. 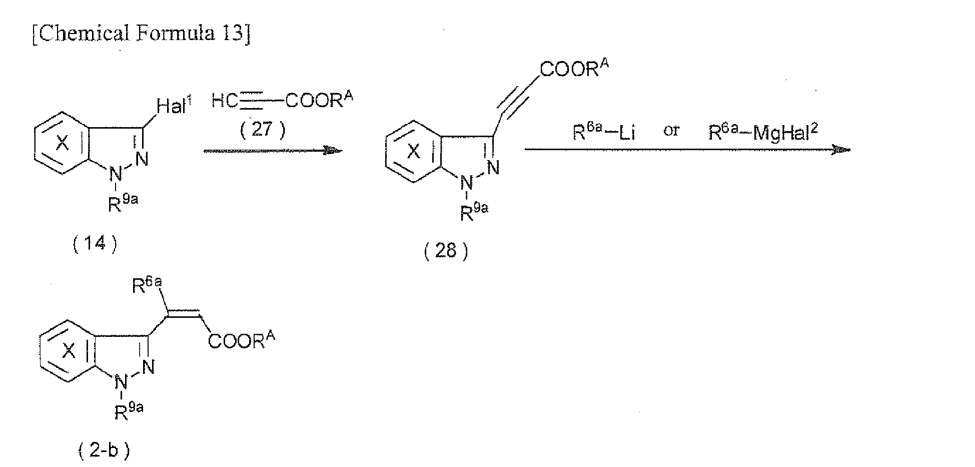 wherein R^{6a} is alkyl, Hal² is chlorine or bromine, and the other symbols have the same meanings as defined above.
Compound (14) and Compound (27) are treated with a palladium catalyst (including dichlorobistriphenylphosphine palladium, tetrakistriphenylphosphine palladium, palladium acetate, trisdibenzylideneacetone dipalladium) in a solvent (including DMSO, DMF, THF, 1,4-dioxane, diethyl ether, acetonitrile, toluene) in the presence of copper (I) iodide and a base (including sodium hydrogencarbonate, potassium hydrogencarbonate, sodium hydroxide, potassium hydroxide, triethylamine, diisopropylethylamine) at 0°C to 100°C for 1 to 24 hours to give Compound (28).
Compound (28) is reacted with R^{6a}-Li or R^{6a}-MgHal² in a solvent (including THF, 1,4-dioxane, diethyl ether, benzene, toluene, xylene, cyclohexane) in the presence of a monovalent copper salt (including copper (I) iodide, copper (I) bromide, copper (I) cyanide) at -78°C to room temperature for 1 to 24 hours to give Compound (2-b).

Method 10: In Method 7, the following Compound (20'): 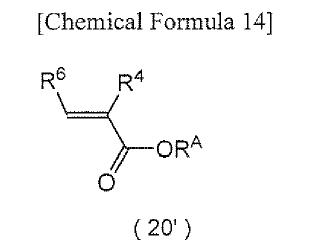 is used instead of Compound (20) to prepare Compound (2'): 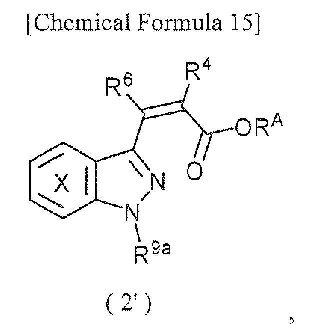 and the Compound (2') may be also used to carry out the above reaction.

Method 11: Compound (19-a) wherein R^{9a} is hydrogen and the formula: 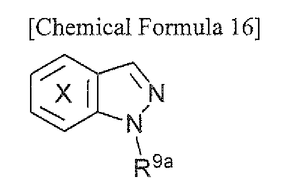 is the fonnula: 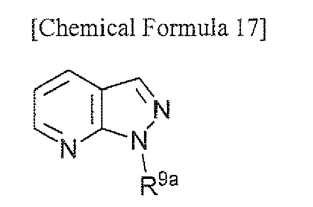 is prepared according to the following method. 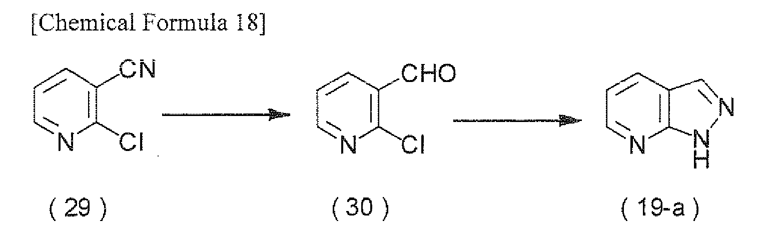 Compound (30) is prepared according to the method described in Chem. Pharm. Bull., 50(8), 1066 (2002). Specifically, Compound (29) is reduced by a reducing agent (including diisobutylaluminum hydride, sodium triacetoxyborohydride, sodium cyanoborohydride, lithium aluminum hydride, sodium borohydride) in a solvent (including benzene, toluene, xylene) at -78°C to room temperature for 1 to 24 hours to give Compound (30).
Compound (19-a) is prepared according to the method described in Chemical Communications 293-294 (1966). Specifically, Compound (30) is reacted with hydrazine or a hydrate thereof in a solvent (including methanol, ethanol, isopropanol), if needed, in the presence of a catalyst (including p-toluenesulfonic acid) at room temperature to under heating for 1 to 48 hours to give Compound (19-a).

Method 12: In the above methods, when the compound of the present invention, intermediates thereof or starting compounds have functional groups (including hydroxyl, amino, carboxy), they may be protected by a conventional protective group used in the organic synthetic chemistry according to the method described in Theodora W. Greene, Peter G. M. Wuts, "Protective Groups in Organic Synthesis" 3rd. ed., John Wiley & Sons, Inc., 1999, and a removal of the protective group after reaction may give the objective compound. The protective group includes a conventional protective group used in the organic synthetic chemistry described in said literature. Specifically, a protective group of hydroxyl includes, for example, tetrahydropyranyl, trimethylsilyl, t-butyldimethylsilyl, benzyl, 4-methoxybenzyl, methoxymethyl, acetyl, that of amino includes, for example, t-butoxycarbonyl, benzyloxycarbonyl, 9-fluorenylmethoxycarbonyl, 2,2,2-trichloroethoxycarbonyl, t-amyloxycarbonyl, 4-methoxybenzyl, 2-nitrobenzenesulfonyl, 2,4-dinitrobenzenesulfonyl, and that of carboxy includes, for example, alkyl such as methyl, ethyl, t-butyl, and benzyl.
The compound of the present invention or intermediates thereof may be prepared according to the above methods, and then the functional groups may be converted or modified according to a conventional manner. Specifically, the following methods are included.

### (1) Conversion of amino into amide

Amino may be converted into the corresponding amide by reacting amino with acyl halide, or by reacting carboxy with amine in the presence of a condensing agent.

### (2) Conversion of carboxy or esters thereof into carbamoyl

Carboxy may be converted into the corresponding carbamoyl by converting carboxy or a salt thereof into acyl halide followed by reacting with amine, by reacting carboxy or a salt thereof with amine in the presence of a condensing agent, or by reacting an ester thereof with amine.

### (3) Hydrolysis of ester

Ester may be converted into the corresponding carboxy or a salt thereof by hydrolyzing ester with alkali hydroxide (including sodium hydroxide, potassium hydroxide) or an acid (including hydrochloric acid, sulfuric acid), or by hydrogenating ester with a metal catalyst.

### (4) N-Alkylation, N-phenylation, N-benzylation

Amino may be converted into the corresponding mono- or di-alkyl-substituted amino, phenyl-substituted amino or benzyl-substituted amino by reacting amino with alkyl halide, phenol halide or benzyl halide. Amino may be also converted into the corresponding mono- or di-alkyl-substituted amino or benzyl-substituted amino by a reductive amination of amino.

### (5) N-Sulfonylation

Amino may be converted into the corresponding alkyl sulfonylamino or phenyl sulfonylamino by reacting amino with alkyl sulfonyl halide or phenyl sulfonyl halide.

### (6) Conversion of amino into ureido

Amino may be converted into alkyl ureido by reacting amino with alkyl isocyanate or carbamoyl halide. Amino may be also converted into ureido by converting amino into isocyanate, carbamoyl halide or carbamate followed by reacting with amine.

### (7) Conversion of amino into carbamate

Amino may be converted into carbamate by reacting amino with alkyl halocarbonate (including methyl chlorocarbonate, ethyl chlorocarbonate), or by converting amino into isocyanate followed by reacting with alcohol.

### (8) Conversion of amino into 3-aminopropionyl or 2-aminoethylsulfonyl

Amino may be converted into the corresponding 3-aminopropionyl or 2-aminoethylsulfonyl by Michael reaction with 2,3-unsaturated carbonyl compound or vinyl sulfonyl compound.

### (9) Conversion of carbamoyl into benzylamine

Carbamoyl may be converted into the corresponding benzylamine by reacting with a reducing agent (including lithium aluminum hydride).

### (10) Conversion of hydroxyl into carbamoyloxy

Hydroxyl may be converted into the corresponding carbamoyloxy by treating with N,N'-disuccinimidyl carbonate.

### (11) Conversion of aromatic nitro into aromatic amine

Aromatic nitro may be converted into aromatic amine by treating with a reducing agent [e.g., a metal reducing agent such as sodium borohydride, lithium borohydride, lithium aluminum hydride, a reduction by a metal (e.g., iron, zinc, tin, tin (II) chloride, titanium, titanium trichloride), a catalytic reduction with a transition metal (e.g., palladium-carbon, platinum, Raney nickel)] according to a conventional manner. In the catalytic reduction, ammonium formate, hydrazine, etc. may be also used as a hydrogen source.

The compound of the present invention and each intermediate thereof which are prepared in the above methods may be purified by a conventional method, for example, chromatography, recrystallization, etc. A solvent for recrystallization includes, for example, an alcohol solvent such as methanol, ethanol or 2-propanol, an ether solvent such as diethyl ether, diisopropyl ether or THF, an ester solvent such as ethyl acetate, an aromatic solvent such as toluene, a ketone solvent such as acetone, a hydrocarbon solvent such as hexane, water, or a mixed solvent thereof. The compound of the present invention may be converted into a pharmaceutically acceptable salt thereof according to a conventional manner, and then recrystallized.

### EFFECT OF INVENTION

The compound of the present invention or a pharmaceutically acceptable salt thereof has I_{Kur} blocking activity, and is useful for preventing or treating cardiac arrhythmia such as atrial fibrillation, atrial flutter, atrial arrhythmia, supraventricular tachycardia in a mammal. The compound of the present invention or a pharmaceutically acceptable salt thereof is also useful for preventing thromboembolism including apoplexy, heart failure including congestive heart failure.

The compound of the present invention or a pharmaceutically acceptable salt thereof may be formulated into a pharmaceutical composition comprising a therapeutically effective amount of said compound or a pharmaceutically acceptable salt thereof and pharmaceutically acceptable carriers. The pharmaceutically acceptable carrier may include a diluent, a binder (e.g., syrup, gum arabic, gelatine, sorbit, tragacanth, polyvinylpyrrolidone), an excipient (e.g., lactose, sucrose, cornstarch, potassium phosphate, sorbit, glycine), a lubricant (e.g., magnesium stearate, talc, polyethylene glycol, silica), a disintegrant (e.g., potato starch) and a wetting agent (e.g., sodium lauryl sulfate), etc.
The compound of the present invention or a pharmaceutically acceptable salt thereof may be administered orally or parenterally, and used in the form of an appropriate pharmaceutical formulation. The appropriate pharmaceutical formulation for an oral administration includes, for example, a solid formulation such as tablet, granule, capsule, powder, or a liquid formulation, a suspension or an emulsifier. The appropriate pharmaceutical formulation for a parenteral administration includes a suppository, an injectable solution or an intravenous fluid preparation using distilled water for injection, physiological saline or aqueous glucose solution, or an inhalation.
The dose of the compound of the present invention or a pharmaceutically acceptable salt thereof varies depending on administration routes, ages, body weights or conditions of patients, but is usually about 0.003 to 100 mg/kg, preferably about 0.01 to 30 mg/kg, particularly about 0.05 to 10 mg/kg, per day.

### BEST MODE FOR CARRYING OUT THE INVENTION

The present invention is further specifically illustrated by Examples and Reference Examples as below, but is not limited thereto,

### EXAMPLES

### Example 1-1

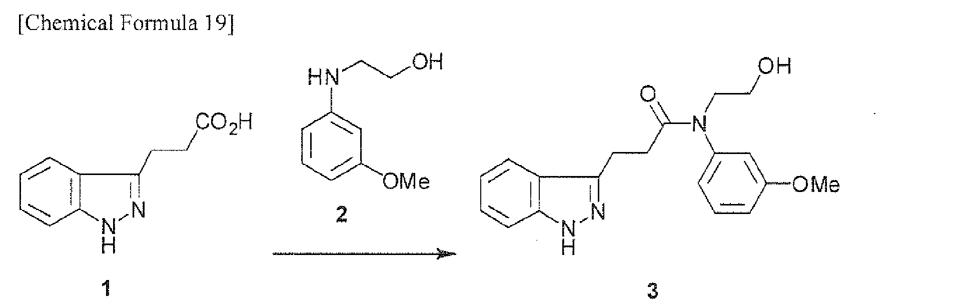

To a solution of Compound 1 (150 mg) and triphenylphosphine (228 mg) in THF (9 ml) was added N-chlorosuccinimide (126 mg) under ice-cooling, and the mixture was stirred for 25 minutes. Then, thereto was added a solution of Compound 2 (237 mg) in THF (1 ml), and the mixture was stirred at room temperature for 3 days. To the reaction mixture was added an aqueous solution of citric acid, and the mixture was extracted with chloroform. The extracted layer was washed with saturated saline, and dried over anhydrous sodium sulfate. The solvent was distilled away under reduced pressure, and the resulting residue was purified by silica gel column chromatography (eluent chloroform-methanol 98:2) to give Compound 3 (105 mg) as a colorless oily substance.
MS (APCI) 340 [M+H]⁺

### Examples 1-2 to 1-22

The corresponding starting compounds were reacted and treated in the similar manner to the above Example to give compounds of Examples 1-2 to 1-22.

### Example 1-23

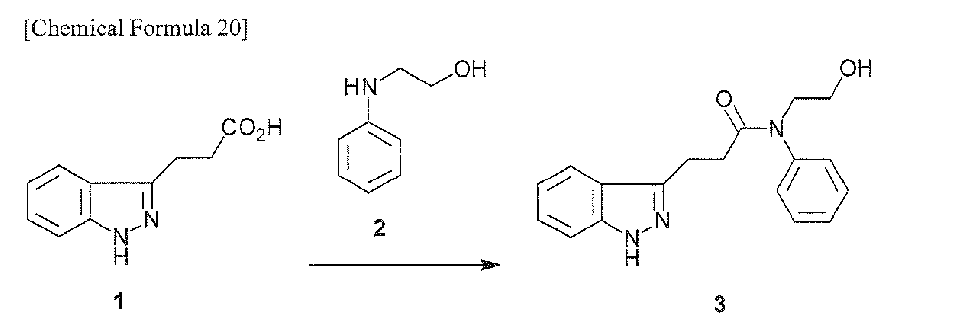

Compound 1 (200 mg) was suspended in chloroform (10 ml), and then thereto were added triethylamine (220 µl), Compound 2 (393 µl) and diethyl cyanophosphonate (239 µl) with stirring under ice-cooling, and the mixture was stirred at room temperature for 2 hours. To the reaction mixture was added an aqueous solution of citric acid, and the mixture was extracted with chloroform. The extracted layer was washed with saturated sodium bicarbonate water, and dried over anhydrous sodium sulfate. The solvent was distilled away under reduced pressure, and the resulting residue was purified by silica gel column chromatography (eluent chloroform-methanol 98:2 to 92:8 gradient) to give Compound 3 (34 mg) as a pale yellow solid. MS (APCI) 310 [M+H]⁺

### Example 1-24

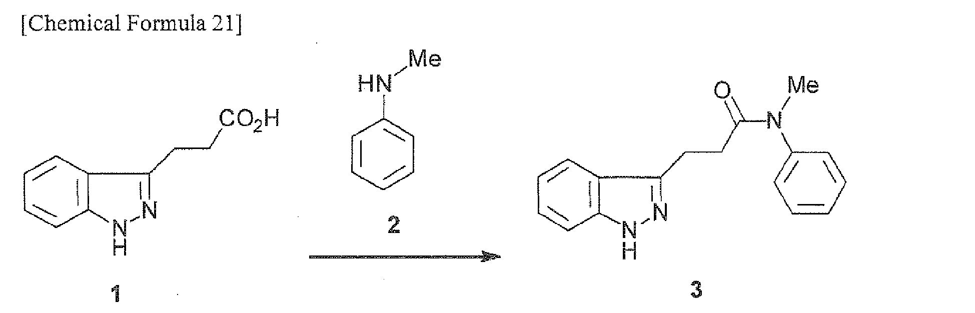

To a solution of Compound 1 (130 mg) and HOBt (120 mg) in DMF (3.4 ml) were added Compound 2 (111 µl) and WSC (170 mg) with stirring under ice-cooling, and the mixture was stirred at room temperature for 4 hours. To the reaction mixture was added an aqueous solution of sodium bicarbonate, and the mixture was extracted with ethyl acetate. The extracted layer was washed with saturated saline, and dried over anhydrous sodium sulfate. The solvent was distilled away under reduced pressure, and the residue was purified by silica gel column chromatography (eluent chloroform-methanol 99:1 to 94:6 gradient) to give Compound 3 (75 mg) as a colorless powder.
MS (APCI) 280 [M+H]⁺

### Example 2-1

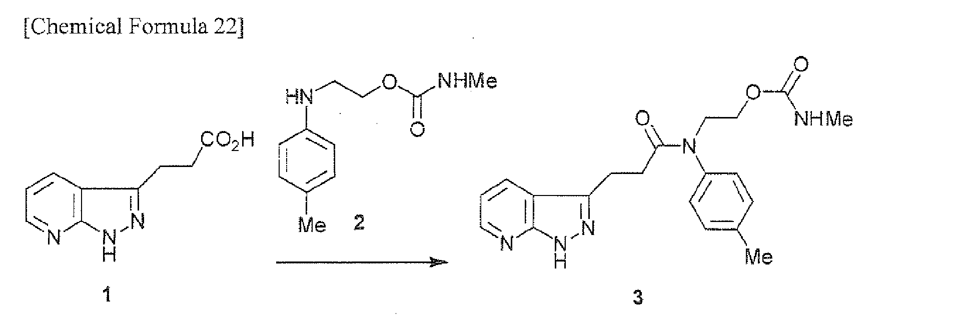

To a solution of Compound 1 (50.0 mg) in THF (6 ml) were added triphenylphosphine (123.5 mg), N-chlorosuccinimide (59.4 mg) and THF (1 ml) at room temperature, and the mixture was stirred for 2 minutes. Then, thereto were added N,N-dimethylaniline (83 µl) and a solution of Compound 2 (70.8 mg) in THF (1 ml), and the mxiture was heated to refulx for 2 days. The reaction mixture was let stand to cool, and then thereto was added ethyl acetate. Then, the mixture was washed with water. The organic layer was washed with 2N-aqueous sodium hydroxide solution and saturated saline, and dried over anhydrous sodium sulfate. The solvent was distilled away under reduced pressure, and the resulting residue was purified by silica gel column chromatography (eluent chloroform-methanol 100:0 to 90:10 gradient) to give Compound. 3 (83.4 mg) as a colorless powder.
MS (APCI) 382 [M+H]⁺

### Examples 2-2 to 2-25

The corresponding starting compounds were reacted and treated in the similar manner to the above Example to give compounds of Examples 2-2 to 2-25.

### Example 2-26

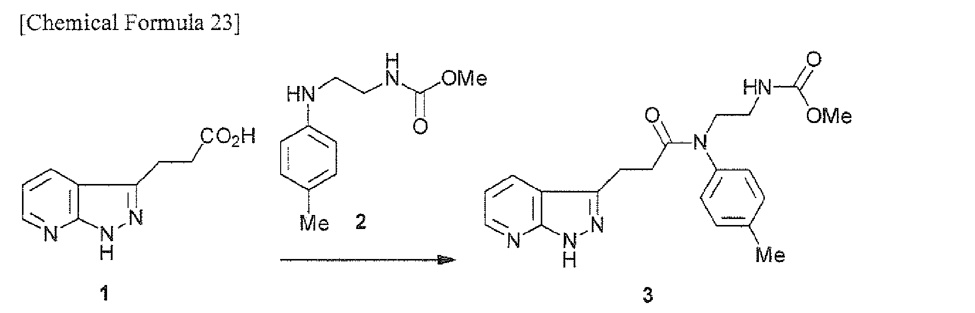

Compound 1 (40 mg) was suspended in methylene chloride (6 ml), and then thereto were added DMF (15 µl) and oxalyl chloride (57 µl), and the mixture was heated to reflux for an hour. The reaction solution was cooled, and then concentrated under reduced pressure. The residue was suspended in chloroform (6 ml), and then thereto was added a solution of Compound 2 (131 mg) in chloroform (2 ml), and the mixture was stirred at room temperature for 30 minutes. To the reaction mixture was added water, and the mixture was separated. The organic layer was dried over anhydrous sodium sulfate, and then the solvent was distilled away under reduced pressure. To the residue were added THF (5 ml), methanol (1 ml) and 1N-aqueous sodium hydroxide solution (210 µl), and the mixture was stirred at room temperature for 15 minutes. The reaction mixture was diluted with ethyl acetate, and then washed with water and saturated saline, and dried over anhydrous sodium sulfate. The solvent was distilled away under reduced pressure, and the resulting residue was purified by silica gel column chromatography (eluent chloroform-methanol 100:0 to 95:5 gradient), and then the resulting solid was triturated by a mixed solvent of ethyl acetate-n-hexane to give Compound 3 (55.5 mg) as a colorless powder.
MS (APCI) 382 [M+H]⁺

### Examples 2-27 to 2-38

The corresponding starting compounds were reacted and treated in the similar manner to the above Example to give compounds of Examples 2-27 to 2-38.

### Example 3-1

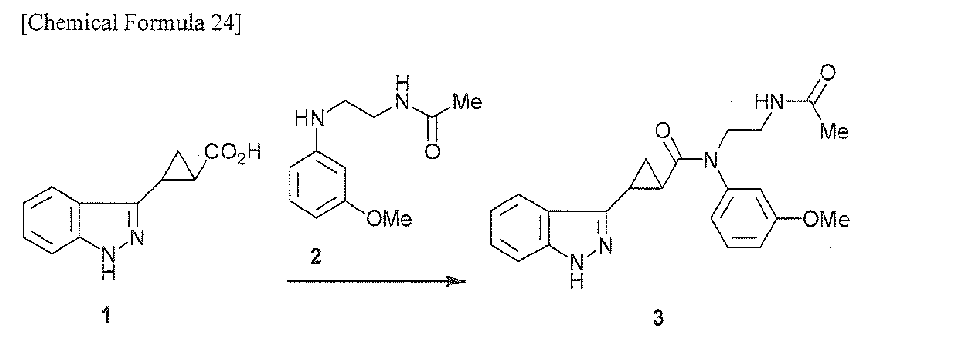

To a solution of Compound 1 (40 mag) and triphenylphosphine (57 mg) in THF (3 ml) was added N-chlorosuccinimide (32 mg), and the mixture was stirred at room temperature for a minute, and then thereto were added a solution of Compound 2 (45 mg) and diisopropylethylamine (69 µl) in THF (1 ml), and the mixture was stirred at room temperature for 16 hours. To the reaction mixture were added triphenylphosphine (26 mg) and N-chlorosuccinimide (13 mg), and the mixture was stirred at room temperature for 4 hours. To the reaction mixture were added triphenylphosphine (26 mg) and N-chlorosuccinimide (13 mg), and the mixture was stirred at room temperature for 19 hours. The reaction mixture was diluted with ethyl acetate, and then washed with water, saturated sodium bicarbonate water and saturated saline, and dried over anhydrous sodium sulfate. The solvent was distilled away under reduced pressure, and the resulting residue was purified by silica gel column chromatography (eluent chloroform-methanol 100:0 to 95:5 gradient) to give Compound 3
(57.4 mg) as a yellow amorphous powder.
MS (APCI) 393 [M+H]⁺

### Example 3-2

The corresponding starting compounds were reacted and treated in the similar manner to the above Example to give compounds of Example 3-2.

### Example 4-1

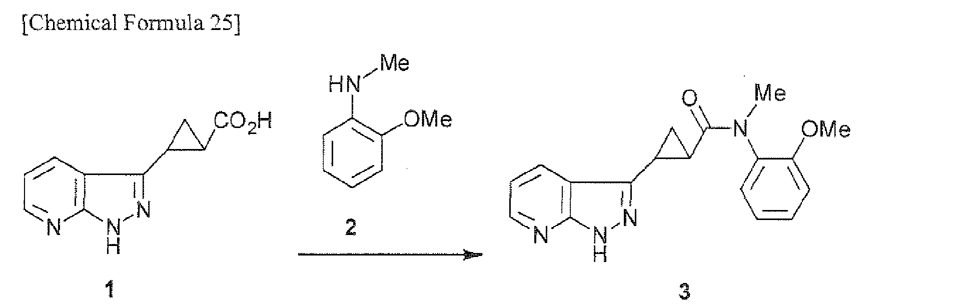

To a solution of Compound 1 (50 mg) and triphenylphosphine (110 mg) in THF (11 ml) were added N-chlorosuccinimide (49 mg) and THF (1 ml), and the mixture was stirred at room temperature for 5 minutes, and then thereto was added a solution of Compound 2 (37 mg) and N,N-dimethylaniline (62 µl) in THF (1 ml), and the mixture was stirred at room temperature for 18 hours. To the reaction mixture were added triphenylphosphine (32 mg) and N-chlorosuccinimide (16 mg), and the mixture was stirred at room temperature for 2 hours. The reaction mixture was diluted with ethyl acetate, and then washed with water, 2N-aqueous sodium hydroxide solution and saturated saline, and dried over anhydrous sodium sulfate. The solvent was distilled away under reduced pressure, and the resulting residue was purified by silica gel column chromatography (eluent chloroform-methanol 97:3 to 94:6 gradient) to give Compound 3 (41.7 mg) as a colorless amorphous powder.
MS (APCI) 323 [M+H]⁺

### Examples 4-2 to 4-8

The corresponding starting compounds were reacted and treated in the similar manner to the above Example to give compounds of Examples 4-2 to 4-8.

### Example 5-1

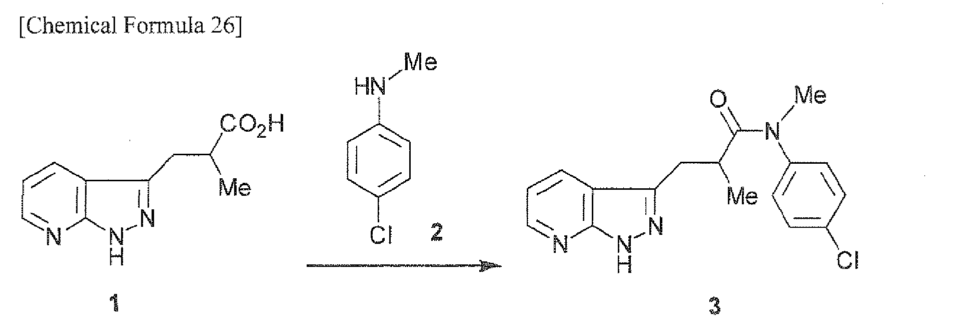

To a solution of Compound 1 (30 mg) and triphenylphosphine (61 mg) in THF (4 ml) was added N-chlorosuccinimide (29 mg), and the mixture was stirred at room temperature for 5 minutes, and then thereto was added Compound 2 (72 mg). The reaction mixture was irradiated with microwave, and stirred at 150°C for 8 hours. The reaction mixture was cooled, and then concentrated under reduced pressure. To the residue was added chloroform, and the mixture was washed with 1N-aqueous sodium hydroxide solution and saturated saline, and dried over anhydrous sodium sulfate. The solvent was distilled away under reduced pressure, and the resulting residue was purified by silica gel column chromatography (eluent chloroform-methanol 100:0 to 95:5 gradient) to give Compound 3 (29.3 mg) as a colorless oily substance.
MS (APCI) 329/331 [M+H]⁺

### Examples 5-2 to 5-7

The corresponding starting compounds were reacted and treated in the similar manner to the above Example to give compounds of Examples 5-2 to 5-7.

### Example 5-8

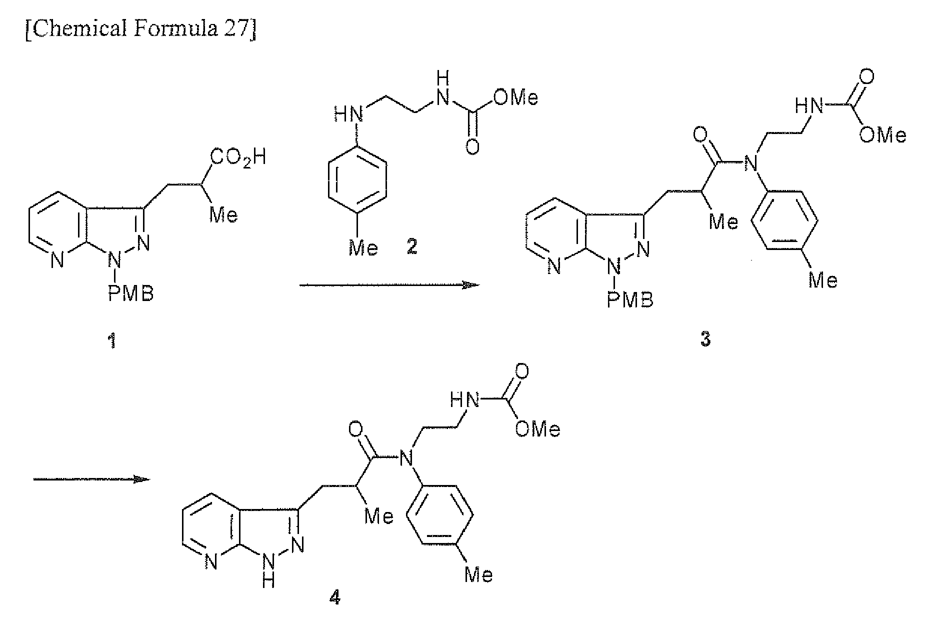

(1) To a solution of Compound 1 (100 mg) and triphenylphosphine (113 mg) in THF (5 ml) was added N-chlorosuccinimide (53 mg), and the mixture was stirred at room temperature for 15 minutes, and then thereto was added a solution of Compound 2 (70 mg) and N,N-dimethylaniline (93 mg) in THF (2 ml), and the mixture was stirred at room temperature for 4 hours. The reaction mixture was concentrated under reduced pressure, and then the resulting residue was purified by silica gel column chromatography (eluent chloroform-methanol 100:0 to 95:5 gradient) to give Compound 3 (122 mg) as a colorless oily substance.
   MS (APCI) 516 [M+H]⁺
(2) Compound 3 (120 mg) was suspended in trifluoroacetic acid (4 ml), and heated to reflux for 3 hours. The reaction mixture was cooled, and concentrated under reduced pressure. To the resulting residue was added a mixed solvent of methanol-ethanol (3:1), and the insoluble was filtered off and washed with a mixed solvent of methanol-ethanol (3:1). The filtrate was concentrated under reduced pressure, and then the resulgint residue was purified by silica gel column chromatography (eluent chloroform-methanol 100:0 to 95:5 gradient) to give Compound 4 (92 mg) as a colorless powder.
MS (APCI) 396[M+H]⁺

### Example 5-9

The corresponding starting compounds were reacted and treated in the similar manner to the above Example to give a compound of Example 5-9.

### Example 6-1

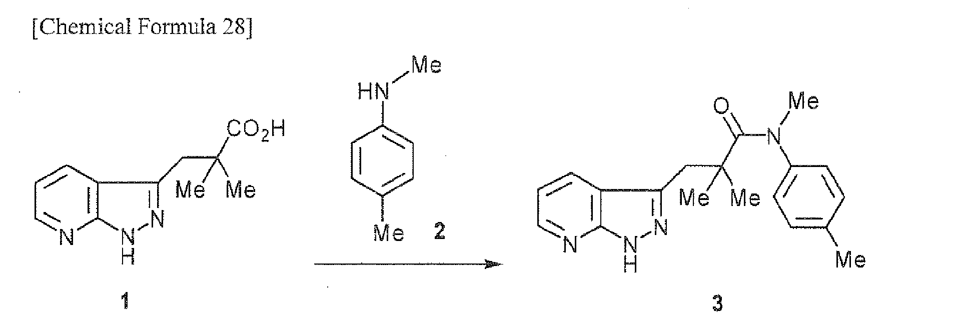

To a solution of Compound 1 (90 mg) and triphenylphosphine (172 mg) in THF (11 ml) were added N-chlorosuccinimide (82 mg), Compound 2 (182 µl) and THF (1 ml) at room temperature. The reaction mixture was irradiated with microwave, and stirred at 130°C for 10 hours. The reaction mixture was cooled, and then diluted with ethyl acetate, washed with 1N-aqueous sodium hydroxide solution and saturated saline, and dried over anhydrous sodium sulfate. The solvent was distilled away under reduced pressure, and the resulting residue was purified by NH silica gel column chromatography (eluent chloroform-methanol 99:1 to 98:2 gradient), then silica gel column chromatography (eluent ethyl acetate-methanol 99:1 to 98:2 gradient) to give Compound 3(54 mg) as a colorless oily substance.
MS (APCI) 323 [M+H]⁺

### Examples 6-2 to 6-5

The corresponding starting compounds were reacted and treated in the similar manner to the above Example to give compounds of Examples 6-2 to 6-5.

### Example 7-1

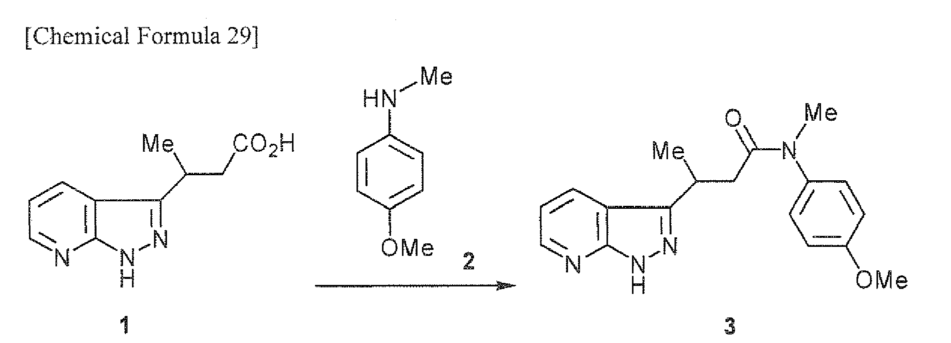

To a solution of Compound 1 (50 mg) and triphenylphosphine (115 mg) in THF (5.5 ml) were added N-chlorosuccinimide (55 mg) and THF (0.5 ml), and the mixture was stirred at room temperature for 2 minutes. Then, thereto was added a solution of Compound 2 (43 mg) and N,N-dimethylaniline (77 µl) in THF (1 ml), and the mixture was heated to reflux for an hour. The reaction mixture was cooled, and then diluted with ethyl acetate, washed with water, 2N-aqueous sodium hydroxide solution and saturated saline, and dried over anhydrous sodium sulfate. The solvent was distilled away under reduced pressure, and the resulting residue was purified by silica gel column chromatography (eluent chloroform methanol 98:2 to 91:9 gradient) to give Compound 3 (67 mg) as a purple oily substance.
MS (APCI) 325 [M+H]⁺

### Example 7-2

The corresponding starting compounds were reacted and treated in the similar manner to the above Example to give a compound of Example 7-2.

### Example 8-1

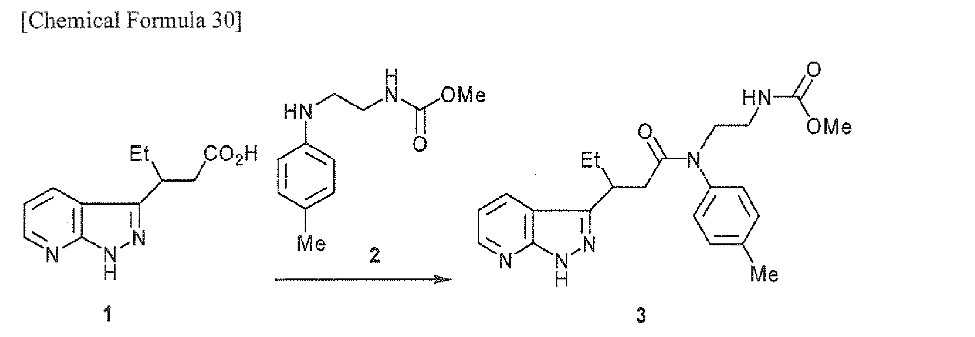

To a solution of Compound 1 (40 mg) and triphenylphosphine (76 mg) in THF (2.5 ml) was added N-chlorosuccinimide (36 mg) at room temperature, and then thereto was added a solution of Compound 2 (42 mg) and N,N-dimethylaniline (44 mg) in THF (0.5 ml). The reaction mixture was irradiated with microwave, and stirred at 150°C for 5 hours. The reaction mixture was cooled, and then concentrated under reduced pressure. To the residue was added chloroform, and the mixture was washed with 1N-aqueous sodium hydroxide solution and saturated saline and dried over anhydrous sodium sulfate. The solvent was distilled away under reduced pressure, and the resulting residue was purified by silica gel column chromatography (eluent chloroform-methanol 100:0 to 95:5 gradient) to give Compound 3 (30.4 mg) as a colorless oily substance.
MS (APCI) 410 [M+H]⁺

### Example 9-1

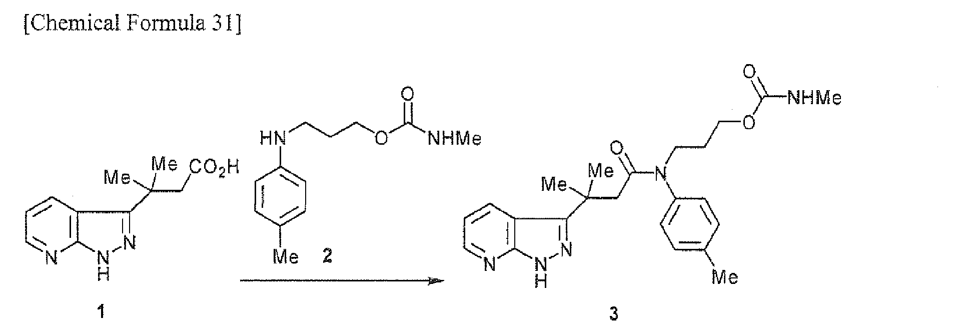

To a solution of Compound 1 (60 mg) and triphenylphosphine (129 mg) in THF (7 ml) were added N-chlorosuccinimide (62 mg) and THF (0.5 ml) with stirring at room temperature, and the mixture was stirred for 2 minutes. Then, thereto was added a solution of N,N-dimethylaniline (89 µl) and Compound 2 (79 mg) in THF (0.5 ml), and the mixture was heated to reflux for 2 days. The reaction mixture was let stand to cool, and then diluted with ethyl acetate, washed with 2N-aqueous sodium hydroxide solution and saturated saline, and dried over anhydrous sodium sulfate. The solvent was distilled away under reduced pressure, and the resulting residue was purified by silica gel column chromatography (eluent chloroform-methanol 98:2 to 91:9 gradient) to give Compound 3 (93 mg) as a colorless oily substance.
MS (APCI) 424 [M+H]⁺

### Examples 9-2 to 9-27

The corresponding starting compounds were reacted and treated in the similar manner to the above Example to give compounds of Examples 9-2 to 9-27.

### Example 9-28

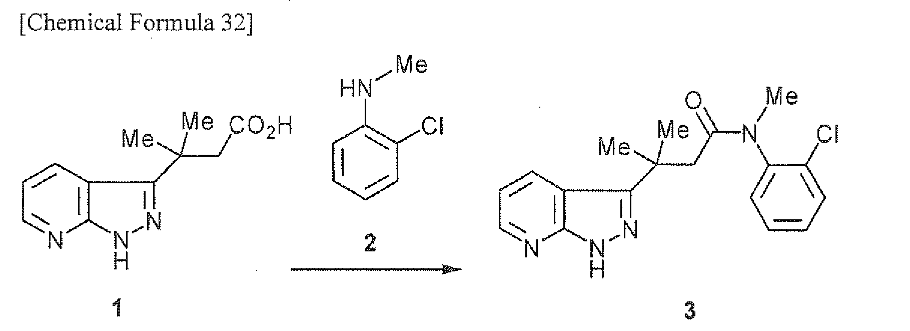

Compound 1 (80 mg) was suspended in methylene chloride (12 ml), and then thereto were added DMF (9 µl) and oxalyl chloride (94 µl), and the mixture was heated to reflux for an hour. The reaction solution was cooled, and then concentrated under reduced pressure. The residue was suspended in chloroform (18 ml), and then thereto was added Compound 2 (180 µl). The mixture was stirred at room temperature for 90 minutes. To the reaction mixture was added water, and the mixture was separated, and then the solvent of the organic layer was distilled away under reduced pressure. To the residue were added THF (5 ml), methanol (1 ml) and 1N-aqueous sodium hydroxide solution (550 µl), and the mixture was stirred at room temperature for an hour. The reaction mixture was diluted with ethyl acetate, and then washed with water and saturated saline, and dried over anhydrous sodium sulfate. The solvent was distilled away under reduced pressure, and the resulting residue was purified by silica gel column chromatography (eluent chloroform-ethyl acetate 50:50 to 0:100 gradient), and then the resulting solid was triturated by a mixed solvent of ethyl acetate-n-hexane to give Compound. 3 (47 mg) as a colorless powder.
MS (APCI) 343/345 [M+H]⁺

### Example 9-29

The corresponding starting compounds were reacted and treated in the similar manner to the above Example to give a compound of Example 9-29.

### Example 9-30

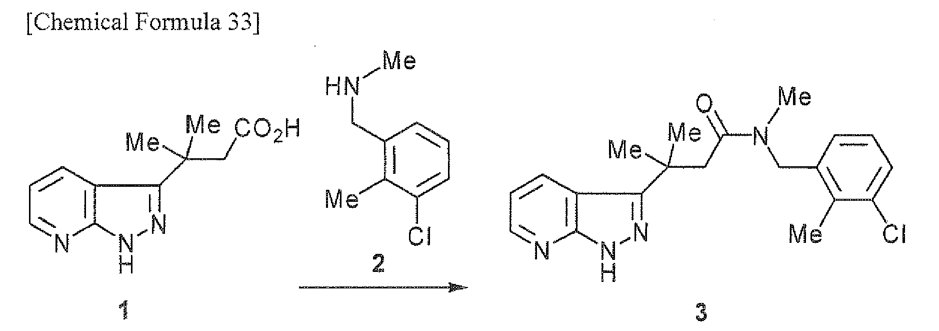

To a solution of Compound 1 (60 mg), Compound 2 (56 mg) and HOBt (48 mg) in DMF (1.5 ml) was added WSC (79 mg), and the mixture was stirred at room temperature for 4 hours. To the reaction mixture was added aqueous sodium hydrogencarbonate solution, and the mixture was extracted with ethyl acetate. The extracted layer was washed with saturated saline, and dried over anhydrous magnesium sulfate. The solvent was distilled away under reduced pressure, and the residue was purified by silica gel column chromatography (eluent chloroform-ethyl acetate 67:33 to 0:100 gradient) to give Compound 3 (79 mg) as a colorless powder.
MS (APCI) 371/373 [M+H]⁺

### Examples 9-31 to 9-33

The corresponding starting compounds were reacted and treated in the similar manner to the above Example to give compounds of Examples 9-31 to 9-33.

### Example 10-1

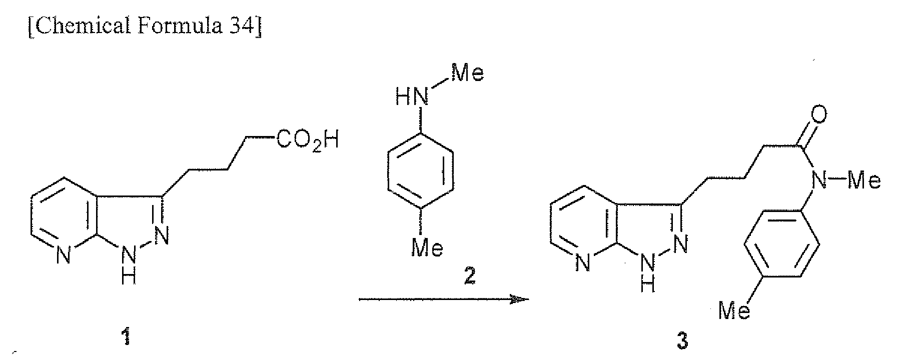

To a solution of Compound 1 (103 mg) in THF (10 ml) were added triphenylphosphine (236 mg) and N-chlorosuccinimide (114 mg) with stirring at room temperature, and the mixture was stirred for 5 minutes. Then, thereto was added a solution of N,N-dimethylaniline (182 mg) and Compound 2 (79 mg) in THF (1 ml), and the mixture was heated to reflux for 16 hours. The reaction mixture was let stand to cool, diluted with ethyl acetate, washed with water, 5%-aqueous sodium hydroxide solution and saturated saline, and dried over anhydrous sodium sulfate. The solvent was distilled away under reduced pressure, and the resulting residue was purified by silica gel column chromatography (eluent chloroform-methanol 100:0 to 95:5 gradient), and then purified by silica gel column chromatography (eluent ethyl acetate-methanol 100:0 to 95:5 gradient) to give Compound 3 (147 mg) as a colorless oily substance.
MS (APCI) 309 [M+H]⁺

### Examples 10-2 to 10-5

The corresponding starting compounds were reacted and treated in the similar manner to the above Example to give compounds of Examples 10-2 to 10-5.

### Example 11-1

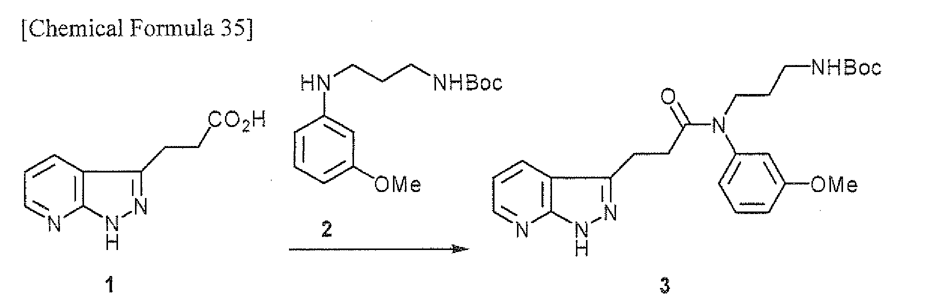

To a solution of Compound 1 (1.00 g) in THF (60 ml) were added triphenylphosphine (1.78 g) and N-chlorosuccinimide (0.84 g) with stirring at room temperature, and the mixture was stirred for 2 minutes. Then, thereto was added a solution of Compound 2 (3.67 g) in THF (30 ml), and the mixture was heated to reflux for 16 hours. The reaction mixture was let stand to cool to room temperature, and then thereto were added triphenylphosphine (0.55 g) and N-chlorosuccinimide (0.28 g), and the mixture was heated to reflux for 5 hours. The reaction mixture was let stand to cool to room temperature, and then thereto were added triphenylphosphine (0.55 g) and N-chlorosuccinimide (0.28 g), and the mixture was heated to reflux for 5 hours. The reaction mixture was let stand to cool to room temperature, and then diluted with ethyl acetate, and washed with water. The organic layer was washed with saturated sodium bicarbonate water and saturated saline, and dried over anhydrous sodium sulfate. The solvent was distilled away under reduced pressure, and the resulting residue was purified by silica gel column chromatography (eluent chloroform-methanol 100:0 to 90:10 gradient) to give Compound 3 (1.90 g) as a colorless amorphous powder.
MS (APCI) 454 [M+H]⁺

### Example 111-2

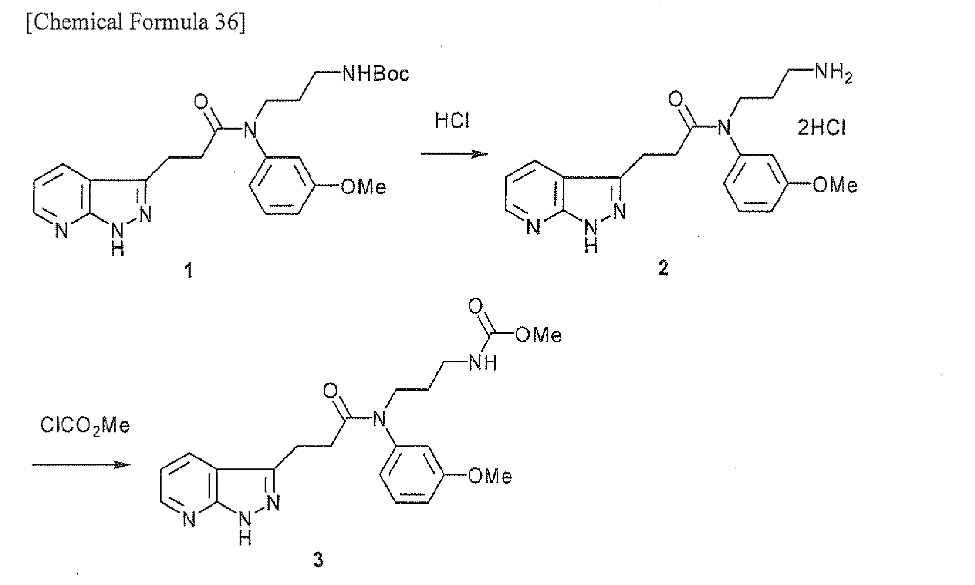

(1) Compound 1 (1.99 g) was dissolved in chloroform (30 ml) and methanol (15 ml), and then thereto was added dropwise 4N-hydrochloric acid-ethyl acetate solution (8.2 ml). The mixture was stirred at room temperature for 17 hours. To the reaction mixture was added 4N-hydrochloric acid-ethyl acetate solution (2.7 ml), and the mixture was stirred at room temperature for 9 hours. The reaction mixture was concentrated under reduced pressure, and the resulting residue was triturated by ethyl acetate to give Compound 2 (1.75 g) as a pale yellow solid.
   MS (APCI) 354 [M+H]⁺
(2) Compound 2 (50 mg) was suspended in chloroform (5 ml), and then thereto was added triethylamine (49 µl) with stirring under ice-cooling, and the mixture was stirred for 5 minutes. Then, thereto was added methyl chloroformate (9.5 µl), and the mixture was stirred under ice-cooling for 30 minutes. To the reaction mixture was added aqueous sodium hydrogencarbonate solution, and the mixture was extracted with ethyl acetate. The extracted layer was washed with saturated saline, and dried over anhydrous magnesium sulfate. The solvent was distilled away under reduced pressure, and the residue was purified by silica gel column chromatography (eluent chloroform-methanol 100:0 to 90:10 gradient) to give Compound 3 (43 mg) as a colorless solid.
   MS (APCI) 412 [M+H]⁺

### Example 11-3

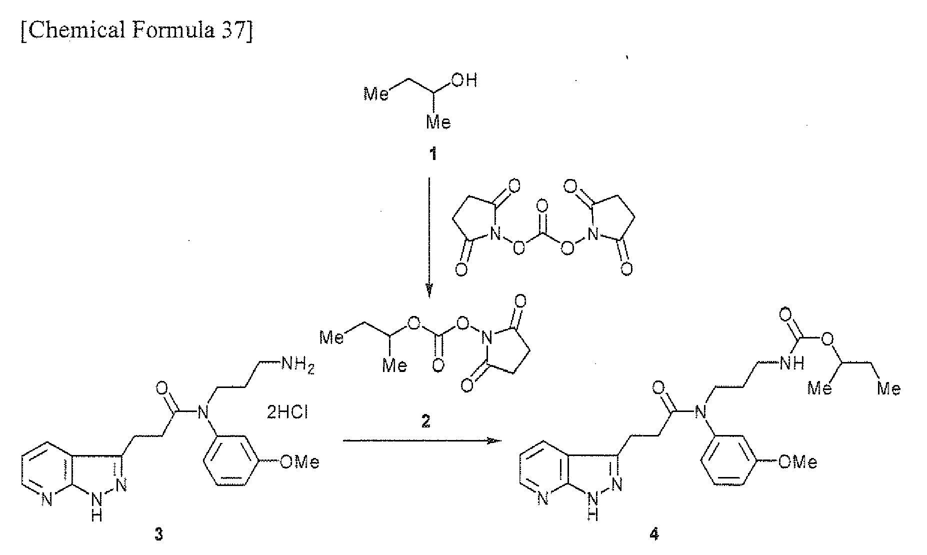

(1) Compound 1 (740 mg) and N,N'-dimethylaminopyridine (366 mg) were dissolved in a mixed solvent of acetonitrile (50 ml) and chloroform (50 ml), and then thereto was added N,N'-disuccinimidyl carbonate (2.56 g), and the mixture was stirred at room temperature for 4 hours. To the reaction mixture was added saturated sodium bicarbonate water, and the mixture was extracted with chloroform. The extracted layer was washed with saturated saline, and dried over anhydrous magnesium sulfate, and then the solvent was distilled away under reduced pressure. To the residue was added ethyl acetate, and the mixture was stirred, and then the insoluble was filtered off and washed with ethyl acetate. The solvent of the filtrate was distilled away under reduced pressure, and then the resulting residue was purified by silica gel column chromatography (eluent ethyl acetate-n-hexane 1:2) to give Compound 2 (595 mg) as a colorless solid.
   MS (APCI) 248[M+MeOH+H]⁺
(2) Compound 3 (50 mg) was suspended in chloroform (5 ml), and then thereto was added triethylamine (49 µl) with stirring under ice-cooling, and the mixture was stirred for 5 minutes.
   Then, thereto was added a solution of Compound 2 (26.5 mg) in chloroform (1 ml), and the mixture was stirred under ice-cooling for 2 hours. To the reaction mixture was added aqueous sodium hydrogencarbonate solution, and the mixture was separated, and then the organic layer was dried over anhydrous magnesium sulfate. The solvent was distilled away under reduced pressure, and the residue was purified by silica gel column chromatography (eluent chloroform-ethyl acetate 95:5 to 40:60 gradient), and then the resulting solid was triturated by a mixed solvent of ethyl acetate-n-hexane to give Compound 4 (31.6 mg) as a colorless powder.
   MS (APCI) 454 [M+H]⁺

### Example 11-4

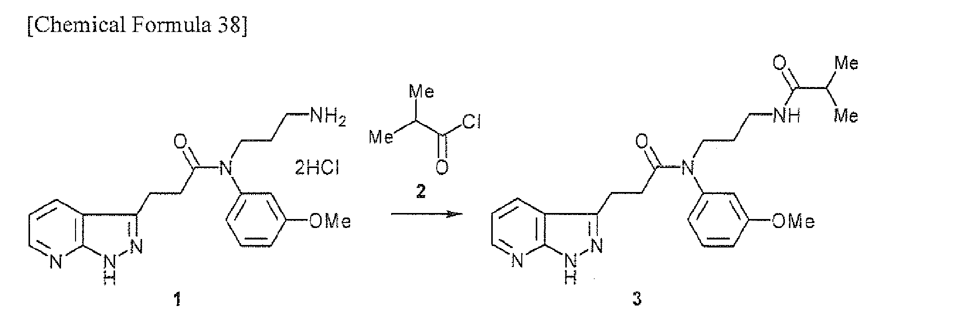

Compound 1 (50 mg) was suspended in chloroform (2 ml), and then thereto was added triethylamine (57 µl) with stirring under ice-cooling, and the mixture was stirred for 5 minutes. Then, thereto was added a solution of Compound 2 (13.1 mg) in chloroform (1 ml), and the mixture was stirred under ice-cooling for 30 minutes. To the reaction mixture was added aqueous sodium hydrogencarbonate solution, and the mixture was separated, and then the organic layer was dried over anhydrous magnesium sulfate. The solvent was distilled away under reduced pressure, and the residue was purified by silica gel column chromatography (eluent chloroform-methanol 99:1 to 88:12 gradient), and then the resulting solid was triturated by a mixed solvent of ethyl acetate-n-hexane to give Compound 3 (47 mg) as a colorless powder.
MS (APCI) 424 [M+H]⁺

### Example 11-5

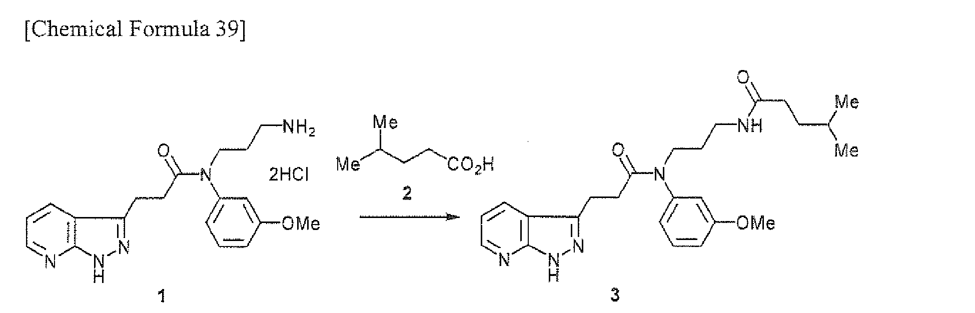

Compound 1 (43 mg). Compound 2 (12.3 mg) and HOBt (16.4 mg) were suspended in DMF (2 ml). Then, thereto were added triethylamine (31 µl), chloroform (0.5 ml) and WSC (29 mg), and the mixture was stirred at room temperature for 7 hours. To the reaction mixture were added ethyl acetate and water, and the mixture was separated, and then the organic layer was washed with saturated sodium bicarbonate water, and dried over anhydrous magnesium sulfate. The solvent was distilled away under reduced pressure, and the residue was purified by silica gel column chromatography (eluent chloroform-methanol 100:0 to 90:10 gradient), and then the resulting solid was triturated by a mixed solvent of ethyl acetate-n-hexane to give Compound 3 (33 mg) as a colorless powder.
MS (APCI) 452 [M+H]⁺

### Example 11-6

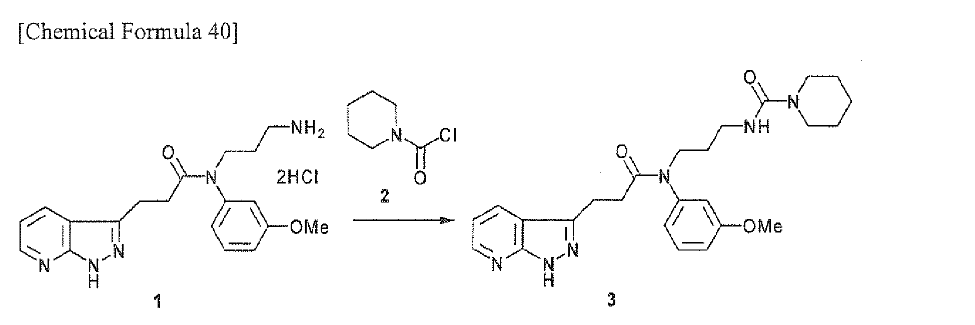

Compound 1 (60 mg) was suspended in chloroform (6 ml), and then thereto was added triethylamine (78 µl) with stirring under ice-cooling, and the mixture was stirred for 5 minutes. Then, thereto was added Compound 2 (19.4 µl), and the mixture was stirred at room temperature for 24 hours. To the reaction mixture were added chloroform and aqueous sodium hydrogencarbonate solution, and the mixture was separated. The organic layer was washed with saturated saline, and then dried over anhydrous sodium sulfate. The solvent was distilled away under reduced pressure, and the residue was purified by silica gel column chromatography (eluent chloroform-methanol 99:1 to 94:6 gradient) to give Compound 3 (44 mg) as a colorless amorphous powder.
MS (APCI) 465 [M+H]⁺

### Example 11-7

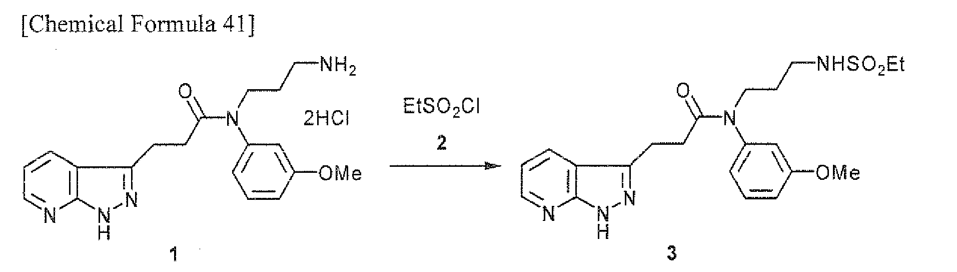

Compound 1 (50 mg) was suspended in chloroform (2 ml), and then thereto was added triethylamine (57 µl) with stirring under ice-cooling, and the mixture was stirred for 5 minutes. Then, thereto was added a solution of Compound 2 (15.8 mg) in chloroform (1 ml), and the mixture was stirred under ice-cooling for 3 hours. To the reaction mixture was added aqueous sodium hydrogencarbonate solution, and the mixture was separated. Then, the organic layer was dried over anhydrous magnesium sulfate. The solvent was distilled away under reduced pressure, and the residue was purified by silica gel column chromatography (eluent chloroform-methanol 99:1 to 88:12 gradient), and then the resulting solid was triturated by a mixed solvent of ethyl acetate-n-hexane to give Compound 3 (33.7 mg) as a colorless powder. MS (APCI) 446 [M+H]⁺

### Example 11-8

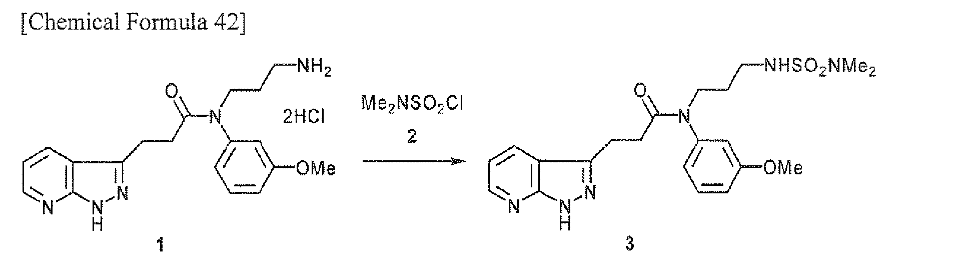

Compound 1 (50 mg) was suspended in chloroform (2 ml), and then thereto was added triethylamine (57 µl) at room temperature, and the mixture was stirred for 5 minutes. Then, thereto was added a solution of Compound 2 (17.9 mg) in chloroform (1 ml), and the mixture was stirred at 50°C for 24 hours and heated to reflux for additional 9 hours. The reaction mixture was let stand to cool, and then thereto was added aqueous sodium hydrogencarbonate solution, and the mixture was separated. Then, the organic layer was dried over anhydrous magnesium sulfate. The solvent was distilled away under reduced pressure, and the residue was purified by silica gel column chromatography (eluent chloroform-methanol 97:3 to 88:12 gradient) to give Compound 3 (43 mg) as a colorless amorphous powder.
MS (APCI) 461 [M+H]⁺

### Examples 11-9 to 11-29 and 12-1 to 12-22

The corresponding starting compounds were reacted and treated in the similar manner to the above Example to give compounds of Examples 11-9 to 11-29 and 12-1 to 12-22.

### Example 13-1

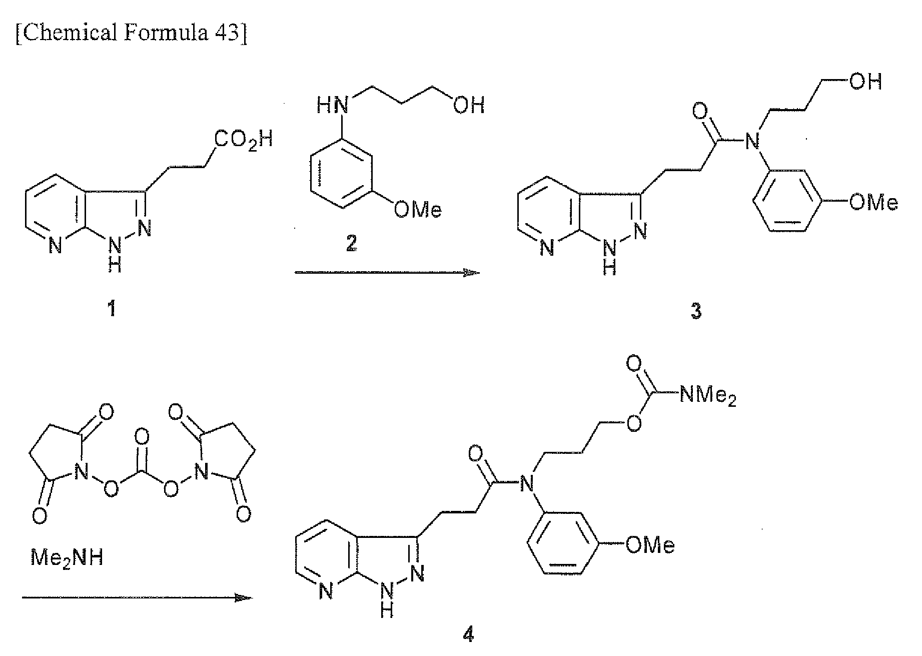

(1) To a solution of Compound 1 (300 mg) in THF (20 ml) were added triphenylphosphine (576 mg), N-chlorosuccinimide (273 mg) and THF (2 ml) at room temperature, and the mixture was stirred for 2 minutes. Then, thereto was added a solution of N,N-dimethylaniline (438 µl) and Compound 2 (313 mg) in THF (2 ml), and the mixture was stirred at 60°C for 18 hours. The reaction mixture was let stand to cool, and then thereto was added ethyl acetate, and then the mixture was washed with water. The organic layer was washed with 2N-aqueous sodium hydroxide solution and saturated saline, and dried over anhydrous sodium sulfate. The solvent was distilled away under reduced pressure, and the resulting residue was purified by silica gel column chromatography (eluent chloroform-methanol 99:1 to 93:7 gradient) to give Compound 3 (392 mg) as a colorless amorphous powder.
   MS (APCI) 355 [M+H]⁺
(2) To Compound 3 (70 mg) were added acetonitrile (2.5 ml) and THF (2.5 ml), and then thereto were added N,N'-disuccinimidyl carbonate (152 mg) and N,N'-dimethylaminopyridine (2.4 mg), and the mixture was stirred at room temperature for 23 hours. Then, thereto was added 2N-dimethylamine-THF solution (595 µl) and the mixture was stirred at room temperature for 2 days. The solvent of the reaction mixture was distilled away under reduced pressure, and then thereto was added 5% aqueous potassium carbonate solution, and the mixture was extracted with ethyl acetate. The extracted layer was washed with saturated saline, and drided over anhydrous sodium sulfate, and then concentrated under reduced pressure. The resulting residue was dissolved in methanol (4 ml) and THF (1 ml), and then thereto was added 2N-aqueous sodium hydroxide solution (0.5 ml), and the mixture was stirred at room temperature for 18 hours. The solvent of the reaction mixture was distilled away under reduced pressure, and then thereto was added water, and the mixture was extracted with ethyl acetate. The extracted layer was washed with saturated saline, dried over anhydrous sodium sulfate, and then the solvent was distilled away under reduced pressure. The residue was purified by silica gel column chromatography (eluent chloroform-methanol 99:1 to 95:5 gradient) to give Compound 4 (70 mg) as a colorless powder.
   MS (APCI) 426[M+H]⁺

### Example 14-1

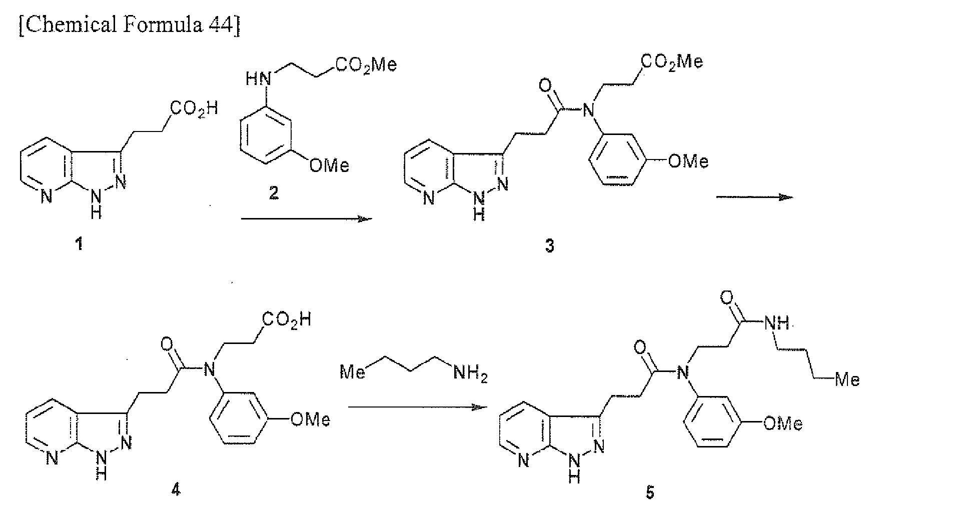

(1) To a solution of Compound 1 (250 mg) in THF (15 ml) were added triphenylphosphine (514 mg), N-chlorosuccinimide (245 mg) and THF (1 ml) at room temperature, and the mixture was stirred for 2 minutes. Then, thereto was added a solution of N,N-dimetbylaniline (365 µl) and Compound 2 (302 mg) in THF (2 ml), and the mixture was stirred at 60°C for 18 hours. The reaction mixture was let stand to cool, and then thereto was added ethyl acetate, and the mixture was washed with water. The organic layer was washed with saturated sodium bicarbonate water and saturated saline, and dried over anhydrous sodium sulfate. The solvent was distilled away under reduced pressure, and the resulting residue was purified by silica gel column chromatography (eluent chloroform-methanol 100:0 to 97:3 gradient) to give Compound 3 (323 mg) as a colorless powder.
   MS (APCI) 383 [M+H]⁺
(2) To a solution of Compound 3 (285 mg) in methanol (4 ml) and THF (4 ml) was added 1N aqueous sodium hydroxide solution (1.49 ml). The reaction mixture was stirred at room temperature for 18 hours, and then the solvent was distilled away under reduced pressure.
   Then, thereto was added 10% aqueous solution of citric acid, and the mixture was extracted with ethyl acetate three times. The extracted layer was combined, and dried over anhydrous magnesium sulfate. The solvent was distilled away under reduced pressure to give Compound 4 (223 mg) as a colorless amorphous powder.
   MS (APCI) 369 [M+H]⁺
(3) Compound 4 (50 mg) and HOBt (24 mg) were dissolved in DMF (1 ml), and then thereto were added WSC (34 mg) and n-butylamine (25 mg) with stirring under ice-cooling, and the mixture was stirred at room temperature for 16 hours. Then, thereto was added aqueous sodium hydrogencarbonate solution, and the mixture was extracted with ethyl acetate.
   The extracted layer was washded with saturated saline, and dried over anhydrous sodium sulfate. The solvent was distilled away under reduced pressure, and the resulting residue was purified by silica gel column chromatography (eluent chloroform-methanol 99:1 to 90:10 gradient) to give Compound 5 (49 mg) as a colorless powder.
   MS (APCI) 424 [M-H]⁺

### Example 15-1

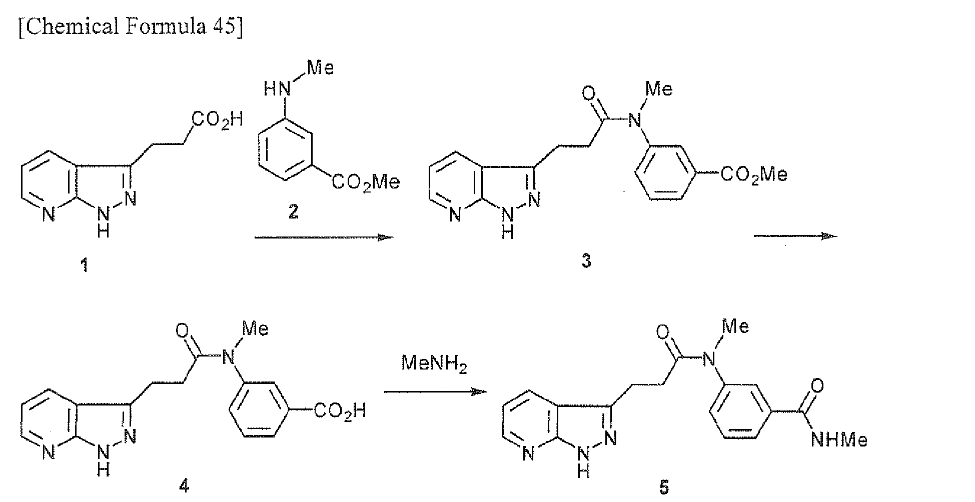

(1) To a solution of Compound 1 (200 mg) in THF (28 ml) were added triphenylphosphine (494 mg), N-chlorosuccinimide (238 mg) and THF (1 ml) at room temperature, and the mixture was stirred for 2 minutes. Then, thereto was added a solution of N,N-dimethylaniline (332 µl) and Compound 2 (224 mg) in THF (3 ml), and the mixture was heated to reflux for 7 hours. The reaction mixture was let stand to cool, and then thereto was added ethyl acetate, and then the mixture was washed with water. The organic layer was washed with 2N-aqueous sodium hydroxide solution and saturated saline, and dried over anhydrous sodium sulfate. The solvent was distilled away under reduced pressure, and the resulting residue was purified by silica gel column chromatography (eluent chloroform-methanol 98:2 to 93:7 gradient) to give Compound 3 (283 mg) as a colorless powder.
   MS (APCI) 339 [M+H]⁺
(2) To a solution of Compound 3 (280 mg) in methanol (4.3 ml) and THF (4.3 ml) was added 2N-aqueous sodium hydroxide solution (4.3 ml). The mixture was stirred at room temperature for 4 hours, and then thereto was added 2N-hydrochloric acid (4.3 ml), and the mixture was extracted with ethyl acetate. The extracted layer was dried over anhydrous sodium sulfate. The solvent was distilled away under reduced pressure, and the resulting solid was triturated by a mixed solvent of methanol-isopropyl ether to give Compound 4 (225 mg) as a colorless powder.
   MS (ESI) 323[M-H]⁻
(3) To a solution of Compound 4 (40 mg) in DMF (1.2 ml) and THF (4.3 ml) were added O-(7-azabenzotriazol-l-yl)-N,N,N',N'-tetramethyluronium hexafluorophosphate (70 mg), WSC (35 mg), 2N-methylamine-THF solution (617 µl) and N,N-dimethylaminopyridine (1 mg), and the mixture was stirred at room temperature for 17 hours. Then, thereto was added aqueous sodium hydrogencarbonate solution, and the mixture was extracted with chloroform. The extracted layer was dried over anhydrous sodium sulfate. The solvent was distilled away under reduced pressure, and the resulting residue was purified by silica gel column chromatography (eluent chloroform-methanol 99:1 to 90:10 gradient) to give Compound 5 (16 mg) as a colorless oily substance.
   MS (APCI) 338 [M+H]⁺

### Example 16-1

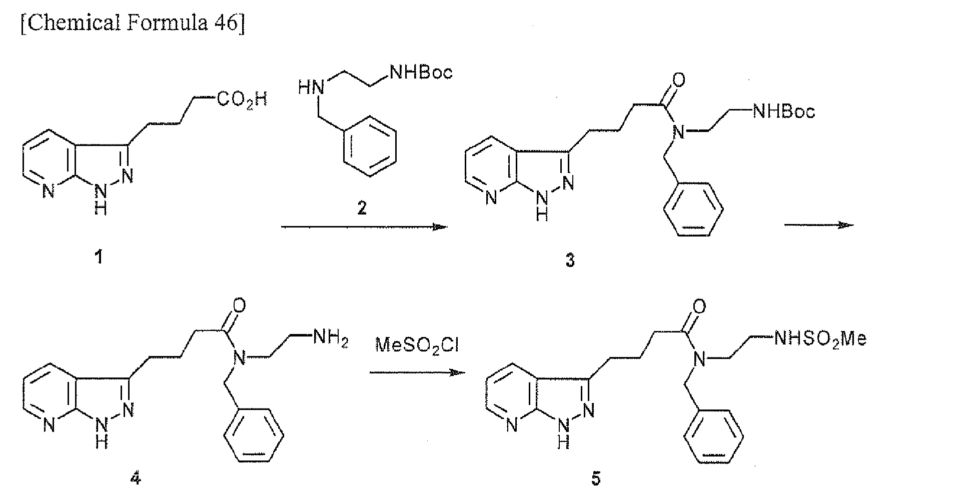

(1) To a solution of Compound 1 (57.3 mg) and Compound 2 (95.9 mg) in DMF (2 ml) were added HOBt (37.7 mg) and WSC (69.6 mg) at room temperature, and the mixture was stirred for 3 days. To the reaction mixture was added water, and the mixture was extracted with ethyl acetate. The extracted layer was washed with saturated saline, and dried over anhydrous sodium sulfate. The solvent was distilled away under reduced pressure, and the residue was purified by silica gel column chromatography (eluent chloroform-methanol 100:0 to 85:15 gradient) to give Compound 3 (110.6 mg) as a colorless oily substance.
   MS (APCI) 452 [M+H]⁺
(2) To a solution of Compound 3 (125 mg) in chloroform (1 ml) was added 4N-hydrochloric acid-ethyl acetate solution (1 ml) at room temperature, and the mixture was stirred for 16 hours. The precipitate in the reaction mixture was filtered, and washed with ethyl acetate. To the resulting solid were added ethyl acetate and 15% ammonia water, and the mixture was separated. The organic layer was washed with saturated saline, and dried over anhydrous sodium sulfate. The solvent was distilled away under reduced pressure to give Compound 4 (94 mg) as a colorless oily substance.
   MS (APCI) 338 [M+H]⁺
(3) To a solution of Compound 4 (39.5 mg) in chloroform (1.5 ml) were added triethylamine (34 mg) and methane sulfonylchloride (9 µl) with stirring under ice-cooling, and the mixture was stirred at room temperature for 16 hours. The reaction mixture was purified by silica gel column chromatography (eluent chloroibrm-methanol 100:0 to 85:15 gradient) to give Compound 5 (39.1 mg) as a colorless oily substance.
   MS (APCI) 416 [M+H]⁺

### Reference Example 1

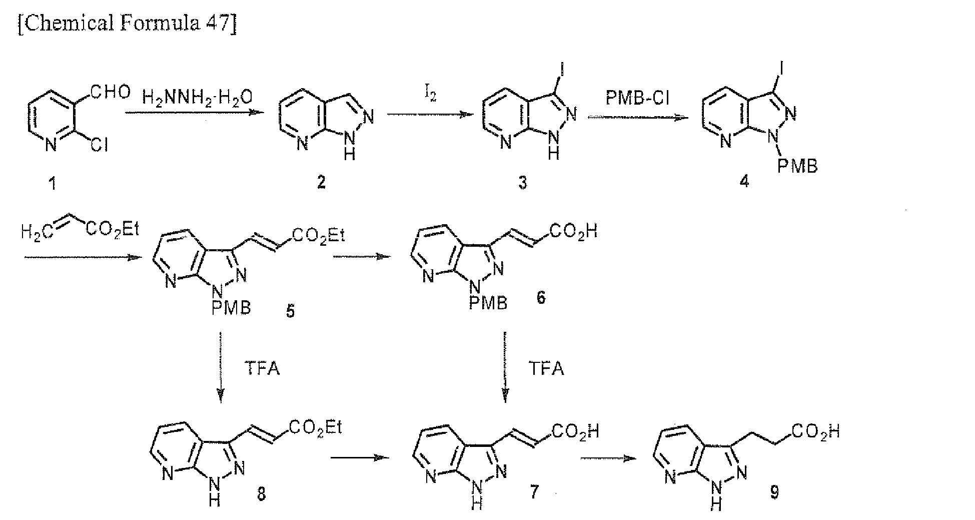

(1) According to the method of Chemical Communications 293-294 (1966), to a mixture of Compound 1 (20.0 g) and p-toluenesulfonic acid monohydrate (15.6 g) was added slowly hydrazine monohydrate (26.6 ml) with stirring under ice-cooling. The mixture was heated to stir at 130°C for 21 hours. The reaction mixture was let stand to cool, and then poured into 25% aqueous potassium carbonate solution, and extracted with ethyl acetate. The extracted layer was combined, and dried over anhydrous magnesium sulfate. The solvent was distilled away under reduced pressure, and the resulting residue was purified by silica gel column chromatography (eluent: hexane-ethyl acetate 2:1 to 1:1). The resulting solid was triturated by hexane-ethyl acetate (1:1) to give Compound 2 (12.27 g) as colorless powdery crystals.
   MS (APCI) 120 [M+H]⁺
(2) Compound 2 (12.2 g) was dissolved in DMF (200 ml), and then thereto was added potassium hydroxide (26.86 g) with stirring under ice-cooling, then added gradually iodine (52.24 g), and then the mixture was warmed slowly to room temperature, and stirred at room temperature for an hour. The reaction mixture was poured into 10% aqueous sodium hydrogen sulfite solution (1 L), and the precipitated crystals were filtered, washed with water, and then dried to give Compound 3 (21.07 g) as pale yellow crystals.
   MS (APCI) 246[M+H]⁺
(3) 60% Sodium hydride (10.8 g) was washed with anhydrous hexane, and suspended in DMF (350 ml). Then, thereto was added dropwise a solution of Compound 3 (55.11 g) in DMF (200 ml) with stirring under ice-cooling over about 2 hours, and the mixture was stirred under ice-cooling for an hour. To the mixture was added p-methoxybenzyl chloride (36.6 ml), and then the mixture was warmed to room temperature, and stirred for an hour. The reaction mixture was poured into ice water (3.5 L), and the precipitated crystals were filtered, washed with water, and then dried. The resulting solid was triturated by chloroform-isopropyl ether (2:1) to give Compound 4 (35.0 g) as pale red crystals.
   Additionally, the filtrate was concentrated under reduced pressure, and the residue was purified by silica gel column chromatography (eluent: chloroform-ethyl acetate 100:1) to give Compound 4 (29.0 g) as pale red crystals.
   MS (APCI) 366[M+H]⁺
(4) To a solution of Compound 4 (24.7 g) in dioxane (430 ml) were added triphenylphosphine (3.55 g), palladium acetate (1.52 g) and triethylamine (123 ml). After argon substitution, thereto was added ethyl acrylate (74 ml), and the mixture was heated to stir at 100°C for 4 hours. The reaction mixture was let stand to cool to room temperature, and concentrated under reduced pressure. The resulting residue was dissolved in ethyl acetate, washed with water and saturated saline, and dried over anhydrous sodium sulfate. The mixture was concentrated under reduced pressure, and the resulting residue was purified by silica gel column chromatography (eluent: hexane-ethyl acetate 4:1 to 3:1), and the resulting solid was triturated by isopropyl ether and dried to give Compound 5 (20.838 g) as colorless crystals.
   MS (APCI) 338[M+H]⁺
(5) To a solution of Compound 5 (19.2 g) in THF-ethanol (80 ml-160 ml) was added 2N aqueous sodium hydroxide solution (140 ml) under ice-cooling, and the mixture was stirred at room temperature for 2 hours. To the reaction mixture was added 2N hydrochloric acid (140 ml) under ice-cooling, then added water. The precipitated crystals were filtered, washed with water, and then dried to give Compound 6 (16.9 g) as colorless crystals. Additionally, the filtrate was extracted with a mixed solution of ethyl acetate-THF, and the extracted layer was washed with saturated saline, and dried over anhydrous sodium sulfate. The mixture was concentrated under reduced pressure, and the residue was triturated by diethyl ether to give Compound 6 (0.52 g) as colorless crystals. They were combined with the previously obtained crystals to give Compound 6 (17.42 g).
   MS (ESI) 308[M-H]⁻
(6) Compound 6 (522.9 mg) was suspended in trifluoroacetic acid (5 ml), and heated to reflux. After 16 hours, the reaction mixture was concentrated under reduced pressure, and triturated by methanol-ethyl acetate to give Compound 7 (0.303 g) as pale yellow crystals.
   MS (APCI) 190[M+H]⁺
(7) Compound 5 (45.7 g) was suspended in trifluoroacetic acid (300 ml), and heated to reflux for 3.5 hours. The reaction mixture was cooled, and concentrated under reduced pressure. Then, to the residue were added ethyl acetate (100 ml) and 10% aqueous potassium carbonate solution, and the mixture was stirred. The precipitated crystals were filtered, washed with water, isopropyl ether and ethyl acetate, and dried to give Compound 8 (28.12 g) as colorless crystals.
   MS (APCI) 218[M+H]⁺
(8) To a solution of Compound 8 (33.22 g) in THF-ethanol (200 ml-200 ml) was added 2N aqueous sodium hydroxide solution (338 ml) under ice-cooling, and the mixture was stirred at room temperature for 16 hours. The reaction mixture was concentrated under reduced pressure, and adjusted to pH 3 by the addition of 2N hydrochloric acid (340 ml) with stirring under ice-cooling. The precipitated crystals were filtered, washed with water, and then dried and triturated by chloroform to give Compound 7 (26.63 g) as colorless crystals.
   MS (APCI) 190[M+H]⁺
(9) Compound 7 (10.00 g) was dissolved in THF (500 ml) and methanol (500 ml), and then thereto was added aqueous 10% palladium carbon (5.00 g), and the mixture was stirred vigorously under hydrogen atmosphere at room temperature for 3 hours. The palladium carbon was filtered off through Celite, and washed with a mixed solvent of THF-methanol (1:1). The solvent of the filtrate was distilled away under reduced pressure, and the resulting residue was triturated by diisopropyl ether to give Compound 9 (9.15 g) as a colorless powder.
   MS (APCI) 192 [M+H]⁺

### Reference Example 2

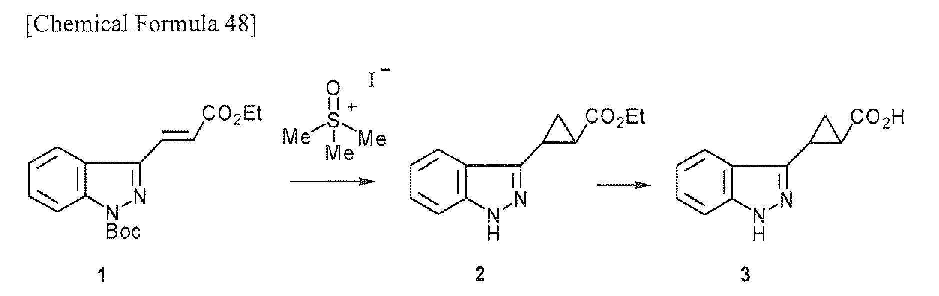

(1) To a suspension of 60% sodium hydride (0.38 g) in DMSO (30 ml) was added gradually trimethylsulfoxonium iodide (2.13 g) at room temperature, and the mixture was stirred for 90 minutes. Then, thereto was added dropwise a solution of Compound 1 (1.00 g) in DMSO (25 ml), and the mixture was stirred at room temperature for 3 hours. The reaction mixture was poured into ice water, and extracted with ethyl acetate. The extracted layer was washed with saturated saline, and then dried over anhydrous magnesium sulfate. The solvent was distilled away under reduced pressure, and the resulting residue was purified by NH silica gel column chromatography (eluent chloroform-ethyl acetate 99:1 to 90:10 gradient) to give Compound 2 (203 mg) as a colorless oily substance.
   MS (APCI) 231 [M+H]⁺
(2) To a solution of Compound 2 (200 mg) in ethanol (2.2 ml) was added 2N-aqueous sodium hydroxide solution (2.2 ml) at room temperature, and the mixture was stirred for 16 hours. The reaction mixture was concentrated under reduced pressure, and adjusted to pH4 by the addition of 2N-hydrochloric acid (2.2 ml) with stirring under ice-cooling. The precipitated crystals were filtered, washed with water, and then dried to give Compound 3 (159 mg) as a colorless powder.
   MS (ESI) 201 [M-H]⁻

### Reference Example 3

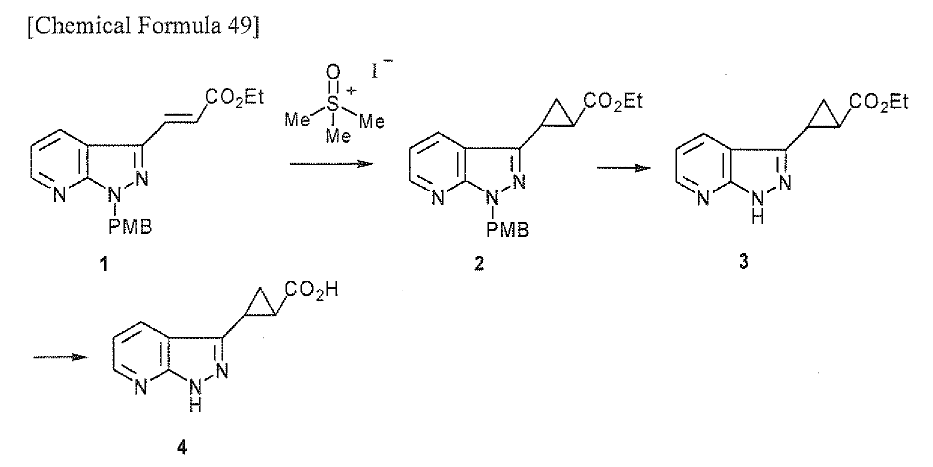

(1) To a suspension of 60% sodium hydride (0.36 g) in DMSO (30 ml) was added gradually trimethylsulfoxonium iodide (2.00 g) at room temperature, and the mixture was stirred for 2 hours. Then, thereto was added dropwise a solution of Compound 1 (1.00 g) in DMSO (25 ml), and the mixture was stirred at room temperature for 4 hours. The reaction mixture was poured into ice water, and extracted with ethyl acetate. The extracted layer was washed with saturated saline, and then dried over anhydrous magnesium sulfate. The solvent was distilled away under reduced pressure, and the resulting residue was purified by silica gel column chromatography (eluent n-hexane-ethyl acetate 70:30 to 40:60 gradient) to give Compound 2 (793 mg) as a colorless oily substance.
   MS (APCI) 352 [M+H]⁺
(2) Compound 2 (784 mg) was suspended in trifluoroacetic acid (9 ml), and heated to reflux for 14 hours. The reaction mixture was cooled, and then concentrated under reduced pressure. To the residue was added saturated sodium bicarbonate water, and the mixture was extracted with ethyl acetate. The extracted layer was washed with saturated saline, and then dried over anhydrous magnesium sulfate. The solvent was distilled away under reduced pressure. To the resulting residue was added methanol, and the mixture was stirred. Then, the insoluble was filtered off, and the filtrate was concentrated under reduced pressure. The residue was triturated by isopropyl ether to give Compound 3 (437 mg) as a colorless powder.
   MS (APCI) 232[M+H]⁺
(3) To a solution of Compound 3 (425 mg) in ethanol (5 ml) and THF (5 ml) was added 2N-aqueous sodium hydroxide solution (4.6 ml) at room temperature, and the mixture was stirred for 15 hours. The reaction mixture was concentrated under reduced pressure, and adjusted to pH3 by the addition of water (4.6 ml) and 2N-hydrochloric acid (4.6 ml) with stirring at room temperature. The precipitated crystals were filtered, washed with water, and then dried to give Compound 4 (365 mg) as a colorless powder.
   MS (APCI) 204[M+H]⁺

### Reference Example 4

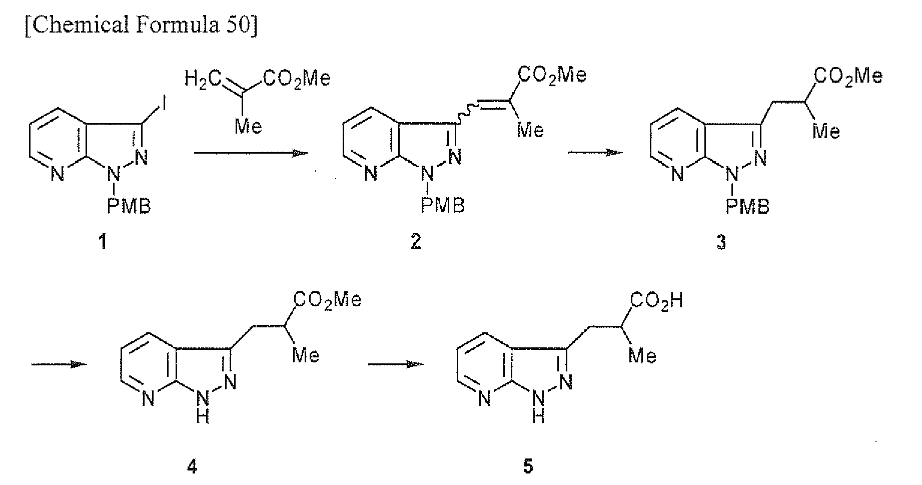

(1) To a solution of Compound 1 (40.00 g) in dioxane (350 ml) were added triphenylphosphine (5.75 g), palladium acetate (2.46 g) and triethylamine (46 ml) under argon atmosphere. Then, thereto was added methyl methacrylate (110 g), and the mixture was heated to reflux for 3 days. The reaction mixture was let stand to cool to room temperature, and concentrated under reduced pressure. To the resulting residue were added ethyl acetate and water, and the insoluble was filtered off through Celite and washed with ethyl acetate. The filtrate was separated, and the organic layer was washed with saturated saline and dried over anhydrous magnesium sulfate. The solvent was distilled away under reduced pressure, and the resulting residue was purified by silica gel column chromatography (eluent n-hexane-ethyl acetate 90:10 to 65:35) to give Compound 2 (20.96 g) as a pale red powder.
   MS (APCI) 338[M+H]⁺
(2) Compound 2 (20.96 g) was dissolved in THF (120 ml) and methanol (60 ml), and then thereto was added anhydrous 10% palladium carbon (5.00 g), and the mixture was stirred vigorously under hydrogen atmosphere at room temperature for 18 hours. The palladium carbon was filtered off through Celite and washed with a mixed solvent of THF-methanol (2:1).
   The solvent of the filtrate was distilled away under reduced pressure, and the resulting residue was purified by silica gel column chromatography (eluent n-hexane-ethyl acetate 70:30) to give Compound 3 (14.69 g) as a colorless oily substance.
   MS (APCI) 340 [M+H]⁺
(3) To Compound 3 (4.50 g) was added trifluoroacetic acid (30 ml), and the mixture was heated to reflux for 3 hours. The reaction mixture was cooled, and then concentrated under reduced pressure. The residue was purified by silica gel column chromatography (eluent n-hexane-ethyl acetate 95:5 to 50:50 gradient) to give Compound 4 (3.26 g) as a colorless powder.
   MS (APCI) 220 [M+H]⁺
(4) To a solution of Compound 4 (3.26 g) in ethanol (20 ml) and THF (20 ml) was added 4N-aqueous sodium hydroxide solution (15 ml), and the mixture was heated to reflux for 2 hours. The reaction mixture was cooled, and then adjusted to pH3 by the addition of 10% aqueous solution of citric acid. The organic solvent was concentrated under reduced pressure, and then the precipitate was filtered and washed with water. The resulting solid was purified by silica gel column chromatography (eluent cbloroform-methanol 100:0 to 90:10 gradient) to give Compound 5 (2.05 g) as a colorless powder.
   MS (APCI) 206 [M+H]⁺

### Reference Example 5

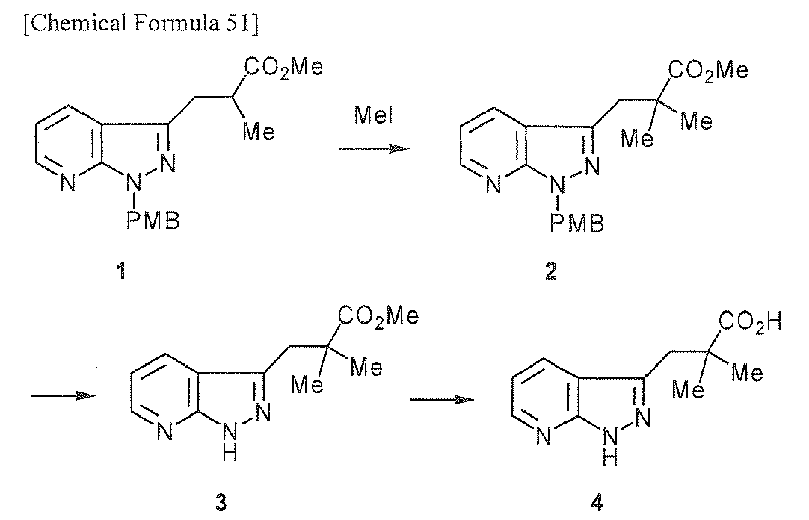

(1) To a solution of Compound 1 (9.00 g) and methyl iodide (3.49 ml) in THF (100 ml) was added t-butoxypotassium (3.27 g) at -78°C. The mixture was warmed to room temperature with stirring for 3 hours, and stirred at room temperature for 15 hours. The reaction mixture was poured into saturated ammonium chloride water, and then the organic layer was concentrated under reduced pressure and extracted with ethyl acetate. The extracted layer was washed with saturated saline, and dried over anhydrous magnesium sulfate. The solvent was distilled away under reduced pressure, and the resulting residue was purified by silica gel column chromatography (eluent n-hexane-ethyl acetate 3:1) to give Compound 2 (6.44 g) as a colorless oily substance.
   MS (APCI) 354[M+H]⁺
(2) To Compound 2 (6.43 g) was added trifluoroacetic acid (30 ml), and the mixture was heated to reflux for 3 hours. The reaction mixture was cooled, and then concentrated under reduced pressure. The residue was purified by silica gel column chromatography (eluent n-hexane-ethyl acetate 90:10 to 50:50 gradient) to give Compound 3 (3.61 g) as a pale yellow powder.
   MS (APCI) 234 [M+H]⁺
(3) To a solution of Compound 3 (3.60 g) in ethanol (40 ml) was added 2N-aqueous sodium hydroxide solution (16 ml), and the mixture was heated to reflux for 2 hours. The reaction mixture was cooled, and then adjusted to pH3 by the addition of 10% aqueous solution of citric acid. The precipitate was filtered, washed with water, and dried to give Compound 4 (2.69 g) as a colorless powder.
   MS (APCI) 220 [M+H]⁺

### Reference Example 6

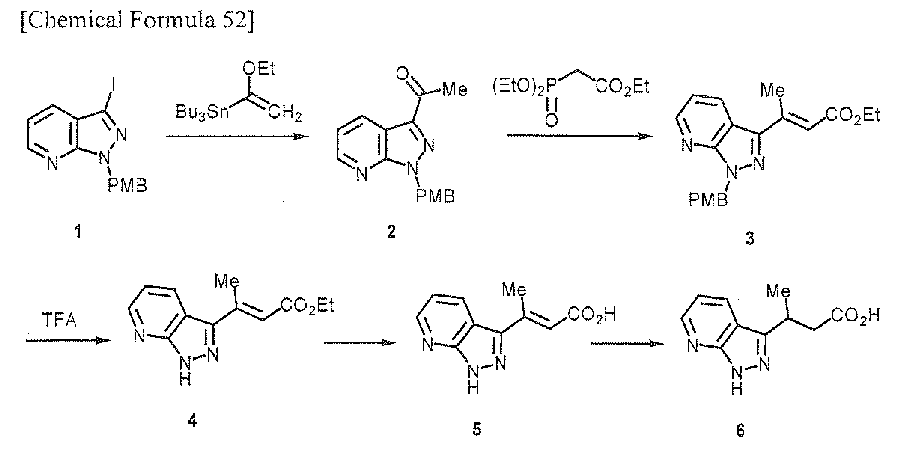

(1) Compound 1 (500 mg) and 1-ethoxy vinyl tributoxy tin (663 mg) were suspended in dioxane (5 ml). To the mixture was added dichlorobistriphenylphosphine palladium (II) (49 mg) under argon atmosphere, and the mixture was heated to reflux for 21 hours. The reaction mixture was let stand to cool, and then thereto was added ethyl acetate, then 10% aqueous potassium fluoride solution, and then mixture was stirred at room temperature for an hour. The insoluble was filtered off through Celite, and to the filtrate was added water, and the mixture was separated. To the organic layer was added 1N hydrochloric acid, and the mixture was stirred vigorously at room temperature. The mixture was neutralized by the addition of saturated aqueous sodium hydrogencarbonate solution, and then the organic layer was separated, washed with saturated saline, and dried over anhydrous magnesium sulfate. The solvent was distilled away under reduced pressure, and the resulting residue was purified by silica gel column chromatography (eluent: hexane-ethyl acetate 9:1 to 2:1) to give Compound 2 (321.6 mg) as a pale yellow solid.
   MS (APCI) 282[M+H]⁺
(2) To a suspension of 60% sodium hydride (2.13 g) in dioxane (40 ml) was added dropwise a solution of diethylphosphonoacetic acid ethyl ester (11.95 g) in dioxane (40 ml) over 15 minutes with stirring at room temperature. The mixture was stirred at room temperature for an hour, and then thereto was added Compound 2 (5.00 g), and the mixture was stirred at 55 to 58°C for 3 hours. The reaction mixture was cooled, and then thereto was added water (100 ml), and the mixture was extracted with ethyl acetate. The extracted layer was combined, washed with saturated saline, dried over anhydrous magnesium sulfate, and concentrated under reduced pressure. The residue was purified by silica gel column chromatography (eluent: chloroform). The resulting fractions (9.41 g) were dissolved in dioxane (30 ml), and then thereto were added 60% sodium hydride (1.07 g) at room temperature and added dropwise a solution of ethanol (1.56 ml) in dioxane (10 ml). The mixture was heated to stir at 45 to 55°C, cooled, and then thereto was added water (40 ml), and the mixture was extracted with ethyl acetate. The extracted layer was combined, washed with saturated saline, dried over anhydrous sodium sulfate, and then concentrated under reduced pressure. The resulting residue was purified by silica gel column chromatography (eluent hexane-ethyl acetate gradient) to give Compound 3 (4.86 g) as a colorless solid.
   MS (APCI) 352[M+H]⁺
(3) Compound 3 (4.86 g) was suspended in trifluoroacetic acid (48.6 ml), and heated to reflux. After 4 hours, the mixture was cooled, and then concentrated under reduced pressure, and the residue was dissolved in ethyl acetate. The mixture was washed with aqueous sodium hydrogencarbonate solution and saturated saline, dried over anhydrous sodium sulfate, and then concentrated under reduced pressure. The residue was purified by silica gel column chromatography (eluent chloroform-ethyl acetate gradient) to give Compound 4 (3.36 g) as a colorless powder.
   MS (APCI) 232[M+H]⁺
(4) Compound 4 (3.36 g) was dissolved in THF-ethanol (34 ml-34 ml), and then thereto was added 2N aqueous sodium hydroxide solution (34.6 ml), and the mixture was heated to stir at 50°C. After 8 hours, the reaction solution was cooled to room temperature. The solvent was distilled away under reduced pressure, and then thereto was added 2N hydrochloric acid (34.5 ml), and the mixture was cooled slowly. The precipitated colorless crystals were filtered and dried to give Compound 5 (2.05 g) as a colorless powder.
   MS (APCI) 204[M+H]⁺
(5) Compound 5 (300 mg) was dissolved in THF (15 ml) and methanol (15 ml), and then thereto was added aqueous 10% palladium carbon (156 mg), and the mixture was stirred vigorously under hydrogen atmosphere at room temperature for 22 hours. The palladium carbon was filtered off through Celite and washed with THF. The solvent of the filtrate was distilled away under reduced pressure, and the resulting residue was purified by silica gel column chromatography (eluent chloroform-methanol 86:14) to give Compound 6 (303 mg) as a colorless solid.
   MS (APCI) 206 [M+H]⁺

### Reference Example 7

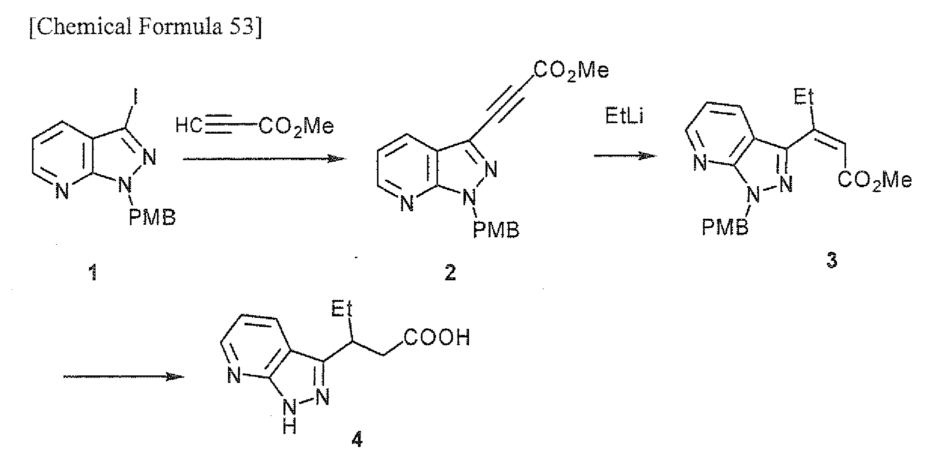

(1) Compound 1 (2.00 g), copper (I) iodide (105 mg), sodium hydrogencarbonate (953 mg) and methyl propiolate (1.84 g) were suspended in DMF (50 ml). To the mixture was added dichlorobistriphenylphosphine palladium (II) (384 mg) under argon atmosphere, and the mixture was heated to stir at 60°C for 6 hours. The reaction mixture was let stand to cool, and then thereto was added ethyl acetate, and then the mixture was washed with saturated saline and dried over anhydrous magnesium sulfate. The solvent was distilled away under reduced pressure, and the resulting residue was purified by silica gel column chromatography (eluent: hexane-ethyl acetate 20:1 1 to 3:1) to give Compound 2 (1.09 g) as a pale yellow powder.
   MS (APCI) 322[M+H]⁺
(2) Copper (I) iodide (1.94 g) was suspended in diethyl ether (20 ml). To the suspension was added dropwise a solution of ethyllithium in 0.5M benzene-cyclohexane (41 ml) over 30 minutes with stirring at 0°C. Then, thereto was added dropwise a solution of Compound 2 (1.09 g) in THF (10 ml) over 20 minutes with stirring at -78°C. The mixture was stirred at - 78°C for 30 minutes, and then thereto was added water (2 ml), and the mixture was warmed to room temperature. The insoluble was filtered off through Celite, and washed with ethyl acetate. To the filtrate was added an aqueous solution of citric acid, and the mixture was separated. The organic layer was washed with saturated saline, dried over anhydrous magnesium sulfate, and concentrated under reduced pressure. The residue was purified by silica gel column chromatography (eluent: hexane-ethyl acetate 20:1 to 7:3) to give Compound 3 (1.15 g) as a red oily substance.
   MS (APCI) 352[M+H]⁺
(3) Compound 3 (865 mg) was dissolved in THF (10 ml) and methanol (10 ml), and then thereto was added anhydrous 10% palladium carbon (1.00 g), and the mixture was stirred vigorously under hydrogen atmosphere at room temperature for 20 hours. The palladium carbon was filtered off through Celite, and washed with a mixed solvent of THF-methanol. The solvent of the filtrate was distilled away under reduced pressure. To the resulting residue were added chloroform (5 ml) and trifluoroacetic acid (5 ml), and the mixture was heated to reflux for 2 hours. The reaction mixture was cooled, and then concentrated under reduced pressure. The residue was purified by silica gel column chromatography (eluent chloroform-methanol 100:0 to 95:5 gradient) to give Compound 4 (173 mg) as a brown powder.
   MS (APCI) 220 [M+H]⁺

### Reference Example 8

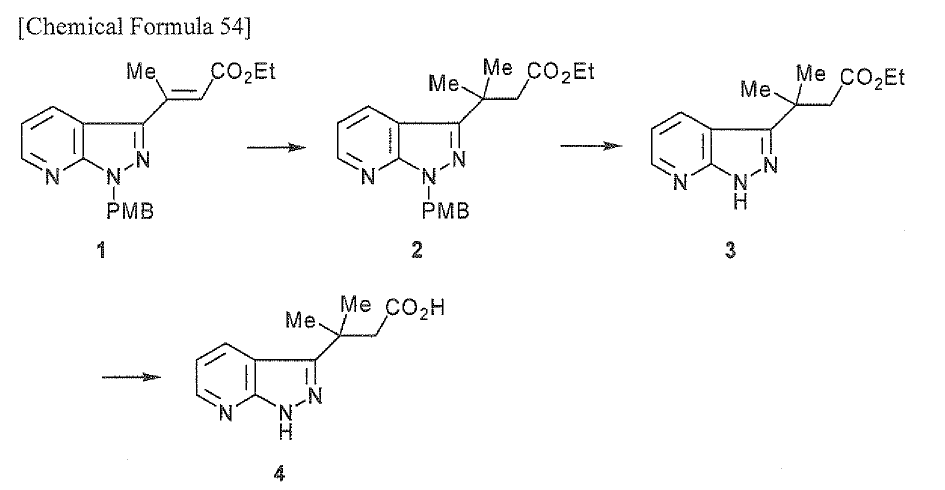

(1) Copper (I) iodide (15.8 g) was suspended in diethyl ether (80 ml). To the suspension was added dropwise 1.04M-methyllithium-diethyl ether solution (160 ml) over 60 minutes with stirring at 0°C under argon atmosphere. The mixture was stirred at 0°C for 5 minutes, and then the solvent was concentrated under reduced pressure. To the residue was added methylene chloride (100 ml), and the mixture was stirred for 5 minutes under ice-cooling, and then the solvent was concentrated under reduced pressure. To the residue was added methylene chloride (530 ml), and then thereto was added trimethylsilyl chloride (10.6 ml) under argon atmosphere with stirring at -78°C, and added dropwise a solution of Compound 1 (9.75 g) in methylene chloride (100 ml) over 10 minutes. The reaction mixture was warmed to 0°C over 3 hours with stirring, and then poured into a 1:1:1 mixed solution of 28% ammonia water-aqueous saturated ammonium chloride solution-water. Then, thereto was added chloroform, and the mixture was separated, and then the organic layer was washed with saturated saline, dried over anhydrous magnesium sulfate and concentrated under reduced pressure. The residue was purified by silica gel column chromatography (eluent chloroform-ethyl acetate 97:3) to give Compound 2 (7.55 g) as a yellow oily substance.
   MS (APCI) 368[M+H]⁺
(2) To Compound 2 (7.16 g) was added trifluoroacetic acid (72 ml), and the mixture was heated to reflux for 2 hours. The reaction mixture was cooled, and then concentrated under reduced pressure. To the residue were added chloroform and saturated sodium bicarbonate water, and the mixture was separated. The organic layer was washed with saturated saline, dried over anhydrous sodium sulfate, and concentrated under reduced pressure. The residue was purified by silica gel column chromatography (eluent chloroform-ethyl acetate 80:20 to 30:70 gradient) to give Compound 3 (4.45 g) as a pale yellow oily substance.
   MS (APCI) 248 [M+H]⁺
(3) To a solution of Compound 3 (4.45 g) in ethanol (90 ml) was added 2N-aclueous sodium hydroxide solution (90 ml), and the mixture was stirred at room temperature for 14 hours. Then, thereto was added chloroform under ice-cooling, and the mixture was adjusted to pH3 to 4 by the addition of 2N-hydrochloric acid with stirring. The mixture was separated, and then the organic layer was dried over anhydrous sodium sulfate and concentrated under reduced pressure. The residue was triturated by isopropyl ether to give Compound 4 (3.78 g) as a colorless powder.
   MS (APCI) 220[M+H]⁺

### Reference Example 9

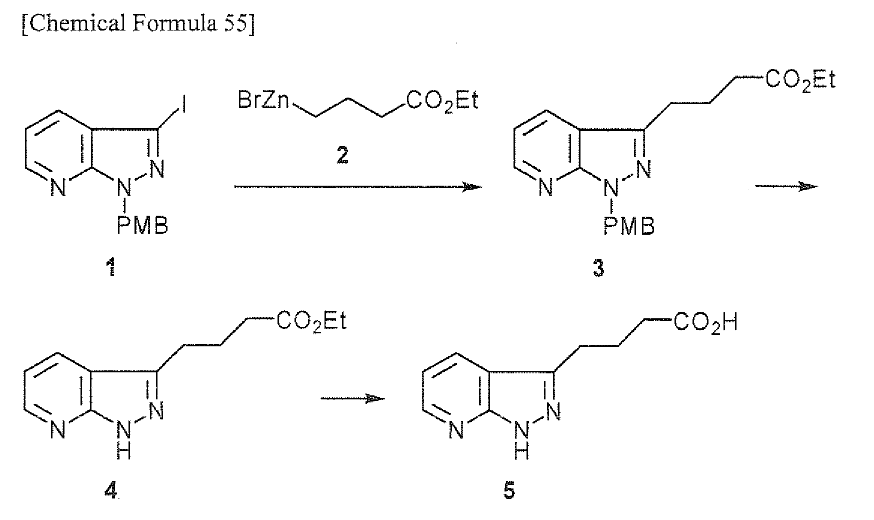

(1) To Compound 1 (2.51 g) and [1,1'-bis(diphenylphosphino)ferrocene]palladium (II) dichloride-methylene chloride (0.41 g) was added a solution of Compound 2 in 0.5M-THF (22 ml) under argon atmosphere. The reaction mixture was heated to reflux for 16 hours. The reaction mixture was cooled, and then thereto were added ethyl acetate and sodium bicarbonate water, and the mixture was stirred. The insoluble was filtered off through Celite and washed with ethyl acetate. The filtrate was separated, and then the organic layer was washed with saturated saline, dried over anhydrous sodium sulfate, and concentrated under reduced pressure. The residue was purified by NH silica gel column chromatography (eluent n-hexane-ethyl acetate 90:10 to 70:30 gradient) to give Compound 3 (1.13 g) as a colorless oily substance.
   MS (APCI) 354 [M+H]⁺
(2) To Compound 3 (1.12 g) was added trifluoroacetic acid (15 ml), and the mixture was heated to reflux for 2 hours. The reaction mixture was cooled, and then concentrated under reduced pressure. To the residue were added ethyl acetate and aqueous sodium carbonate solution, and the mixture was separated. The organic layer was washed with saturated saline, dried over anhydrous sodium sulfate, and concentrated under reduced pressure. The residue was purified by silica gel column chromatography (eluent n-hexane-ethyl acetate 50:50 to 0:100 gradient) to give Compound 4 (665 mg) as a colorless powder.
   MS (APCI) 234 [M+H]⁺
(3) To a solution of Compound 4 (661 mg) in ethanol (20 ml) was added 2N-aqueous sodium hydroxide solution (14 ml) under ice-cooling, and the mixture was stirred at room temperature for 3 hours. The mixture was adjusted to pH3 to 4 by the addition of 1N-hydrochloric acid with stirring under ice-cooling. The solvent of the mixture was concentrated to about halves under reduced pressure, and then extracted with chloroform. The extracted layer was washed with saturated saline, and then dried over anhydrous sodium sulfate and concentrated under reduced pressure to give Compound. 5 (507 mg) as a colorless powder.
   MS (APCI) 206[M+H]⁺

### Reference Example 10-1

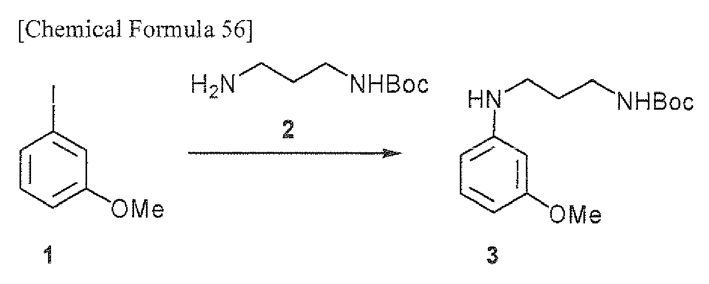

Compound 1 (12.2 g), Compound 2 (10.9 g), cesium acetate (25.0 g) and copper iodide (10.0 g) were suspended in DMSO (50 ml). The suspension was heated to stir at 90°C for 16 hours under argon atmosphere. The reaction mixture was cooled, and then thereto were added 5% brine and 10% ammonia water, and the mixture was extracted with ethyl acetate. The extracted layer was washed with ammonia water and saturated saline, and dried over anhydrous sodium sulfate, and then the solvent was distilled away under reduced pressure. The residue was purified by silica gel column chromatography (eluent n-hexane-ethyl acetate 2:1) to give Compound 3 (8.41 g) as a colorless oily substance.
MS (APCI) 281 [M+H]⁺

### Reference Examples 10-2 to 10-6

The following compounds were prepared by reacting and treating in the similar manner to Reference Example 10-1.

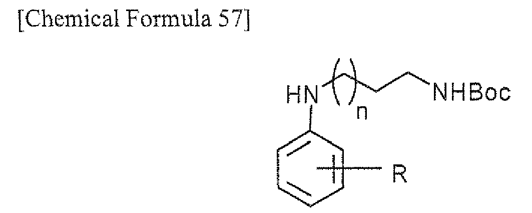

| Reference Example | R | n | MS(APCI) [M+H]⁺ |
|---|---|---|---|
| 10-2 | 4-OMe | 1 | 281 |
| 10-3 | 4-Me | 1 | 265 |
| 10-4 | 3-OMe | 0 | 267 |
| 10-5 | 4-OMe | 0 | 267 |
| 10-6 | 4-Me | 0 | 251 |

### Reference Example 11-1

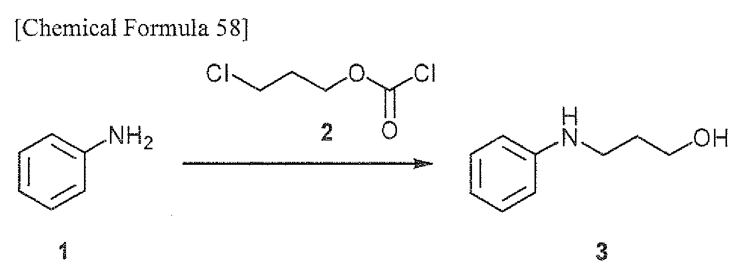

To a solution of Compound 1 (3.78 g) in toluene (50 ml) was added dropwise a solution of Compound 2 (2.50 ml) in toluene (10 ml) under ice-cooling, and the mixture was stirred at room temperature for 2 hours. The precipitate was filtered off, and to the filtrate was added 5% aqueous solution of citric acid, and the mixture was extracted with ethyl acetate. The extracted layer was washed with saturated saline, and dried over anhydrous sodium sulfate, and then the solvent was distilled away under reduced pressure. The resulting oily substance was dissolved in ethanol (25 ml), and then thereto was added potassium hydroxide (5.55 g), and the mixture was heated to reflux for an hour. The reaction mixture was cooled, and then diluted with ethanol. The insoluble was filtered off, and then the filtrate was concentrated under reduced pressure, and the resulting residue was purified by silica gel column chromatography (eluent chloroform -ethyl acetate 10:1) to give Compound 3 (2.92 g) as a pale brown oily substance.

### MS (APCI) 152[M+H]⁺

### Reference Examples 11-2 to 11-10

The following compounds were prepared by reacting and treating in the similar manner to Reference Example 11-1.

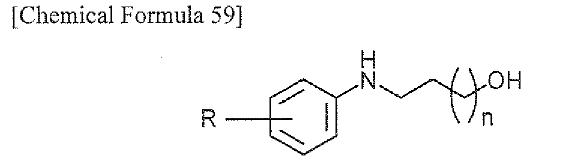

| Reference Example | R | n | MS(APCI) [M+H]+ |
|---|---|---|---|
| 11-2 | 2-OMe | 0 | 168 |
| 11-3 | 3-OMe | 0 | 168 |
| 11-4 | 4-OMe | 0 | 168 |
| 11-5 | 2-Me | 0 | 152 |
| 11-6 | 3-Me | 0 | 152 |
| 11-7 | 4-Me | 0 | 152 |
| 11-8 | 3-MeO | 1 | 182 |
| 11-9 | 4-MeO | 1 | 182 |
| 11-10 | 4-Me | 1 | 166 |

### Reference Example 12-1

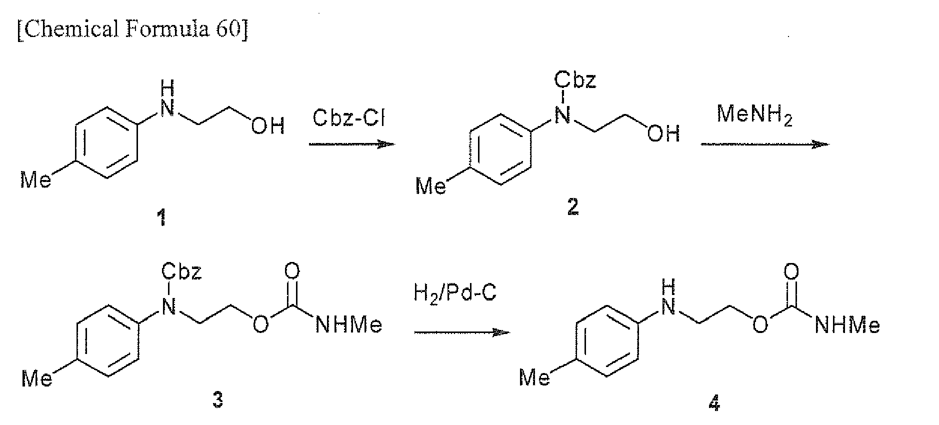

(1) To a solution of Compound 1 (2.27 g) and pyridine (1.82 ml) in chloroform (30 ml) was added dropwise carbobenzoxy chloride (2.36 ml) over 8 minutes under ice-cooling, and the mixture was stirred for an hour. Then, thereto was added concentrated ammonia water (0.3 ml) under ice-cooling, and the mixture was stirred for 10 minutes. Then, thereto was added 1N hydrochloric acid (30 ml), and the mixture was separated. The organic layer was washed with saturated saline, and dried over anhydrous sodium sulfate, and then the solvent was distilled away under reduced pressure. The residue was purified by silica gel column chromatography (eluent n-hexane-ethyl acetate 90:10 to 40:60 gradient) to give Compound 2 (3.70 g) as a colorless oily substance.
   MS (APCI) 286[M+H]⁺
(2) Compound 2 (856 mg) was dissolved in acetonitrile (15 ml), and then thereto were added N,N'-disuccinimidyl carbonate (929 mg) and N,N'-dimethylammopyridine (37 mg), and the mixture was stirred at room temperature for 16 hours. Then, thereto was added 40% aqueous methylamine solution (2.33 g), and the mixture was stirred at room temperature for an hour. To the reaction mixture was added brine, and the mixture was extracted with ethyl acetate. The extracted layer was washed with saturated saline, and dried over anhydrous sodium sulfate, and then the solvent was distilled away under reduced pressure. The residue was purified by silica gel column chromatography (eluent chloroform-ethyl acetate 100:0 to 80:20 gradient) to give Compound 3 (1.01 g) as a colorless oily substance.
   MS (APCI) 343 [M+H]⁺
(3) Compound 3 (0.99 g) was dissolved in ethyl acetate (2 ml) and ethanol (10 ml), and then thereto was added aqueous 10% palladium carbon (0.50 g), and the mixture was stirred vigorously for an hour under hydrogen atmosphere. The palladium carbon was filtered off through Celite and washed with ethanol-ethyl acetate. The filtrate was concentrated under reduced pressure, and the residue was purified by silica gel column chromatography (eluent chloroform-ethyl acetate 100:0 to 70:30 gradient) to give Compound 4 (0.47 g) as a colorless powder.
   MS (APCI) 209[M+H]⁺

### Reference Examples 12-2 to 12-10

The following compounds were prepared by reacting and treating in the similar manner to Reference Example 12-1.

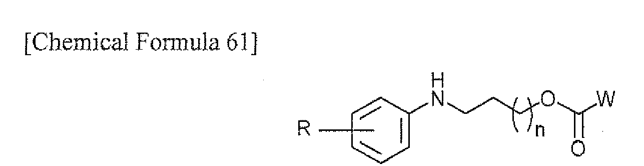

| Reference Example | R | n | W | MS(APCI) [M+H]+ |
|---|---|---|---|---|
| 12-2 | 3-OMe | 0 | -NMe₂ | 239 |
| 12-3 | 3-OMe | 0 | 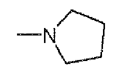 | 265 |
| 12-4 | 4-Me | 0 | -NMe₂ | 223 |
| 12-5 | 4-OMe | 0 | -NMe₂ | 239 |
| 12-6 | 4-Me | 0 | -NHEt | 223 |
| 12-7 | 4-Me | 0 | -NH(CH₂)₂OMe | 253 |
| 12-8 | 4-Me | 1 | -NHMe | 223 |
| 12-9 | 4-Me | 1 | -NMe₂ | 237 |
| 12-10 | 4-OMe | 1 | -NHMe | 239 |

### Reference Example 13-1

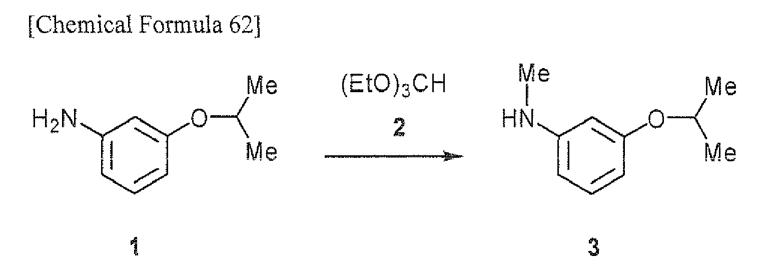

To Compound 1 (1.00 g) were added Compound 2 (3.00 ml) and trifluoroacetic acid (0.05 ml), and the mixture was heated to reflux for 24 hours. The reaction mixture was cooled, and then concentrated under reduced pressure. To a solution of the residue in ethanol (20 ml) was added sodium borohydride (1.00 g) under ice-cooling, and the mixture was stirred for an hour, and then heated to reflux for 2 hours. The reaction mixture was cooled, and then thereto was added water. The solvent was concentrated to about halves under reduced pressure, and extracted with ethyl acetate. The extracted layer was washed with saturated saline, and dried over anhydrous magnesium sulfate, and then the solvent was distilled away under reduced pressure. The residue was purified by silica gel column chromatography (eluent n-hexane-ethyl acetate 100:0 to 50:50 gradient) to give Compound 3 (0.96 g) as a pale yellow oily substance.
MS (APCI) 166[M+H]⁺

### Reference Example 13-2

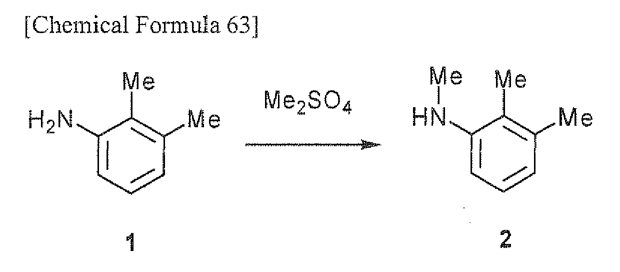

To a suspension of Compound 1 (500 mg) and tetrabutylammonium bromide (67 mg) in toluene (5 ml) were added 15% aqueous sodium hydroxide solution (3.3 g) and dimethylsulfuric acid (0.41 ml) at room temperature, and the mixture was stirred for 19 hours. The reaction mixture was diluted with diethyl ether, and then separated. The organic layer was washed with saturated saline, and dried over anhydrous magnesium sulfate, and then the solvent was distilled away under reduced pressure. The residue was purified by silica gel column chromatography (eluent n-hexane-ethyl acetate 100:0 to 15:85 gradient) to give Compound 2 (167 mg) as a red oily substance.

### MS (APCI) 136[M+H]⁺

### Reference Examples 13-3 to 13-10

The following compounds were prepared by reacting and treating in the similar manner to Reference Examples 13-1 and 13-2.

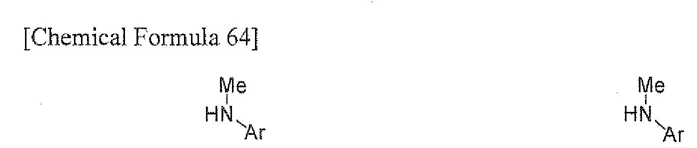

| Reference Example | Ar | MS(APCI) [M+H]+ | Reference Example | Ar | MS(APCI) [M+H]+ |
|---|---|---|---|---|---|
| 13-3 | 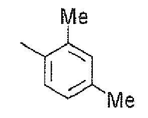 | 136 | 13-7 | 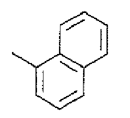 | 158 |
| 13-4 | 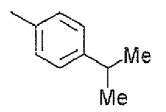 | 150 | 13-8 | 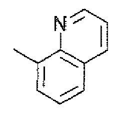 | 159 |
| 13-5 | 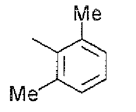 | 136 | 13-9 | 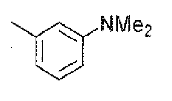 | 151 |
| 13-6 | 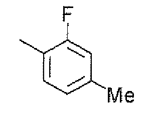 | 140 | 13-10 | 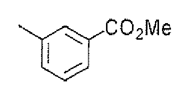 | 166 |

### Reference Example 14-1

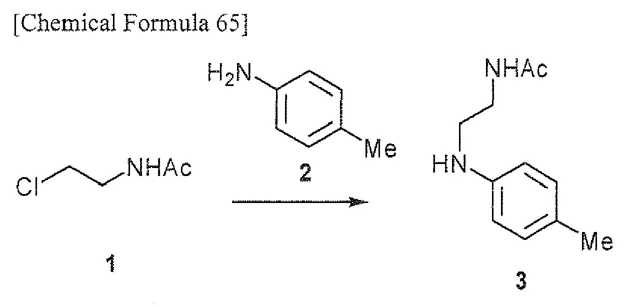

To Compound 1 (800 mg), Compound 2 (2.14 g) and potassium iodide (549 mg) was added toluene (13 ml), and the mixture was heated to reflux for an hour. The reaction mixture was cooled, and then thereto was added sodium bicarbonate water, and the mixture was extracted with ethyl acetate. The extracted layer was washed with saturated saline, and dried over anhydrous magnesium sulfate, and then the solvent was distilled away under reduced pressure. The residue was purified by silica gel column chromatography (eluent n-hexane-ethyl acetate 1:2 to chloroform-methanol 9:1) to give Compound 3 (762 mg) as a pale red oily substance.
MS (APCI) 193[M+H]⁺

### Reference Example 14-2

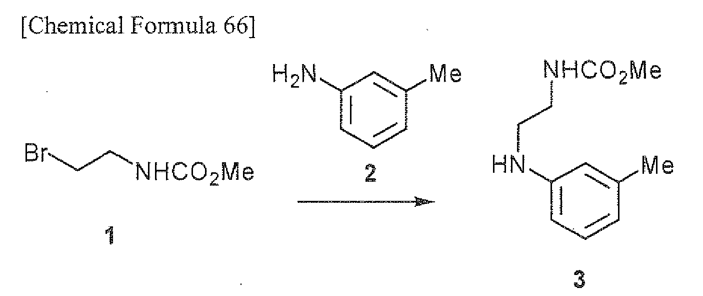

To Compound 1 (1000 mg) synthesized from 2-bromoethylamine hydrobromide was added Compound 2 (1.46 ml), and the mixture was heated to stir at 100°C for 4 hours. The reaction mixture was cooled, and then thereto was added sodium bicarbonate water, and the mixture was extracted with chloroform. The extracted layer was washed with saturated saline, and dried over anhydrous magnesium sulfate, and then the solvent was distilled away under reduced pressure. The residue was purified by silica gel column chromatography (eluent n-hexane-ethyl acetate 80:20 to 60:40 gradient) to give Compound 3 (777 mg) as a yellow oily substance.

### MS (APCI) 209[M+H]⁺

### Reference Examples 14-3 to 14-27

The following compounds were prepared by reacting and treating in the similar manner to Reference Examples 14-1 and 14-2.

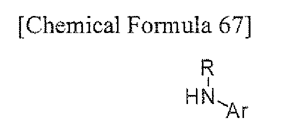

| Reference Example | Ar | R | MS(APCI) [M+H]+ |
|---|---|---|---|
| 14-3 | 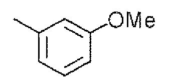 | (CH₂)₄NHBoc | 295 |
| 14-4 | 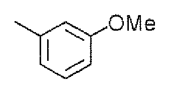 | (CH₂)₃SO₂NMe₂ | 273 |
| 14-5 | 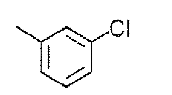 | (CH₂)₃NHSO₂Me | 263/265 |
| 14-6 | 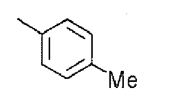 | (CH₂)₃NHSO₂Me | 243 |
| 14-7 | 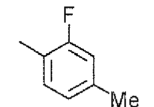 | (CH₂)₃NHSO₂Me | 261 |
| 14-8 | 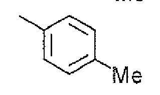 | (CH₂)₃SO₂Me | 228 |
| 14-9 | 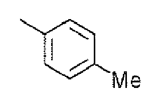 | (CH₂)₃OMe | 180 |
| 14-10 | 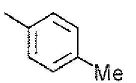 | (CH₂)₂NHCO₂Me | 209 |
| 14-11 | 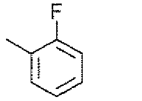 | (CH₂)₂NHCO₂Me | 213 |
| 14-12 | 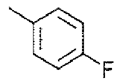 | (CH₂)₂NHCO₂Me | 213 |
| 14-13 | 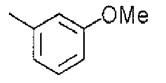 | (CH₂)₂NHCO₂Me | 225 |
| 14-14 | 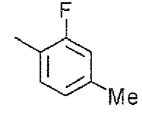 | (CH₂)₂NHCO₂Me | 227 |
| 14-15 | 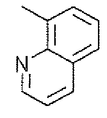 | (CH₂)₂NHCO₂Me | 246 |

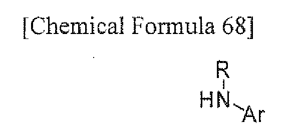

| Reference Example | Ar | R | MS(APCI) [M+H]+ |
|---|---|---|---|
| 14-16 | 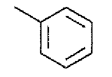 | (CH₂)₂NHCO₂CH(Me)₂ | 223 |
| 14-17 | 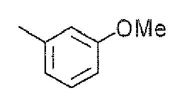 | (CH₂)₂NHAc | 209 |
| 14-18 | 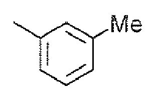 | (CH₂)₂NHAc | 193 |
| 14-19 | 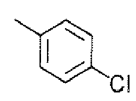 | (CH₂)₂OCONHMe | 229/231 |
| 14-20 | 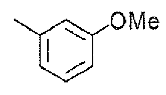 | (CH₂)₂OMe | 182 |
| 14-21 | 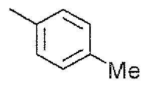 | CH₂CHF₂ | 172 |
| 14-22 | 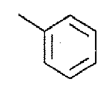 | (CH₂)₂F | 140 |
| 14-23 | 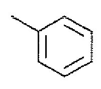 | 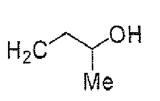 | 152 |
| 14-24 | 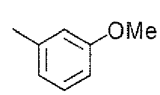 | 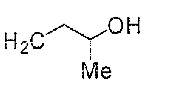 | 182 |
| 14-25 | 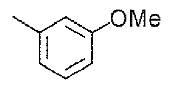 | CH₂CN | 163 |
| 14-26 | 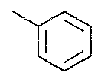 | 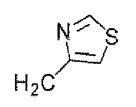 | 191 |
| 14-27 | 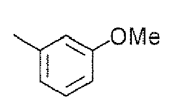 | Et | 152 |

### Reference Example 15-1

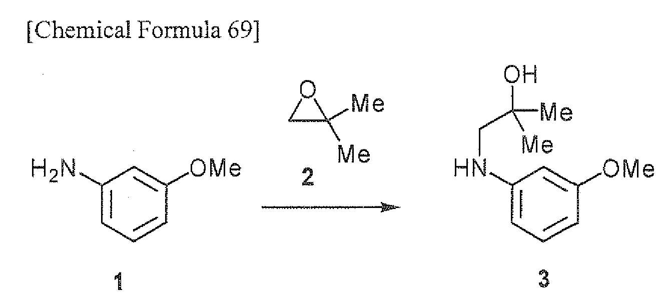

Lithium perchlorate (16.2 g) was suspended in diethyl ether (30 ml), and then thereto were added Compound 1 (1.94 g) and Compound 2 (1.20 g) under ice-cooling. The mixture was stirred at room temperature for 22 hours, and then poured into water and extracted with ethyl acetate. The extracted layer was washed with saturated saline, and dried over anhydrous magnesium sulfate, and then concentrated under reduced pressure. The resulting residue was purified by silica gel column chromatography (eluent n-hexane-ethyl acetate 4:1) to give Compound 3 (2.04 g) as a brown oily substance.
MS (APCI) 196[M+H]⁺

### Reference Example 15-2

The following compound was prepared by reacting and treating in the similar manner to Reference Example 15-1.

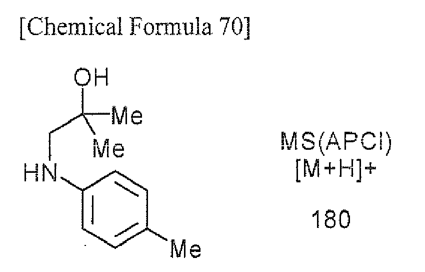

### Reference Example 16

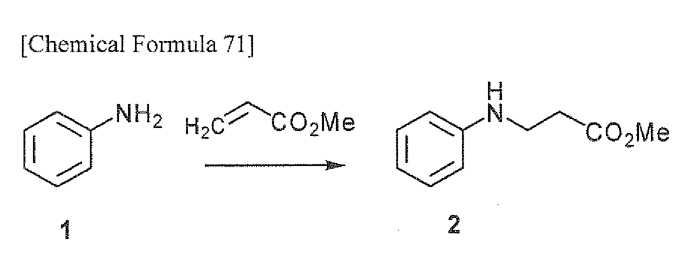

Compound 2 was prepared according to the method of J. Chem. Soc., 4166 (1957). MS (APCI) 180[M+H]⁺

### Reference Example 17-1

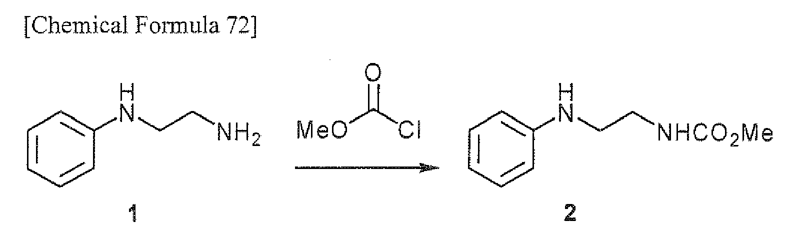

To a solution of Compound 1 (1.36 g) in ethyl acetate (17 ml) were added 10% sodium bicarbonate water (16.8 ml) and methyl chloroformate (1.08 ml) under ice-cooling. The reaction mixture was stirred at room temperature for 2 hours, and then separated. The organic layer was dried over anhydrous magnesium sulfate, and then concentrated under reduced pressure. The resulting residue was purified by silica gel column chromatography (eluent n-hexane-ethyl acetate 3:2) to give Compound 2 (1.00 g) as a yellow oily substance.
MS (APCI) 195 [M+H]⁺

### Reference Example 17-2

The following compound was obtained by reacting and treating in the similar manner to Reference Example 17-1.

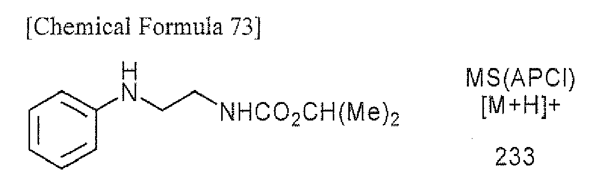

### Reference Example 18-1

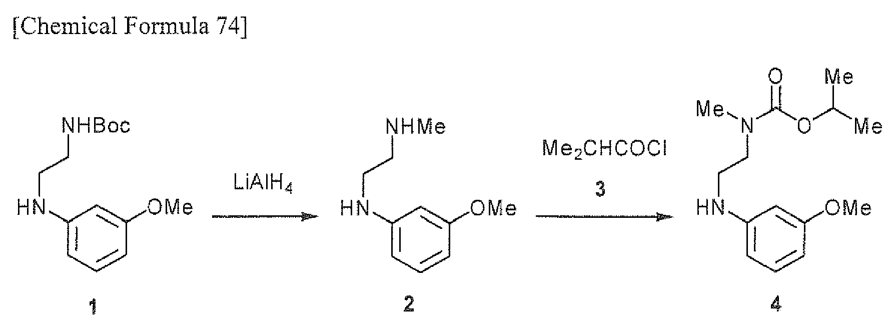

(1) Lithium aluminum hydride (570 mg) was suspended in THF (20 ml), and then thereto was added dropwise a solution of Compound 1 (1000 mg) in THF (10 ml) under ice-cooling. The mixture was heated to reflux for 1 hours. The reaction mixture was cooled, and then thereto were added sequentially water (0.57 ml), 10% aqueous sodium hydroxide solution (0.86 ml) and water (1.42 ml). The mixture was stirred at room temperature for an hour, and then the insoluble was filtered off, and the filtrate was concentrated under reduced pressure to give Compound 2 (680 mg) as a yellow oily substance.
   MS(APCI) 181[M+H]⁺
(2) To a solution of Compound 2 (300 mg) in chloroform (5 ml) was added triethylamine (0.35 ml), and then thereto was added dropwise a solution of Compound 3 (204 mg) in chloroform (4 ml) over 15 minutes under ice-cooling. The mixture was stirred for an hour under ice-cooling, and then thereto was added sodium bicarbonate water, and the mixture was separated. The organic layer was dried over anhydrous magnesium sulfate, and then concentrated under reduced pressure. The resulting residue was purified by silica gel column chromatography (eluent n-hexane-ethyl acetate 80:20 to 55:45 gradient) to give Compound 4 (387 mg) as a colorless oily substance.
   MS (APCI) 267[M+H]⁺

### Reference Example 18-2

The following compound was obtained by reacting and treating in the similar manner to Reference Example 18-1.

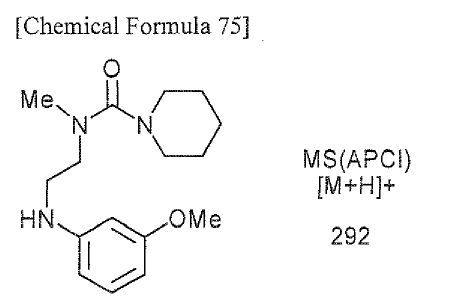

### Reference Example 19-1

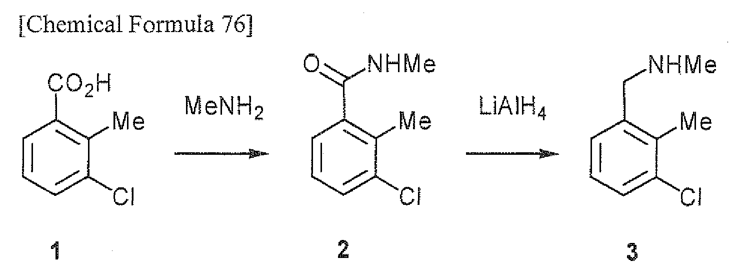

(1) To a solution of Compound 1 (2.56 g) and HOBt (2.2 g) in DMF (50 ml) was added WSC (3.5 g), and the mixture was stirred at room temperature for 2.5 hours. Then, thereto was added 40% aqueous methylamine solution (3.8 ml), and the mixture was stirred at room temperature for 20 hours. The reaction mixture was concentrated under reduced pressure, and then to the residue were added ethyl acetate and water, and the mixture was separated. The organic layer was washed with saturated sodium bicarbonate water and saturated saline, and dried over anhydrous magnesium sulfate, and then concentrated under reduced pressure. The residue was triturated by a mixed solvent of n-hexane-ethyl acetate to give Compound 2 (2.38 g) as a colorless solid.
   MS (APCI) 184/186[M+H]⁺
(2) To a suspension of lithium aluminum hydride (1.24 g) in THF (40 ml) was added Compound 2 (2.00 g) over 3 minutes with stirring at 50°C, and the mixture was heated to reflux for 5.5 hours. The reaction mixture was cooled, and then thereto were added sequentially water (1.24 ml), 10% aqueous sodium hydroxide solution (1.86 ml) and water (3.10 ml). The mixture was stirred at room temperature for an hour, and then the insoluble was filtered off, and the filtrate was concentrated under reduced pressure to give Compound 3 (1.74 g) as a pale yellow oily substance.
   MS (APCI) 170/172[M+H]⁺

### Reference Examples 19-2 to 19-5

The following compounds were obtained by reacting and treating in the similar manner to Reference Example 19-1.

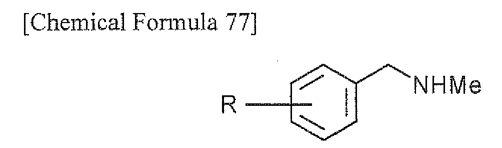

| Reference Example | R | MS(APCI) [M+H]+ |
|---|---|---|
| 19-2 | 2-Me | 136 |
| 19-3 | 2-Me, 5-F | 154 |
| 19-4 | 2-Me, 5-Cl | 170/172 |
| 19-5 | 2-Me,6-F | 154 |

### Reference Example 20

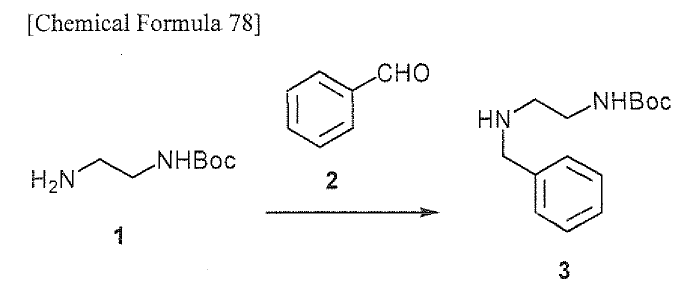

To Compound 1 (1.06 g) and Compound 2 (1.60 g) was added chloroform (30 ml), and then thereto was added sodium triacetoxyborohydride (4.24 g), and the mixture was stirred at room temperature for 3 hours. To the reaction mixture was added aqueous sodium carbonate solution, and the mixture was separated. The organic layer was washed with saturated saline, and dried over anhydrous sodium sulfate, and then the solvent was distilled away under reduced pressure. The residue was purified by silica gel column chromatography (eluent chloroform-methanol 100:0 to 85:15 gradient) to give Compound 3 (2.03 g) as a yellow oily substance.
MS (APCI) 251 [M+H]⁺

The following tables show chemical structures and physical data of the above Example compounds.

**[Table 1-1]**

| | | | | | | | | | | |
|---|---|---|---|---|---|---|---|---|---|---|
| 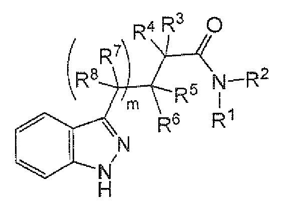 | | | | | | | | | | |

| No. | R¹ | R² | R³ | R⁴ | R⁵ | R⁶ | m | R⁷ | R⁸ | MS(APCI) [M+H]⁺ |
|---|---|---|---|---|---|---|---|---|---|---|
| 01-01 | -(CH₂)₂OH | 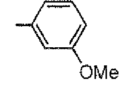 | H | H | H | H | 0 | - | - | 340 |
| 01-02 | -(CH₂)₂OH | 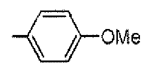 | H | H | H | H | 0 | - | - | 340 |
| 01-03 | -(CH₂)₂OH | 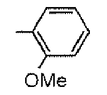 | H | H | H | H | 0 | - | - | 340 |
| 01-04 | -(CH₂)₂OH | 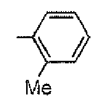 | H | H | H | H | 0 | - | - | 324 |
| 01-05 | -(CH₂)₂OH | 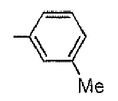 | H | H | H | H | 0 | - | - | 324 |
| 01-06 | -(CH₂)₂OH | 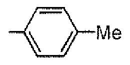 | H | H | H | H | 0 | - | - | 324 |
| 01-07 | -(CH₂)₃OH | 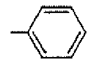 | H | H | H | H | 0 | - | - | 324 |
| 01-08 | -CH₂CH(OH)Me | 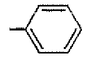 | H | H | H | H | 0 | - | - | 324 |
| 01-09 | -CH₂CH(OH)Me | 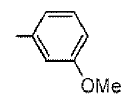 | H | H | H | H | 0 | - | - | 354 |
| 01-10 | -CH₂C(Me₂)OH | 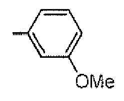 | H | H | H | H | 0 | - | - | 368 |
| 01-11 | -(CH₂)₂OMe | 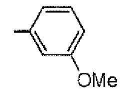 | H | H | H | H | 0 | - | - | 354 |
| 01-12 | Et | 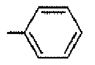 | H | H | H | H | 0 | - | - | 294 |
| 01-13 | Bu | 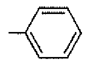 | H | H | H | H | 0 | - | - | 322 |

**[Table 1-2]**

| | | | | | | | | | | |
|---|---|---|---|---|---|---|---|---|---|---|
| 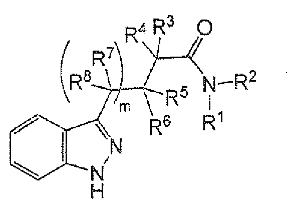 | | | | | | | | | | |

| No. | R¹ | R² | R³ | R⁴ | R⁵ | R⁶ | m | R⁷ | R⁸ | MS(APCI) [M+H]⁺ |
|---|---|---|---|---|---|---|---|---|---|---|
| 01-14 | Me | 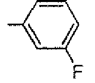 | H | H | H | H | 0 | - | - | 298 |
| 01-15 | Me | 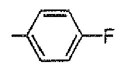 | H | H | H | H | 0 | - | - | 298 |
| 01-16 | 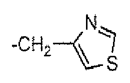 | 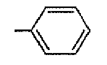 | H | H | H | H | 0 | - | - | 363 |
| 01-17 | -(CH₂)₂NHAc | 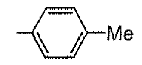 | H | H | H | H | 0 | - | - | 365 |
| 01-18 | -(CH₂)₂NHAc | 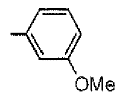 | H | H | H | H | 0 | - | - | 381 |
| 01-19 | -(CH₂)₂NHAc | 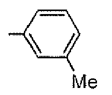 | H | H | H | H | 0 | - | - | 365 |
| 01-20 | -(CH₂)₂F | 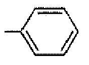 | H | H | H | H | 0 | - | - | 312 |
| 01-21 | -(CH₂)₃SO₂Me | 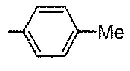 | H | H | H | H | 0 | - | - | 400 |
| 01-22 | -CH₂CN | 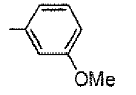 | H | H | H | H | 0 | - | - | 335 |
| 01-23 | -(CH₂)₂OH | 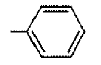 | H | H | H | H | 0 | - | - | 310 |
| 01-24 | Me | 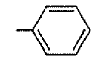 | H | H | H | H | 0 | - | - | 280 |

**[Table 1-3]**

| | | | | | | | | | | |
|---|---|---|---|---|---|---|---|---|---|---|
| 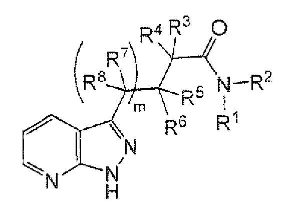 | | | | | | | | | | |

| No. | R¹ | R² | R³ | R⁴ | R⁵ | R⁶ | m | R⁷ | R⁸ | MS(APCI) [M+H]⁺ |
|---|---|---|---|---|---|---|---|---|---|---|
| 02-01 | -(CH₂)₂OCONHMe | 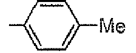 | H | H | H | H | 0 | - | - | 382 |
| 02-02 | Me | 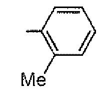 | H | H | H | H | 0 | - | - | 295 |
| 02-03 | Me | 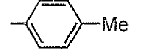 | H | H | H | H | 0 | - | - | 295 |
| 02-04 | -(CH₂)₄NHCOO^{t}Bu | 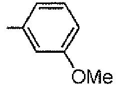 | H | H | H | H | 0 | - | - | 468 |
| 02-05 | Me | 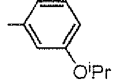 | H | H | H | H | 0 | - | - | 339 |
| 02-06 | -(CH₂)₃SO₂NMe₂ | 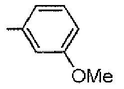 | H | H | H | H | 0 | - | - | 446 |
| 02-07 | -(CH₂)₂NHCOOⁱPr | 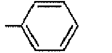 | H | H | H | H | 0 | - | - | 396 |
| 02-08 | Me | 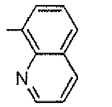 | H | H | H | H | 0 | - | - | 332 |
| 02-09 | Me | 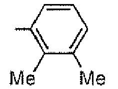 | H | H | H | H | 0 | - | - | 309 |
| 02-10 | Me | 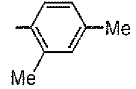 | H | H | H | H | 0 | - | - | 309 |
| 02-11 | Me | 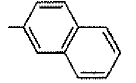 | H | H | H | H | 0 | - | - | 331 |
| 02-12 | Me | 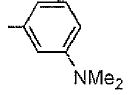 | H | H | H | H | 0 | - | - | 324 |
| 02-13 | Me | 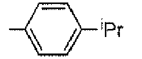 | H | H | H | H | 0 | - | - | 323 |

**[Table 1-4]**

| | | | | | | | | | | |
|---|---|---|---|---|---|---|---|---|---|---|
| 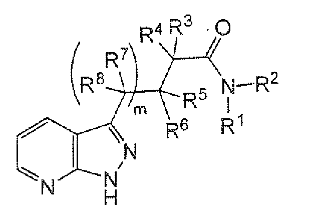 | | | | | | | | | | |

| No. | R¹ | R² | R³ | R⁴ | R⁵ | R⁶ | m | R⁷ | R⁸ | MS(APCI) [M+H]⁺ |
|---|---|---|---|---|---|---|---|---|---|---|
| 02-14 | Et | 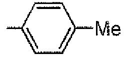 | H | H | H | H | 0 | - | - | 309 |
| 02-15 | -(CH₂)₂OCONMe₂ | 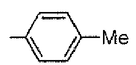 | H | H | H | H | 0 | - | - | 396 |
| 02-16 | -(CH₂)₂OCONMe₂ | 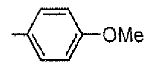 | H | H | H | H | 0 | - | - | 412 |
| 02-17 | Me | 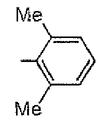 | H | H | H | H | 0 | - | - | 309 |
| 02-18 | -(CH₂)₃OCONHMe | 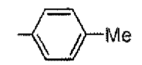 | H | H | H | H | 0 | - | - | 396 |
| 02-19 | -(CH₂)₃OCONMe₂ | 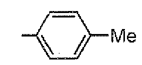 | H | H | H | H | 0 | - | - | 410 |
| 02-20 | -(CH₂)₃OCONHMe | 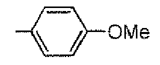 | H | H | H | H | 0 | - | - | 412 |
| 02-21 | -(CH₂)₂OCONHEt | 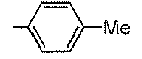 | H | H | H | H | 0 | - | - | 396 |
| 02-22 | Me | 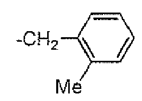 | H | H | H | H | 0 | - | - | 309 |
| 02-23 | -(CH₂)₂NHCOOMe | 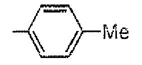 | H | H | H | H | 0 | - | - | 382 |
| 02-24 | -(CH₂)₂OH | 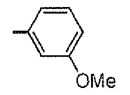 | H | H | H | H | 0 | - | - | 341 |
| 02-25 | Me | 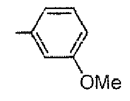 | H | H | H | H | 0 | - | - | 311 |
| 02-26 | Me | 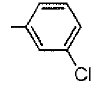 | H | H | H | H | 0 | - | - | 315/317 |

**[Table 1-5]**

| 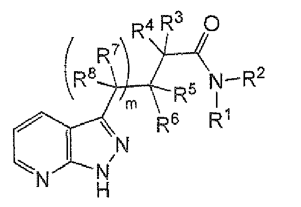 | | | | | | | | | | |
|---|---|---|---|---|---|---|---|---|---|---|
| No. | R¹ | R² | R³ | R⁴ | R⁵ | R⁶ | m | R⁷ | R⁸ | MS(APCI) [M+H]⁺ |
| 02-27 | Me | 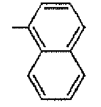 | H | H | H | H | 0 | - | - | 331 |
| 02-28 | Me | 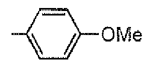 | H | H | H | H | 0 | - | - | 311 |
| 02-29 | Me | 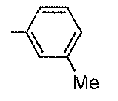 | H | H | H | H | 0 | - | - | 295 |
| 02-30 | Et | 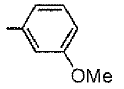 | H | H | H | H | 0 | - | - | 325 |
| 02-31 | -(CH₂)₂N(Me)COOⁱPr | 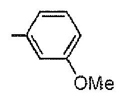 | H | H | H | H | 0 | - | - | 440 |
| 02-32 | 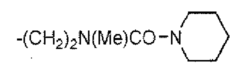 | 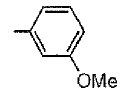 | H | H | H | H | 0 | - | - | 465 |
| 02-33 | -(CH₂)₂OCONMe₂ | 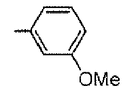 | H | H | H | H | 0 | - | - | 412 |
| 02-34 | 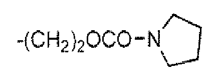 | 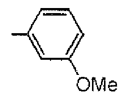 | H | H | H | H | 0 | - | - | 438 |

**[Table 1-6]**

| | | | | | | | | | | | |
|---|---|---|---|---|---|---|---|---|---|---|---|
| 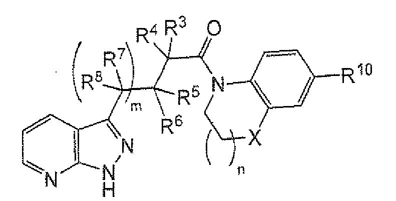 | | | | | | | | | | | |

| No. | R¹⁰ | n | R³ | R⁴ | R⁵ | R⁶ | m | R⁷ | R⁸ | X | MS(APCI) [M+H]⁺ |
|---|---|---|---|---|---|---|---|---|---|---|---|
| 02-35 | H | 1 | H | H | H | H | 0 | - | - | CH₂ | 307 |
| 02-36 | Me | 1 | H | H | H | H | 0 | - | - | CH₂ | 321 |
| 02-37 | H | 2 | H | H | H | H | 0 | - | - | CH₂ | 321 |
| 02-38 | H | 1 | H | H | H | H | 0 | - | - | O | 308 |

**[Table 1-7]**

| 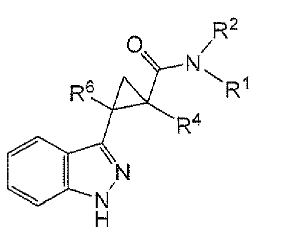 | | | | | |
|---|---|---|---|---|---|
| No. | R¹ | R² | R⁴ | R⁶ | MS(APCI) [M+H]⁺ |
| 03-01 | -(CH₂)₂NHAc | 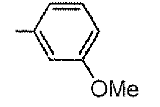 | H | H | 393 |
| 03-02 | -(CH₂)₂OH | 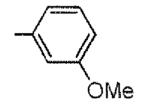 | H | H | 352 |

**[Table 1-8]**

| | | | | | |
|---|---|---|---|---|---|
| 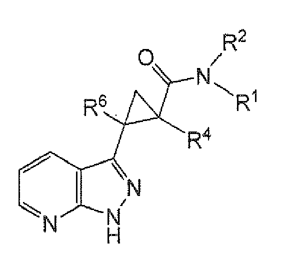 | | | | | |

| No. | R¹ | R² | R⁴ | R⁶ | MS(APCI) [M+H]⁺ |
|---|---|---|---|---|---|
| 04-01 | Me | 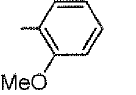 | H | H | 323 |
| 04-02 | -(CH₂)₂NHCOOMe | 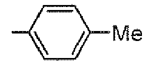 | H | H | 394 |
| 04-03 | Me | 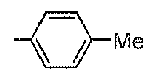 | H | H | 307 |
| 04-04 | -(CH₂)₂OCONHMe | 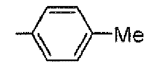 | H | H | 394 |
| 04-05 | Me | 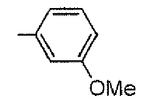 | H | H | 323 |
| 04-06 | -CH₂C(Me)₂OH | 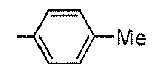 | H | H | 365 |
| 04-07 | -(CH₂)₃OCONHMe | 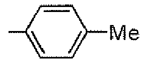 | H | H | 408 |
| 04-08 | -(CH₂)₂OCONMe₂ | 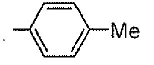 | H | H | 408 |

**[Table 1-9]**

| | | | | | | | | | | |
|---|---|---|---|---|---|---|---|---|---|---|
| 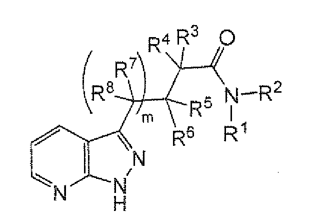 | | | | | | | | | | |

| No. | R¹ | R² | R³ | R⁴ | R⁵ | R⁶ | m | R⁷ | R⁸ | MS(APCI) [M+H]⁺ |
|---|---|---|---|---|---|---|---|---|---|---|
| 05-01 | Me | 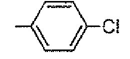 | Me | H | H | H | 0 | - | - | 329/331 |
| 05-02 | Me | 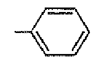 | Me | H | H | H | 0 | - | - | 295 |
| 05-03 | -(CH₂)₂NHCOOMe | 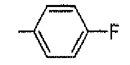 | Me | H | H | H | 0 | - | - | 400 |
| 05-04 | Me | 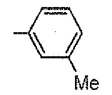 | Me | H | H | H | 0 | - | - | 309 |
| 05-05 | Me | 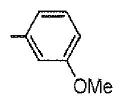 | Me | H | H | H | 0 | - | - | 325 |
| 05-06 | Me | 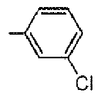 | Me | H | H | H | 0 | - | - | 329/331 |
| 05-07 | Me | 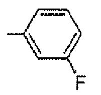 | Me | H | H | H | 0 | - | - | 313 |
| 05-08 | -(CH₂)₂NHCOOMe | 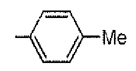 | Me | H | H | H | 0 | - | - | 396 |
| 05-09 | Me | 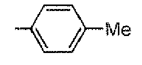 | Me | H | H | H | 0 | - | - | 309 |

**[Table 1-10]**

| | | | | | | | | | | |
|---|---|---|---|---|---|---|---|---|---|---|
| 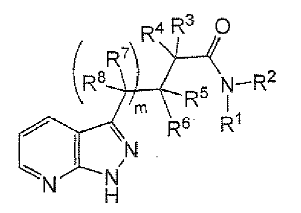 | | | | | | | | | | |

| No. | R¹ | R² | R³ | R⁴ | R⁵ | R⁶ | m | R⁷ | R⁸ | MS(APCI) [M+H]⁺ |
|---|---|---|---|---|---|---|---|---|---|---|
| 06-01 | Me | 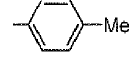 | Me | Me | H | H | 0 | - | - | 323 |
| 06-02 | Me | 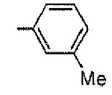 | Me | Me | H | H | 0 | - | - | 323 |
| 06-03 | -(CH₂)₃NHSO₂Me | 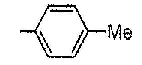 | Me | Me | H | H | 0 | - | - | 444 |
| 06-04 | Me | 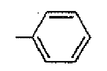 | Me | Me | H | H | 0 | - | - | 309 |
| 06-05 | Me | 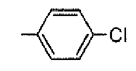 | Me | Me | H | H | 0 | - | - | 343/345 |

**[Table 1-11]**

| | | | | | | | | | | |
|---|---|---|---|---|---|---|---|---|---|---|
| 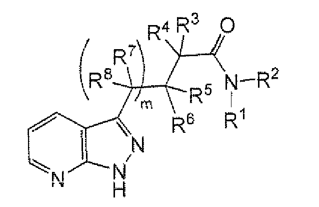 | | | | | | | | | | |

| No. | R¹ | R² | R³ | R⁴ | R⁵ | R⁶ | m | R⁷ | R⁸ | MS(APCI) [M+H]⁺ |
|---|---|---|---|---|---|---|---|---|---|---|
| 07-01 | Me | 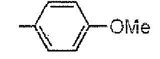 | H | H | Me | H | 0 | - | - | 325 |
| 07-02 | -(CH₂)₃NHSO₂Me | 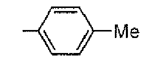 | H | H | Me | H | 0 | - | - | 430 |
| 08-01 | -(CH₂)₂NHCOOMe | 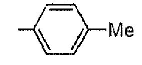 | H | H | Et | H | 0 | - | - | 410 |

**[Table 1-12]**

| | | | | | | | | | | |
|---|---|---|---|---|---|---|---|---|---|---|
| 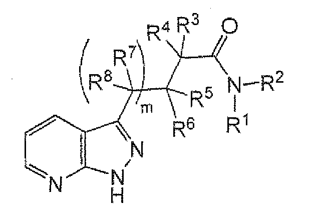 | | | | | | | | | | |

| No. | R¹ | R² [M+H]⁺ | R³ | R⁴ | R⁵ | R⁶ | m | R⁷ | R⁸ | MS(APCI) |
|---|---|---|---|---|---|---|---|---|---|---|
| 09-01 | -(CH₂)₃OCONHMe | 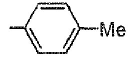 | H | H | Me | Me | 0 | - | - | 424 |
| 09-02 | Me | 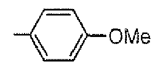 | H | H | Me | Me | 0 | - | - | 339 |
| 09-03 | Me | 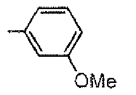 | H | H | Me | Me | 0 | - | - | 339 |
| 09-04 | -(CH₂)₂NHCOOMe | 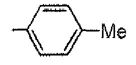 | H | H | Me | Me | 0 | - | - | 410 |
| 09-05 | Me | 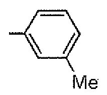 | H | H | Me | Me | 0 | - | - | 323 |
| 09-06 | Me | 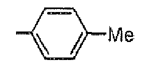 | H | H | Me | Me | 0 | - | - | 323 |
| 09-07* | Me | 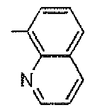 | H | H | Me | Me | 0 | - | - | 360 |
| 09-08 | Me | 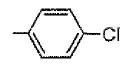 | H | H | Me | Me | 0 | - | - | 343/345 |
| 09-09 | -(CH₂)₂OCONHMe | 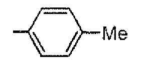 | H | H | Me | Me | 0 | - | - | 410 |
| 09-10 | -(CH₂)₂NHCOOMe | 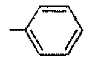 | H | H | Me | Me | 0 | - | - | 396 |
| 09-11 | -(CH₂)₂NHCOOMe | 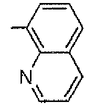 | H | H | Me | Me | 0 | - | - | 447 |
| 09-12 | Me | 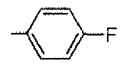 | H | H | Me | Me | 0 | - | - | 327 |
| 09-13 | Me | 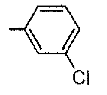 | H | H | Me | Me | 0 | - | - | 343/345 |

| | | | | | | | | | | |
|---|---|---|---|---|---|---|---|---|---|---|
| *: methanesulfonate | | | | | | | | | | |

**[Table 1-13]**

| | | | | | | | | | | |
|---|---|---|---|---|---|---|---|---|---|---|
| 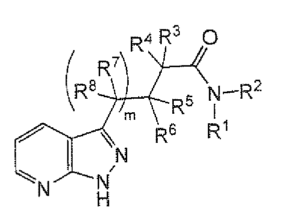 | | | | | | | | | | |

| No. | R¹ | R² | R³ | R⁴ | R⁵ | R⁶ | m | R⁷ | R⁸ | MS(APCI) [M+H]⁺ |
|---|---|---|---|---|---|---|---|---|---|---|
| 09-14 | Me | 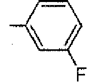 | H | H | Me | Me | 0 | - | - | 327 |
| 09-15 | Me | 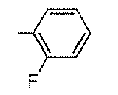 | H | H | Me | Me | 0 | - | - | 327 |
| 09-16 | Me | 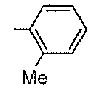 | H | H | Me | Me | 0 | - | - | 323 |
| 09-17 | Me | 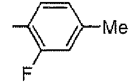 | H | H | Me | Me | 0 | - | - | 341 |
| 09-18 | -(CH₂)₂NHCOOMe | 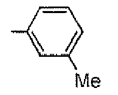 | H | H | Me | Me | 0 | - | - | 410 |
| 09-19 | -(CH₂)₂NHCOOMe | 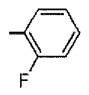 | H | H | Me | Me | 0 | - | - | 414 |
| 09-20 | -(CH₂)₂NHCOOMe | 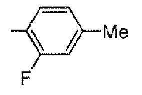 | H | H | Me | Me | 0 | - | - | 428 |
| 09-21 | Me | 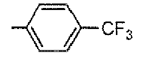 | H | H | Me | Me | 0 | - | - | 377 |
| 09-22 | -(CH₂)₃NHSO₂Me | 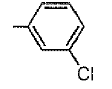 | H | H | Me | Me | 0 | - | - | 464/466 |
| 09-23 | -(CH₂)₃OMe | 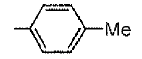 | H | H | Me | Me | 0 | - | - | 381 |
| 09-24 | -(CH₂)₂OCONH(CH₂)₂OMe | 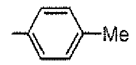 | H | H | Me | Me | 0 | - | - | 454 |

**[Table 1-14]**

| | | | | | | | | | | |
|---|---|---|---|---|---|---|---|---|---|---|
| 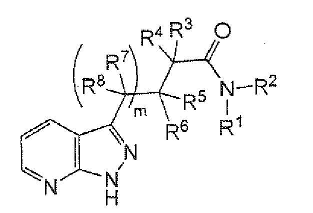 | | | | | | | | | | |

| No. | R¹ | R² | R³ | R⁴ | R⁵ | R⁶ | m | R⁷ | R⁸ | MS(APCl) [M+H]⁺ |
|---|---|---|---|---|---|---|---|---|---|---|
| 09-25 | -CH₂CHF₂ | 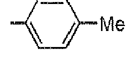 | H | H | Me | Me | 0 | - | - | 373 |
| 09-26 | -(CH₂)₂OCONHMe | 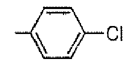 | H | H | Me | Me | 0 | - | - | 430/432 |
| 09-27 | Me | 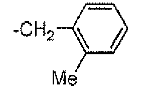 | H | H | Me | Me | 0 | - | - | 337 |
| 09-28 | Me | 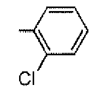 | H | H | Me | Me | 0 | - | - | 343/345 |
| 09-29 | -(CH₂)₃NHSO₂Me | 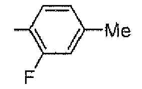 | H | H | Me | Me | 0 | - | - | 462 |
| 09-30 | Me | 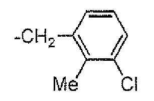 | H | H | Me | Me | 0 | - | - | 371/373 |
| 09-31 | Me | 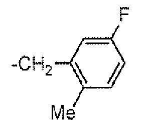 | H | H | Me | Me | 0 | - | - | 355 |
| 09-32 | Me | 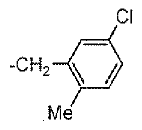 | H | H | Me | Me | 0 | - | - | 371/373 |
| 09-33 | Me | 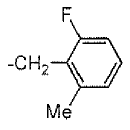 | H | H | Me | Me | 0 | - | - | 355 |

**[Table 1-15]**

| | | | | | | | | | | |
|---|---|---|---|---|---|---|---|---|---|---|
| 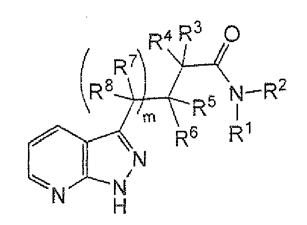 | | | | | | | | | | |

| No. | R¹ | R² | R³ | R⁴ | R⁵ | R⁶ | m | R⁷ | R⁸ | MS(APCI) [M+H]⁺ |
|---|---|---|---|---|---|---|---|---|---|---|
| 10-01 | Me | 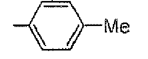 | H | H | H | H | 1 | H | H | 309 |
| 10-02 | -(CH₂)₂OCONHMe | 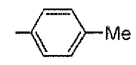 | H | H | H | H | 1 | H | H | 396 |
| 10-03 | Me | 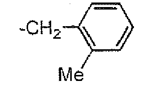 | H | H | H | H | 1 | H | H | 323 |
| 10-04 | Me | 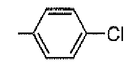 | H | H | H | H | 1 | H | H | 329/331 |
| 10-05 | -(CH₂)₃NHSO₂Me | 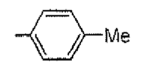 | H | H | H | H | 1 | H | H | 430 |

**[Table 1-16]**

| | | | | | | | | | | |
|---|---|---|---|---|---|---|---|---|---|---|
| 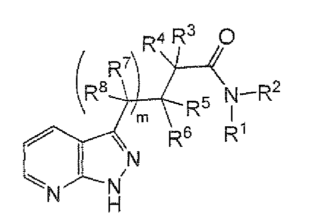 | | | | | | | | | | |

| No. | R¹ | R² | R³ | R⁴ | R⁵ | R⁶ m | | R⁷ | R⁸ | MS(APCl) [M+H]⁺ |
|---|---|---|---|---|---|---|---|---|---|---|
| 11-01 | -(CH₂)₃NHCOO^{t}Bu | 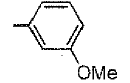 | H | H | H | H | 0 | - | - | 454 |
| 11-02 | -(CH₂)₃NHCOOMe | 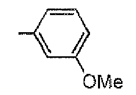 | H | H | H | H | 0 | - | - | 412 |
| 11-03 | -(CH₂)₃NHCOOCH(Me)Et | 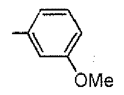 | H | H | H | H | 0 | - | - | 454 |
| 11-04 | -(CH₂)₃NHCO^{j}Pr | 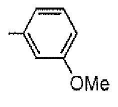 | H | H | H | H | 0 | - | - | 424 |
| 11-05 | -(CH₂)₃NHCO(CH₂)₂CHMe₂ | 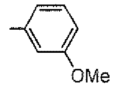 | H | H | H | H | 0 | - | - | 452 |
| 11-06 | 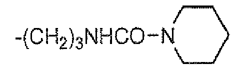 | 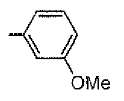 | H | H | H | H | 0 | - | - | 465 |
| 11-07 | -(CH₂)₃NHSO₂Et | 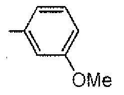 | H | H | H | H | 0 | - | - | 446 |
| 11-08 | -(CH₂)₃NHSO₂NMe₂ | 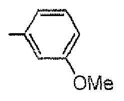 | H | H | H | H | 0 | - | - | 461 |
| 11-09 | -(CH₂)₃NHCOOEt | 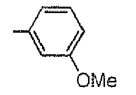 | H | H | H | H | 0 | - | - | 426 |
| 11-10 | -(CH₂)₃NHCOO¹Pr | 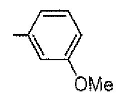 | H | H | H | H | 0 | - | - | 440 |
| 11-11 | -(CH₂)₃NHSO₂(CH₂)₂OMe | 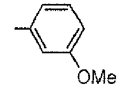 | H | H | H | H | 0 | - | - | 476 |

**[Table 1-17]**

| | | | | | | | | | | |
|---|---|---|---|---|---|---|---|---|---|---|
| 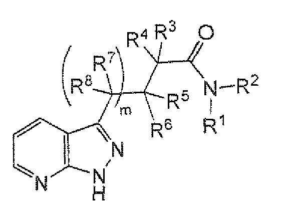 | | | | | | | | | | |

| No. | R¹ | R² | R³ | R⁴ | R⁵ | R⁶ | m | R⁷ | R⁸ | MS(APCl) [M+H]⁺ [M+H]⁺ |
|---|---|---|---|---|---|---|---|---|---|---|
| 11-12 | -(CH₂)₃NHCOPh | 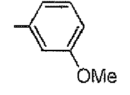 | H | H | H | H | 0 | - | - | 458 |
| 11-13 | -(CH₂)₃NHCOCH₂Ph | 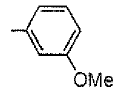 | H | H | H | H | 0 | - | - | 472 |
| 11-14 | -(CH₂)₃NHCOCH(Me)F | 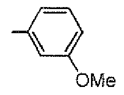 | H | H | H | H | 0 | - | - | 428 |
| 11-15 | 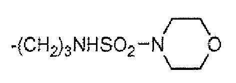 | 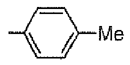 | H | H | H | H | 0 | - | - | 487 |
| 11-16 | -(CH₂)₃NHCOCF₃ | 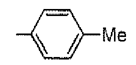 | H | H | H | H | 0 | - | - | 434 |
| 11-17 | -(CH₂)₃NHCOⁱPr | 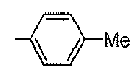 | H | H | H | H | 0 | - | - | 408 |
| 11-18 | -(CH₂)₃NHSO₂Et | 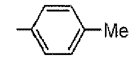 | H | H | H | H | 0 | - | - | 430 |
| 11-19 | -(CH₂)₃NHSO₂Me | 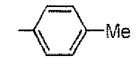 | H | H | H | H | 0 | - | - | 416 |
| 11-20 | -(CH₂)₃NHCOOMe | 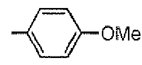 | H | H | H | H | 0 | - | - | 412 |

**[Table 1-18]**

| | | | | | | | | | | |
|---|---|---|---|---|---|---|---|---|---|---|
| 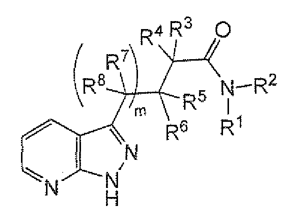 | | | | | | | | | | |

| No. | R¹ | R² | R³ | R⁴ | R⁵ | R⁶ | m | R⁷ | R⁸ | MS(APCl) [M+H]⁺ [M+H]⁺ |
|---|---|---|---|---|---|---|---|---|---|---|
| 11-21 | -(CH₂)₃NHCOOⁱPr | 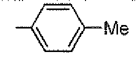 | H | H | H | H | 0 | - | - | 424 |
| 11-22 | -(CH₂)₃NHSO₂(CH₂)₂OMe | 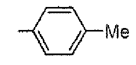 | H | H | H | H | 0 | - | - | 460 |
| 11-23 | -(CH₂)₃NHSO₂Me | 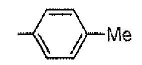 | H | H | Me | Me | 0 | - | - | 444 |
| 11-24 | -(CH₂)₃NHSO₂(CH₂)₂OMe | 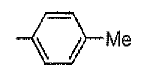 | H | H | Me | Me | 0 | - | - | 488 |
| 11-25 | -(CH₂)₃NHCOOMe | 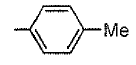 | H | H | Me | Me | 0 | - | - | 424 |
| 11-26 | -(CH₂)₃NHCOOMe | 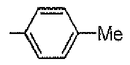 | H | H | H | H | 0 | - | - | 396 |
| 11-27 | -(CH₂)₃NHCOEt | 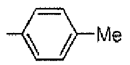 | H | H | Me | Me | 0 | - | - | 422 |
| 11-28 | -(CH₂)₃NHCONMe₂ | 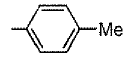 | Me | H | H | H | 0 | - | - | 423 |
| 11-29 | -(CH₂)₃NHCOCF₃ | 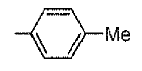 | H | H | Me | Me | 0 | - | - | 462 |

**[Table 1-19]**

| | | | | | | | | | | |
|---|---|---|---|---|---|---|---|---|---|---|
| 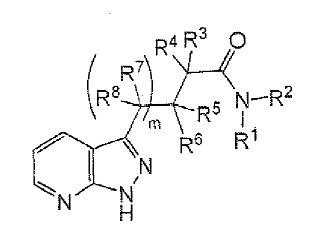 | | | | | | | | | | |

| No. | R¹ | R² | R³ | R⁴ | R⁵ | R⁶ | m | R⁷ | R⁸ | MS(APCl) [M+H]⁺ |
|---|---|---|---|---|---|---|---|---|---|---|
| 12-01 1 | -(CH₂)₂NHCOOMe | 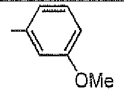 | H | H | H | H | 0 | - | - | 398 |
| 12-02 | -(CH₂)₂NHCOOⁱPr | 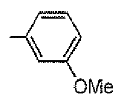 | H | H | H | H | 0 | - | - | 426 |
| 12-03 | -(CH₂)₂NHCOOⁱBu | 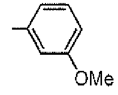 | H | H | H | H | 0 | - | - | 440 |
| 12-04 | -(CH₂)₂NHSO₂NMe₂ | 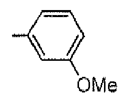 | H | H | H | H | 0 | - | - | 447 |
| 12-05 | 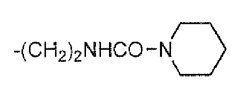 | 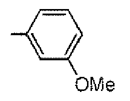 | H | H | H | H | 0 | - | - | 451 |
| 12-06 | -(CH₂)₂NHCOOCH(Me)Et | 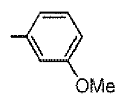 | H | H | H | H | 0 | - | - | 440 |
| 12-07 | -(CH₂)₂NHCOPh | 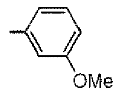 | H | H | H | H | 0 | - | - | 444 |
| 12-08 | -(CH₂)₂NHCOCH₂Ph | 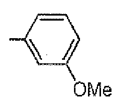 | u | H | H | H | 0 | - | - | 458 |
| 12-09 | -(CH₂)₂NHCO(CH₂)₂CHMe₂ | 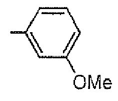 | H | H | H | H | 0 | - | - | 438 |
| 12-10 | -(CH₂)₂NHCOOMe | 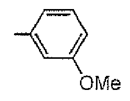 | H | H | Me | Me | 0 | - | - | 426 |
| 12-11 | -(CH₂)₂NHCOOⁱPr | 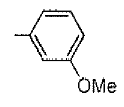 | H | H | Me | Me | 0 | - | - | 454 |

**[Table 1-20]**

| | | | | | | | | | | |
|---|---|---|---|---|---|---|---|---|---|---|
| 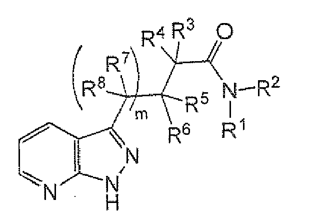 | | | | | | | | | | |

| No. | R¹ | R² | R³ | R⁴ | R⁵ | R⁶ | m | R⁷ | R⁸ | MS(APCl) [M+H]⁺ |
|---|---|---|---|---|---|---|---|---|---|---|
| 12-12 | -(CH₂)₂NHCOO(CH₂)₂OⁱPr | 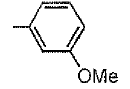 | H | H | H | H | 0 | - | - | 470 |
| 12-13 | 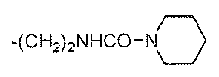 | 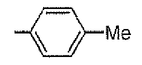 | H | H | H | H | 0 | - | - | 435 |
| 12-14 | 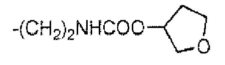 | 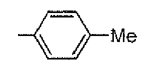 | H | H | H | H | 0 | - | - | 438 |
| 12-15 | -(CH₂)₂NHCOOⁱpr | 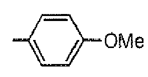 | H | H | H | H | 0 | - | - | 426 |
| 12-16 | -(CH₂)₂NHCOOⁱPr | 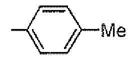 | H | H | H | H | 0 | - | - | 410 |
| 12-17 | -(CH₂)₂NHSO₂Me | 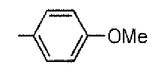 | H | H | H | H | 0 | - | - | 418 |
| 12-18 | 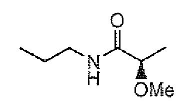 | 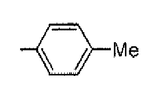 | H | H | H | H | 0 | - | - | 410 |
| 12-19 | 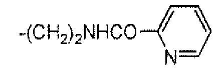 | 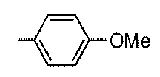 | H | H | H | H | 0 | - | - | 445 |
| 12-20 | .-(CH₂)₂NHCOPh | 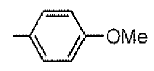 | H | H | H | H | 0 | - | - | 444 |
| 12-21 | -(CH₂)₂NHCOEt | 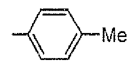 | H | H | Me | Me | 0 | - | - | 408 |
| 12-22 | -(CH₂)₂NHCOEt | 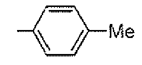 | H | H | H | H | 0 | - | - | 380 |

**[Table 1-21]**

| | | | | | | | | | | |
|---|---|---|---|---|---|---|---|---|---|---|
| 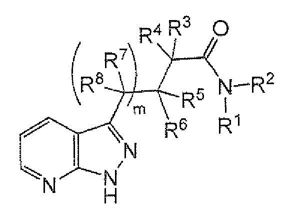 | | | | | | | | | | |

| No. | R¹ | R² | R³ | R⁴ | R⁵ | R⁶ | m | R⁷ | R⁸ | MS(APCl) [M+H]⁺ |
|---|---|---|---|---|---|---|---|---|---|---|
| 13-01 | -(CH₂)₃OCONMe₂ | 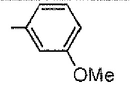 | H | H | H | H | 0 | - | - | 426 |
| 14-01 | -(CH₂)₂CONHBu | 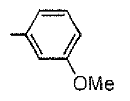 | H | H | H | H | 0 | - | - | 424 |
| 15-01 | Me | 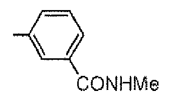 | H | H | H | H | 0 | - | - | 338 |
| 16-01 | -(CH₂)₂NHSO₂Me | 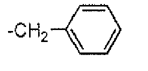 | H | H | H | H | 1 | H | H | 416 |

### Experiment 1: Effects on atrial effective refractory periods (ERP) in anesthetized dogs

### (1) Surgery

Mongrel dogs of either sex were anesthesized by intravenously administering pentobarbital sodium (induction: 30 mg/kg, continuance: 5 mg/kg/hr) and cannulated into their respiratory tracts to give artificial respiration (15 cc x 20 cycles/min). Catheters for continuous anesthesia and sample administration were respectively inserted into their ambilateral median antebrachial veins. A catheter was inserted into left femoral veins, and KN fluid replacement 3B (Otsuka Pharmaceutical Co., Ltd.) was continuously administered (50 to 100 ml/hr). Blood pressure was measured by a pressure-distortion amplifier via a pressure transducer from a catheter inserted into left femoral artery, and heart rate was measured by pulse wave as a trigger, respectively. After median thoracotomy, a pericardium was dissected to expose heart, and electrodes for electrical stimulation and myocardial electrographic measurement were installed in atrium. An electrode for electrocardiographic measurement was installed on body surface (Induction II).

### (2) Measurement of ERP

Atrial ERP was measured by using S1-S2 extrastimulus technique. A fundamental stimulus cycle was 200 ms, and square-wave stimulus, which were 2 to 4 times of thresholds inducing excitation and 2 ms wide, were applied. After 8 time continuous S1 stimulus, S2 stimulus were applied and shortened by 5 ms in S1-S2 connection phase. The longest S1-S2 interval which atrial activation associated with S2 stimulus disappeared was assumed to be ERP. Existence or nonexistence of atrial activations was judged from atrial electrogram. During rest, it was confirmed that ERP (ms) would be stably obtained twice or more, and then sample or solvent was intravenously administered. A given time after drug administration, ERP was measured. Comparing ERP after and before starting drug administration, rates of changes (%) was calculated.
As a result, the preferable compounds of the present invention, particularly compounds of the following Table, showed over 10% of ERP extension activities by 1 mg/kg administration.

**[Table 2]**

| Examples | ERP extension activities (1 mg/kg) |
|---|---|
| 1-1 | 13% |
| 2-1 | 14% |
| 2-3 | 10% |
| 2-14 | 10% |
| 2-23 | 10% |
| 5-8 | 15% |
| 9-4 | 10% |
| 9-5 | 10% |
| 9-6 | 10% |
| 9-9 | 20% |
| 11-8 | 10% |
| 11-23 | 10% |
| 12-2 | 11% |
| 12-10 | 19% |
| 12-21 | 11% |

### INDUSTRIAL APPLICABILITY

The compound of the present invention or a pharmaceutically acceptable salt thereof has I_{Kur} blocking activity and is useful for preventing or treating cardiac arrhythmia such as atrial fibrillation.

## Claims

1. A compound of the general formula: 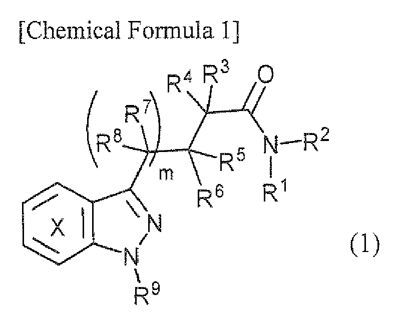 wherein Ring X is benzene or pyridine;
R¹ is optionally substituted alkyl;
R² is optionally substituted aryl, optionally substituted heterocyclic group, optionally substituted arylalkyl or optionally substituted heterocyclic-substituted alkyl;
provided that if Ring X is pyridine, R¹ and R² may combine each other together with the adjacent nitrogen atom to form a heterocyclic group of the following formula: 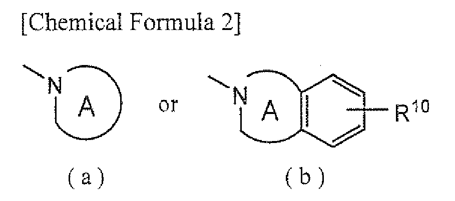 wherein Ring A is heterocyclic group, and R¹⁰ is hydrogen or alkyl;
R³, R⁴, R⁵, R⁶, R⁷, R⁸ and R⁹ are each hydrogen or alkyl; and R³ and R⁵ may combine each other together with the adjacent carbon atom to form cycloalkyl;
m is 0 or 1; or a pharmaceutically acceptable salt thereof.

2. The compound of claim 1, wherein R¹ is alkyl optionally substituted by 1 to 3 groups selected from (1) halogen, (2) hydroxyl, (3) optionally substituted amino, (4) alkylsulfonyl, (5) optionally substituted aminosulfonyl, (6) alkoxy, (7) cyano, (8) heterocyclic group, (9) optionally substituted carbamoyloxy, (10) optionally substituted carbamoyl, and (11) heterocyclic-substituted carbonyloxy, or a pharmaceutically acceptable salt thereof.

3. The compound of claim 1, wherein R¹ is alkyl optionally substituted by 1 to 3 groups selected from (1) hydroxyl, (2) amino optionally substituted by 1 or 2 groups selected from alkanoyl, alkoxycarbonyl, alkylsulfonyl, or aminosulfonyl optionally mono- or di-substituted by alkyl, and (3) carbamoyloxy optionally mono- or di-substituted by alkyl, or a pharmaceutically acceptable salt thereof.

4. The compound of any one of claims 1 to 3, wherein R² is
aryl optionally substituted by 1 to 3 groups selected from (1) halogen, (2) alkyl, (3) alkoxy, (4) haloalkyl, (5) optionally substituted amino and (6) optionally substituted carbamoyl;
arylalkyl optionally substituted by 1 to 3 groups selected from (1) halogen and (2) alkyl; or heterocyclic group, or a pharmaceutically acceptable salt thereof.

5. The compound of any one of claims 1 to 3, wherein R² is aryl optionally substituted by 1 to 3 groups selected from alkyl and alkoxy, or a pharmaceutically acceptable salt thereof.

6. The compound of any one of claims 1 to 5, wherein cycloalkyl which R³ and R⁵ combine each other together with the adjacent carbon atom to form is cyclopropyl, or a pharmaceutically acceptable salt thereof.

7. The compound of any one of claims 1 to 6, wherein m is 0, or a pharmaceutically acceptable salt thereof.

8. The compound of any one of claims 1 to 7, wherein R⁹ is hydrogen, or a pharmaceutically acceptable salt thereof.

9. A medicine comprising the compound of any one of claims 1 to 8 or a pharmaceutically acceptable salt thereof.

10. An I_{Kur} blocking agent comprising as the active ingredient the compound of any one of claims 1 to 8 or a pharmaceutically acceptable salt thereof.

11. A preventive or therapeutic agent for cardiac arrhythmia comprising as the active ingredient the compound of any one of claims 1 to 8 or a pharmaceutically acceptable salt thereof.

12. A preventive or therapeutic agent for atrial fibrillation comprising as the active ingredient the compound of any one of claims 1 to 8 or a pharmaceutically acceptable salt thereof.
